(19) Europäisches Patentamt / European Patent Office / Office européen des brevets

(11) **EP 4 238 621 A2**

(12) **EUROPÄISCHE PATENTANMELDUNG**

(43) Veröffentlichungstag:
06.09.2023   Patentblatt 2023/36

(21) Anmeldenummer: 23183100.9

(22) Anmeldetag: **28.05.2015**

(51) Internationale Patentklassifikation (IPC):
*A63B 71/06* (2006.01)   *G02C 7/10* (2006.01)
*A61F 9/02* (2006.01)

(52) Gemeinsame Patentklassifikation (CPC):
**G02C 7/101; A61F 9/023;** A63B 2071/0666

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.05.2014   DE 102014107587**
**11.06.2014   DE 102014108190**

(62) Dokumentnummer(n) der früheren Anmeldung(en) nach Art. 76 EPÜ:
**15727609.8 / 3 149 541**

(71) Anmelder: **Inoptec Limited**
**85406 Zolling (DE)**

(72) Erfinder: **Knoll, Ralf**
**85406 Zolling (DE)**

(74) Vertreter: **Köllner & Partner mbB**
**Vogelweidstraße 8**
**60596 Frankfurt am Main (DE)**

Bemerkungen:
Diese Anmeldung ist am 03.07.2023 als Teilanmeldung zu der unter INID-Code 62 erwähnten Anmeldung eingereicht worden.

(54)   **ELEKTRONISCHE BRILLE**

(57)   Es wird ein System mit einer Brille vorgeschlagen. Die Brille hat ein Brillenglas mit einer Flüssigkristallzelle (LC), deren Transmission (TR) zwischen transmittierend und blockierend umgeschaltet werden kann. Ferner weist die Brille mindestens einen augenseitigen Sensor (IL, IR) zum Messen der Helligkeit auf. Die Brille verfügt ferner über einen geschlossenen Regelkreis für die Regelung der Transmission der Flüssigkristallzelle, wobei die Regelung derart ausgebildet ist, dass Zeiten des Zustands hoher Transmission ($T_{on}$) mit zunehmender Blendung kürzer werden, wobei ein Sollwert für die Helligkeit am Auge vorgegeben ist und als Istwert die vom Sensor gemessene Helligkeit in Blickrichtung des Auges herangezogen wird. Ferner ist eine Anzeige vorhanden, die derart gesteuert wird, dass sie während der Zeiten des Zustands hoher Transmission ($T_{on}$) der Flüssigkristallzelle (LC) leuchtet und dass das zeitliche Integral des Produkts aus ihrer Leuchtintensität und der Transmission (TR) der Flüssigkristallzelle (LC) bei einer Veränderung der Zeiten des Zustands hoher Transmission ($T_{on}$) konstant bleibt

Fig. 1

EP 4 238 621 A2

**Beschreibung**

Gebiet der Erfindung

Die Erfindung betrifft eine elektronische Brille und ein System zur Blendunterdrückung.

Stand der Technik

Die durch einen Lichtmodulator hindurchtretende Lichtintensität lässt sich mit Hilfe von diversen am Markt verfügbaren Flüssigkristall-Zellen (TN, STN, Fe-LC, etc.) derart elektrisch steuern, dass mindestens 2 Zustände erreicht werden, nämlich durchlässig transparent - oder undurchlässig dunkel - wie es bei gängigen aktiven 3D-Fernseh- oder Kinobrillen der Fall ist (sog. Shutter-Brillen).

Gemäß dieser Grundidee wurden schon in den 60iger Jahren erste Versuche zu "elektronischen Sonnenbrillen" unternommen, um dem Träger einer solchen Brille eine variable Transmission zu bieten.

Einige bekannte elektronische Sonnenbrillen arbeiten mit einer reinen Steuerungen (statt einer Regelung), d.h. die Fotosensoren liegen an der Außenseite der Brille, so dass lediglich die Helligkeit gemessen wird, die auf die Brille von außen auftrifft (s. z. B US 5,172,256 A oder DE 10 2012 217 326 A1). Demensprechend wird mittels einer Kennlinie, die nur auf reinen Erfahrungswerten beruht, ein LCD entsprechend hell oder dunkel geschaltet.

Zudem existieren oftmals viel zu wenige Sensoren, deren Empfangsrichtung zudem unspezifisch ist (die Sensoren zeigen nach vorn oder in Richtung Himmel). Dies führt sehr oft zu völlig falschen und sogar gegenteiligen Reaktionen der Brille. Beispiel: Schaut der Träger der Brille beispielsweise in eine dunkles Bertachtungsgebiet (dunkle Ecke), während aber gleichzeitig die Brille von einem verirrten Strahl Sonnenlicht erfasst wird (durch zufällige Reflexionen an Gegenständen oder bewegte Blätter im Wald, die ein feines, variables Hell-Dunkel-Muster werfen), so wird das LC dunkel obwohl es eigentlich hell werden sollte, weil der Träger den dunklen Bereich sehen will.

Elektronische Systeme zur Unterdrückung von Blendlicht mit dem Ziel der Sichtverbesserung gibt es schon seit mehr als 80 Jahren (siehe z.B. US 2,066,680 A). In diesem Patent von 1934 wird das Licht der eigenen Scheinwerfer mit Hilfe rotierender mechanischer Schlitz- oder Fächerscheiben ("Chopper") in ein rechteckförmiges Signal (längs der Zeitachse) moduliert, während eine völlig identische Schlitz- oder Fächerscheibe in Blickrichtung vor dem Gesichtsfeld des Nutzers (Visor) exakt das gleiche vollbringt: d.h. mit exakt gleicher Frequenz und Phasenlage, wird synchron zum modulierten Scheinwerferlicht die Außenwelt vom Nutzer wahrgenommen.

Ist das Visor, durch welches der Nutzer blickt, beispielsweise 50% der Zeit geschlossen (Puls-Pause-Verhältnis = 1:1), so wird 50% des unerwünschten Lichtes (z.B. tief stehende Sonne) unterdrückt, und somit die Sichtbarkeit der zu betrachtenden Gegenstände erhöht.

Später ersetzten elektronisch ansteuerbare Lichtmodulatoren die mechanischen Lichtmodulatoren, insbesondere in Form von Flüssigkristallzellen, und auch die Lichtquellen wurden zunehmend schneller und einfacher elektronisch ansteuerbar (siehe z.B. DE 101 34 770 A1, DE 2 001 086 A, WO 2013/143 998 A2).

Aufgabe

Aufgabe der Erfindung ist es, Brillen und Systeme anzugeben, die für Sichtverbesserungen des Brillenträgers unter unterschiedlichen Bedingungen sorgen.

Lösung

Diese Aufgabe wird durch die Gegenstände der unabhängigen Ansprüche gelöst. Vorteilhafte Weiterbildungen der Gegenstände der unabhängigen Ansprüche sind in den Unteransprüchen gekennzeichnet. Der Wortlaut sämtlicher Ansprüche wird hiermit durch Bezugnahme zum Inhalt dieser Beschreibung gemacht.

Im Folgenden werden in unterschiedlichen Abschnitten unterschiedliche Aspekte geschildert, die die Aufgabe lösen oder zu deren Lösung beitragen. Für einen Fachmann ist klar, dass fast alle dieser unterschiedlichen Aspekte miteinander kombiniert werden können.

Eye-Tracker

Zur Lösung der Aufgabe wird eine Brille für einen Träger mit mindestens einem Auge vorgeschlagen. Die Brille hat mindestens ein Brillenglas, wobei das mindestens eine Brillenglas eine Flüssigkristallzelle aufweist, deren Transmission durch eine geeignete Ansteuerung veränderbar ist. Ferner weist die Brille einen Eye-Tracker auf, der die Blickrichtung des Auges bestimmen kann. Ferner mindestens einen Sensor zum Messen der Helligkeit des auf ihn treffenden sichtbaren Lichts, wobei der Sensor augenseitig des Brillenglases angeordnet ist, die Helligkeit durch das mindestens eine Brillenglas messen kann, und

- ein abbildendes System mit einer Kamera oder
- mindestens 3 Sensoren, die ein Koordinatensystem aufspannen, oder
- ein Facettenauge

aufweist.

Ein elektronisches Facettenauge besteht aus vielen Einzelaugen, ähnlich zu dem in der Biologie verwendeten Begriff der "Ommatidien" in der Beschreibung eines Facettenauges von Fluginsekten, jedoch bestehend aus elektrischen Photosensoren, die wiederum am unteren Ende von lichtleitenden Trichtern (ohne Linse) sitzen - oder mit jeweils einer vorgeschalteten Mikro-Linse versehen sind - oder einer Kombination aus beiden (Trichter und Mikro-Linse) (siehe z.B. EP 0813079 A2).

Der mindestens eine Sensor kann die Helligkeit des aus der mit dem Eye-Tracker bestimmten Blickrichtung des Auges auf ihn treffenden sichtbaren Lichts bestimmen.

Die Brille verfügt ferner über einen geschlossenen Regelkreis für die Regelung der Transmission der Flüssigkristallzelle, wobei ein Sollwert für die Helligkeit am Auge vorgegeben ist, und wobei der Regelkreis als Istwert die vom Sensor gemessene Helligkeit in Blickrichtung des Auges heranzieht.

Mit einer solchen Brille lässt sich die Helligkeit schnell und exakt an die Blendung aus der tatsächlichen Blickrichtung des Brillenträgers anpassen, etwa wenn einem Autofahrer ein anderes Auto entgegenkommt oder wenn ein Autofahrer an einem sonnigen Tag in einen Tunnel fährt oder aus einem Tunnel heraus fährt.

Erst jetzt, im Zuge von extremer Miniaturisierung und "tragbarer Elektronik" (wearable electronics), ist es möglich, mittels Miniatur-Elektronik, die sich auch in eine Brille leicht und einfach integrieren lässt, solche leistungsfähigen und sicheren Systeme zur Sichtverbesserung zu realisieren.

Um die Anwendungsmöglichkeiten der Brille zu erweitern, bietet es sich an, die Transmission der Flüssigkristallzelle der Brille nicht auf eine jeweils passende Graustufe einzustellen, sondern die Brille in kurzer Folge zwischen so weit wie möglich transmittierend und so weit als möglich Licht blockierend umzuschalten. Damit das menschliche Auge davon möglichst nichts sieht, sollte ein Zyklus (Periode) aus einer transmittierenden Zeitspanne und einer blockierenden Zeitspanne maximal ein Vierundzwanzigstel (1/24) einer Sekunde dauern.

Solche Systeme funktionieren besonders gut, wenn der Mensch die Regelung nicht mehr wahrnimmt, also mit Zykluszeiten oberhalb der Flimmer-Grenzfrequenz (Critical Flicker Frequency - CFF) von etwa 60Hz arbeiten.

Um dies erreichen zu können, sollte die Flüssigkristallzelle derart ausgebildet sein, dass sie ihre Transmission von 90% auf 10% und von 10% auf 90% in maximal 10 ms ändern kann.

Hat man eine solche Flüssigkristallzelle, kann die Transmission der Flüssigkristallzelle zwischen Zuständen hoher und niedriger Transmission umgeschaltet werden. Dazu müssen Mittel zum Regeln oder Steuern der Zeiten des Zustands hoher und niedriger Transmission der Flüssigkristallzelle sowie des Wechsels zwischen diesen beiden Zuständen vorhanden sein. Sinnvollerweise ist die Regelung oder Steuerung und der geschlossene Regelkreises derart ausgebildet, dass die Zeiten des Zustands hoher Transmission mit zunehmender Helligkeit des auf den mindestens einen Sensor treffenden sichtbaren Lichts kürzer werden (Pulsweiten-Modulation, PWM).

Noch präziser und noch schonender für die Augen des Brillenträgers wird die Regelung, wenn der Regelkreis derart ausgebildet ist, dass er bei der Bestimmung der Helligkeit aus der Blickrichtung des Auges eine nutzerspezifische Augen/Netzhaut-Empfindlichkeitskurve zur Gewichtung der Helligkeit berücksichtigen kann.

Die nutzerspezifischen Augen/Netzhaut-Empfindlichkeitskurve berücksichtigt z.B. das Alter des Brillenträgers, sonstige allgemeine und/oder individuelle augenspezifische Parameter, vornehmlich bezüglich des Einfallswinkels ins Auge, aber auch bezüglich anderer lichttechnischer Größen die Einfluss auf die Wahrnehmung haben, wie z.B. Leuchtdichte, Entfernung der Lichtquelle bzw. Lichtintensität oder Lichtstärke (Lichtfluss pro Winkel Steradiant), Beleuchtungsstärke, deren jeweilige absolute Größen als Schwelle am Auge, Lichtfluss, Größe der Störquelle (Punkt vs. Fläche), Farbe oder Spektralverteilung der Quelle und deren zeitliche Variation, Voreinstellung des Auges (photopisches vs. skotopisches Sehen, etc.).

Diese Empfindlichkeitskurven können heuristisch und logisch ermittelt werden, werden aber in der Regel empirisch ermittelt, wie beispielsweise herangezogen und ausgewertet in der Arbeit: Douglas Mace, Philip Garvey, Richard J. Porter, Richard Schwab, Werner Adrian: "Counter-measures for Reducing the Effects of Headlight Glare"; Prepared for: The AAA Foundation for Traffic Safety, Washington, D.C., December 2001.

Die vorgenannten Empfindlichkeitskurven des menschlichen Auges sind als Gewichtungsfaktoren in verschiedenen Tabellen (Lookup Table - LUT) oder als berechenbare Formel hinterlegt - jedenfalls so, dass im geschlossenen Regelkreis des Systems, bestehend aus Innensensor, einem Micro-Controller und dem eingestellten Sollwert, diese Gewichtungsfaktoren in Echtzeit in das Stellsignal zum Einstellen des Transmission der Flüssigkristallzelle einbezogen werden.

Beispielsweise beschreibt eine Formel von Adrian und Bhanji (Adrian, W. und Bhanji, A. (1991). "Fundamentals of disability glare. A formula to describe stray light in the eye as a function of the glare angle and age." Proceedings of the First International Symposium on Glare, Orlando, Florida, pp. 185-194) zur Bestimmung der "unmöglichen Lesbarkeit und Erkennbarkeit von Objekten bei Gegenlicht" (Engl. "Disability Glare"), die Abhängigkeit vom Einfallswinkel des Störlichtes ins Auge, unter dem zunehmend nichts mehr erkennbar ist.

Beispiel: Fällt Störlicht direkt senkrecht in das Auge, so ist die Blendung am höchsten (Maximum in der Gewichtungsformel). Nachdem der Eye-Tracker die Blickrichtung bestimmt (Vektor ET(x.y,z), und der Innensensor und/oder der Außensensor die Einfallsrichtung des Störlichtes (Vektor Glare(x,y,z), so kann der Microcontroller prüfen, ob diese beide Vektoren kollinear sind, d.h. die gleiche Richtung aufweisen, und dementsprechend mit der vorgenannten Gewichtungskurve maximal bewerten. Ist die Gewichtungskurve beispielsweise als LUT hinterlegt, so bewegt sich diese mit Blickrichtungs-Vektor ET(x,y,z) virtuell im Speicher des Microcontrollers der Augenbewegung entsprechend hin und her. Ist sie als Formel hinterlegt, wird dementsprechend der Vektor in einen Winkel umgerechnet.

Dadurch muss man die Empfindlichkeitskurven nicht mehr als spezielle Vorsatzlinsen anfertigen (z.B. individuelle Freiform-Plastiklinse), die das Licht entsprechend "gewichten" bevor es auf einen Photosensor trifft. Jegliche gewichtende Vorsatzlinsen, oder gar bewegliche Vorsatzlinsen, die die Empfindlichkeit der Retina nachbilden, können entfallen, da alles rein in Software abgebildet ist, während alle Sensoren starr montiert sind.

Besonders schonend für das Auge und präzise von der Regelung her ist es, wenn die Brille ein Brillengestell aufweist, dass das dem mindestens einen Brillenglas zugeordnete Auge lichtdicht gegenüber dem Umgebungslicht abdichtet.

Als besonders schonend für das Auge hat sich eine Festlegung des Sollwerts des Regelkreises auf eine mittlere Helligkeit im Bereich von 20 bis 400 lx (Lux) erwiesen. Ein solcher Wert erlaubt eine Regelung auf eine gleichbleibende Helligkeit für die Augen des Brillenträgers, wenn sich die äußere Helligkeit von sehr hell bis hinunter zu dem eingestellten Sollwert oder umgekehrt ändert, etwa wenn ein Auto an einem Sommertag in einen Tunnel einfährt oder aus einem Tunnel herausfährt. Die Änderungen der Helligkeit bzw. der Beleuchtungsstärke können in einem solchen Moment einen Faktor 1000 oder mehr ausmachen. Der Brillenträger ist diesen gegebenenfalls sehr schnellen Helligkeitsschwankungen nicht ausgesetzt. Sie werden von der Regelung der Brille stets nivellierend ausgeglichen.

Die Einfahrt in einen dunklen Tunnel oder dunklen Schattenbereich (Wald etc.) an einem hellen sonnigen Tag ist ein typischer Anwendungsfall. Da der hier eingestellte Sollwert tagsüber einer dunklen Sonnenbrille entspricht, ist das Auge stets von vornherein dunkel adaptiert und vorbereitet, so dass beim Eintritt in den Dunkelbereich das Brillenglas lediglich in Echtzeit transparenter und klarer (offener) geregelt werden muss, um sofort im Dunkeln sehen zu können. Die ohne diese Brille benötigte Dunkeladaptionszeit des menschlichen Auges von ca. 30 Sekunden wird somit auf einen Sekundenbruchteil (z.B. 10 Millisekunden) reduziert, so dass man sofort im Dunkeln sehen kann. Bei der Tunnelausfahrt zurück ins Helle gilt genau das Umgekehrte.

Weitere Regelungsmöglichkeiten, die weiter unten geschildert werden, eröffnen sich, wenn die Brille mindestens einen weiteren Helligkeits-Sensor aufweist, der auf der vom Auge abgewandten Seite der Brille angeordnet ist (Außensensor) und die Helligkeit des Umgebungslichts bestimmt.

Zum Beispiel kann dann der Sollwert des Regelkreises abhängig von der Helligkeit des Umgebungslichts verändert werden, wobei eine solche Veränderung des Sollwerts um mindestens einen Faktor zehn langsamer ist als die Regelung der Transmission der Flüssigkristallzelle erfolgen sollte, damit das Auge des Brillenträgers sich ohne Schwierigkeiten an dieser Änderung adaptieren kann.

Auf plötzliche Helligkeit-Veränderungen sollte die Brille innerhalb von 10 $\mu$s bis einer Sekunde derart reagieren, dass die Flüssigkristallzelle (LC) auf den Zustand niedriger Transmission eingestellt wird.

Die Brille wird in extremen Situationen, bei sogenannter "Disability Glare", bei der (siehe oben) ein Mensch nichts mehr lesen oder sehen kann, nämlich dann, wenn exakt senkrecht ins Auge (unter null Grad) eine extrem starke Blendung auftritt, wie beim direkten Blick in die Sonne, komplett geschlossen, d.h. sie wird auf völlig schwarz eingestellt.

Eine solche Regelung ist insofern nicht kritisch, als dass es keine Rolle spielt, ob man nichts sieht wegen extremer Blendung oder weil die Brille schwarz abdunkelt, jedoch hat letzterer Zustand den Vorteil dass das Auge geschützt und dunkeladaptiert bleibt.

Nach einer gewissen Zeit bzw. einer Blickrichtungsänderung des Brillenträgers wird die Brille anschließend langsam wieder Licht transportieren lassen.

Noch präziser wird die Regelung der Blendunterdrückung, wenn die Brille zwei Brillengläser für zwei Augen eines Brillenträgers aufweist, sowie je einen augenseitigen Sensor für jedes Brillenglas zum Messen der Helligkeit des auf das jeweilige Auge treffenden sichtbaren Lichts. Mithilfe je eines Regelkreises für jedes Brillenglas kann man dann die Regelung für jedes Auge individuell erfolgen.

Eine Verstärkung des Helligkeits-/Kontrast-Umfangs kann mit einer derartigen Brille erreicht werden, wenn die Sollwerte für die beiden Augen um 1% bis 60% voneinander abweichen. Typische Werte für die Rechts-Links-Abweichungen in der Praxis liegen bei 5% - 30%. In Analogie zur High Dynamik Range (HDR) Fotografie kann hier von "HDR-Sehen" gesprochen werden.

Solche Systeme standen bis dato nur theoretisch zur Verfügung, aber erst jetzt, durch die Verfügbarkeit von extrem schnellen Modulatoren und sehr schnellen Prozessoren können intelligente und sicherheitsrelevante Mehrkanal-Echtzeit-Regelungen zur Sichtverbesserung realisiert werden, wobei das linke und rechte Auge getrennt behandelt werden, und/oder mehrere Nutzer für Gruppenanwendungen einbezogen werden können.

Um dies zu gewährleisten, sollte bei der Regelung der Helligkeit des auf ein Auge treffenden sichtbaren Lichts die Regelung der Helligkeit für das andere Auge berücksichtigt werden.

Die Brille kann auch mit einer Lichtquelle kombiniert werden, die an der vom Auge abgewandten Seite der Brille angeordnet ist. Sinnvollerweise wird dann die Lichtquelle abhängig von der Blickrichtung des Brillenträgers gesteuert. Auf diese Weise kann der Verdunkelung entgegengewirkt werden, die durch das Shuttern der Brille zur Vermeidung von Blendung hervorgerufen wird. Denkbar sind zum Beispiel vier LEDs, an jeder Brillenecke eine.

Der Eye-Tracker stellt dann fest, welche von den vier LEDs je nach Blickrichtung bevorzugt mit Energie versorgt werden soll - entweder nur eine in Blickrichtung korrespondierende LED bei Blick nach außen oben/unten - oder zwei in Blickrichtung korrespondierende LEDs bei Blick seitlich mittig - oder alle vier LED bei Blick geradeaus nach vorn.

Weitere Möglichkeiten:

Anstelle von oder zusätzlich zu den vier starr montierten LEDs an den Ecken einer Brille, können auch beliebige andere Lichtquellen/Scheinwerfer mit Hilfe des Eye-Trackers in ihrer Leuchtrichtung in die Blickrichtung gesteuert werden.

Zu diesem Zweck sind diese Lampen elektro-mechanisch schwenkbar, ähnlich wie beim elektronisch einschwenkenden Kurvenlicht bei Kraftfahrzeugen oder bei schwenkbaren 3-Achsen-Überwachungskameras oder bei frei beweglichen von Hand getragenen Systemen, die aber via elektronischer oder massenträger kardanischer Aufhängung (Engl. Gimbal oder Steadycam-Verfahren) ein eigenes stabiles Koordinatensystem bezüglich Erde oder Träger aufrecht erhalten und in dessen Relation der Scheinwerfer dann in Blickrichtung schwenken kann.

Somit kommen alle Arten von LED-Scheinwerfern auf allen Arten von Halterungen in Frage: Auto, Helm, Fahrrad, Motorrad, Hand, Schulter, Körper, Gewehr usw..

Besonders effektiv ist dies, wenn die Leuchtzeiten und die Leuchtintensität der Lichtquelle derart geregelt werden, dass die Lichtquelle während der Zeiten des Zustands hoher Transmission der Flüssigkristallzelle leuchtet. Dabei sollte das zeitliche Integral des Produkts aus der Leuchtintensität der Lichtquelle und der Transmission der Flüssigkristallzelle bei einer Veränderung der Zeiten des Zustands hoher Transmission innerhalb einer vorgegebenen Toleranz konstant bleibt.

Eine solche blitzende Lichtquelle kann beispielsweise ein Autoscheinwerfer sein, der für den Autofahrer die Straße und das Umfeld stets mit konstanter Helligkeit beleuchtet, während die Blendung durch entgegenkommende Fahrzeuge durch das Shuttern der Brille effektiv verhindert wird. Aber auch andere Arten von Scheinwerfern, wie Fahrradlampen, Helmlampen, Taschenlampen, sind in dem hier beschriebenen Sinne einsetzbar.

Da die von einem Außenstehenden oder entgegenkommenden Fahrzeug wahrgenommene Helligkeit des Autoscheinwerfers unter diesen Bedingungen stets konstant ist, unabhängig davon, wie das Puls-Pausen-Verhältnis geregelt ist, kann ein solcher Autoscheinwerfer problemlos im Sinne einer Ersatz-Strategie (*replacement strategy*) oder ein Hinzukaufen von Zusatzscheinwerfern im Sinne einer Zubehör-Strategie (*special accessories strategy*) angeboten werden.

Erst jetzt ist es möglich, mittels leistungsstarker Weißlicht- und/oder RGB-LED/LASER, solche leistungsfähigen und sicheren Systeme zur Sichtverbesserung zu realisieren.

Neben einem Autoscheinwerfer sind insbesondere auch

- eine Lichtquelle zum Blenden eines Lebewesens, eines optischen Sensors oder einer Kamera, und/oder
- eine Anzeige an der vom Auge abgewandten Seite des Brillenglases und/oder
- eine Anzeige augenseitig des Brillenglases und/oder
- ein Head-Up-Display

als Lichtquellen denkbar.

Als Anzeigen an der vom Auge abgewandten Seite des Brillenglases kommen beispielsweise ein Smartphone, Tablett, Laptop, Cockpit-Anzeige, etc. in Betracht.

Als Anzeigen augenseitig des Brillenglases kommen zum Beispiel "Google Glass" oder Anzeigen zur virtuellen oder erweiterte Realität ("*augmented reality*") in Betracht.

Unter Head-up-Displays (HUD) werden unterschiedliche Anzeigen subsummiert, einige davon augenseitig der Brille, einige außerhalb der Brille, etwa in Form eines Helms mit Display. Allen gemeinsam ist, dass man durch sie hindurch schauen kann, das Head-up-Display jedoch zusätzliche Informationen eingeblendet.

Alle diese Anzeigen werden auf die geschilderte Weise gegen die Sonne oder sonstige störende Blendlichtquellen ablesbar.

Das System lässt sich hervorragend kombinieren mit den weiter unten geschilderten Systemen und Verfahren zur Eigenlicht-Erkennung.

Im Folgenden werden einzelne Verfahrensschritte näher beschrieben. Die Schritte müssen nicht notwendigerweise in der angegebenen Reihenfolge durchgeführt werden, und das zu schildernde Verfahren kann auch weitere, nicht genannte Schritte aufweisen.

Die Aufgabe wird außerdem gelöst durch ein Verfahren zum Regeln der Helligkeit des auf mindestens ein Auge treffenden sichtbaren Lichts, mit folgenden Schritten:

> 1. eine Brille wird zur Verfügung gestellt, wobei die Brille folgendes aufweist:

>> 1.1 mindestens ein Brillenglas;
>> 1.2 wobei das mindestens eine Brillenglas eine Flüssigkristallzelle (LC) aufweist, deren Transmission (TR) durch eine geeignete Ansteuerung veränderbar ist;

> 2. einen Eye-Tracker (ET), der die Blickrichtung des Auges bestimmt;
> 3. mindestens ein Sensor (IL, IR) zum Messen der Helligkeit des auf den Sensor treffenden sichtbaren Lichts wird zur Verfügung gestellt;

>> 3.1 wobei der mindestens eine Sensor (IL, IR) augenseitig des Brillenglases angeordnet ist;
>> 3.2 wobei der mindestens eine Sensor (IL, IR) die Helligkeit durch das mindestens eine Brillenglas misst;
>> 3.3. wobei der mindestens eine Sensor (IL, IR)

>>> 3.3.1 ein abbildendes System mit einer Kamera oder
>>> 3.3.2 mindestens drei Sensoren, die ein Koordinatensystem aufspannen oder
>>> 3.3.3 ein Facettenauge
>>> aufweist;

>> 3.4 wobei der mindestens eine Sensor (IL, IR) die Helligkeit des aus der mit dem Eye-Tracker (ET) bestimmten Blickrichtung des Auges auf ihn treffenden sichtbaren Lichts bestimmt;

> 4. ein geschlossener Regelkreis (MC) für die Regelung der Transmission der Flüssigkristallzelle (LC) wird zur Verfügung gestellt;

>> 4.1 wobei ein Sollwert für die Helligkeit am Auge vorgegeben ist;
>> 4.2 wobei der Regelkreis als Istwert die vom Sensor gemessene Helligkeit in Blickrichtung des Auges heranzieht.

Verbesserung der Lesbarkeit einer Anzeige-Einrichtung

Zur Lösung der Aufgabe wird ferner ein System zum Sichtverbessern durch Blendunterdrückung vorgeschlagen. Das System umfasst:

- eine Brille für einen Träger mit mindestens einem Auge, mit
- mindestens einem Brillenglas, wobei das mindestens eine Brillenglas eine Flüssigkristallzelle aufweist, deren Transmission durch eine geeignete Ansteuerung veränderbar ist. Die Flüssigkristallzelle ist derart ausgebildet, dass die Transmission der Flüssigkristallzelle zwischen Zuständen hoher und niedriger Transmission umgeschaltet werden kann. Diesbezüglich verfügt die Brille auch über entsprechende Mittel zum Regeln oder Steuern der Zeiten des Zustands hoher Transmission der Flüssigkristallzelle.

Zusätzlich weist die Brille mindestens einen Sensor zum Messen der Helligkeit des auf ihn treffenden sichtbaren Lichts, wobei der mindestens eine Sensor augenseitig des Brillenglases angeordnet ist und die Helligkeit durch das Brillenglas hindurch nach vorne misst.

Ein geschlossener Regelkreis regelt die Transmission der Flüssigkristallzelle. Dabei ist die Regelung derart ausgebildet ist, dass die Zeiten des Zustands hoher Transmission mit zunehmender Blendung kürzer werden (Pulsweiten-Modulation, PWM). Für die Helligkeit am Auge des Brillenträgers wird ein Sollwert vorgegeben, wobei der Regelkreis als Istwert die vom Sensor gemessene Helligkeit heranzieht.

Ferner verfügt das System über eine Anzeige sowie Mittel zum Steuern oder Regeln der Leuchtzeiten und der Leuchtintensität der Anzeige derart, dass diese während der Zeiten des Zustands hoher Transmission der Flüssigkristallzelle leuchtet. Dabei bleibt das zeitliche Integral des Produkts aus der Leuchtintensität der Anzeige und der Transmission der Flüssigkristallzelle bei einer Veränderung der Zeiten des Zustands hoher Transmission innerhalb einer vorgegebenen Toleranz konstant.

Verdoppelt sich etwa die Helligkeit des Umgebungslichts, so reagiert das System einerseits mit einer Halbierung der Zeiten des Zustands hoher Transmission der Flüssigkristallzelle, wodurch die erhöhte Blendung effektiv ausgeglichen ist. Gleichzeitig wird die Leuchtzeit der Anzeige verkürzt und ihre Leuchtintensität verdoppelt. Dadurch bleibt die vom Brillenträger wahrgenommene Helligkeit der Anzeige unverändert.

Alle diese Vorgänge des Umschaltens der Transmission der Flüssigkristallzelle und des Ein- und Ausschaltens der Anzeige sollten mit einer solchen Frequenz und Schnelligkeit stattfinden, dass für den Träger der Brille kein Flimmern oder sonstige wahrnehmbare Effekte auftreten. D.h. alle für den Träger potenziell wahrnehmbaren Effekte sollten mit mindestens 24 Hz, besser mit mindestens 60 Hz erfolgen.

Als Anzeigen kommen insbesondere infrage:

- eine Anzeige an der vom Auge abgewandten Seite des Brillenglases und/oder
- eine Anzeige augenseitig des Brillenglases und/oder
- ein Head-Up-Display.

Als Anzeigen an der vom Auge abgewandten Seite des Brillenglases kommen beispielsweise ein Smartphone, Tablett, Laptop, Cockpit-Anzeige, etc. oder auch ein Head-up-Display (HUD) in Betracht.

Als Anzeigen augenseitig des Brillenglases kommen zum Beispiel "Google Glass" oder Anzeigen zur virtuellen oder erweiterte Realität (*"augmented reality"*) in Betracht.

Alle diese Anzeigen werden auf die geschilderte Weise auch bei starker Sonneneinstrahlung oder gar bei direkter Blendung durch die Sonne als Gegenlicht ablesbar.

Vorzugsweise weist die Brille einen Eye-Tracker auf, der die Blickrichtung des Auges bestimmen kann. Der mindestens eine Sensor weist in einem solchen Fall

- ein abbildendes System mit einer Kamera oder
- mindestens 3 Sensoren, die ein Koordinatensystem aufspannen, oder
- ein Facettenauge

auf.

Ein elektronisches Facettenauge besteht aus vielen Einzelaugen, ähnlich zu dem in der Biologie verwendeten Begriff der "Ommatidien" in der Beschreibung eines Facettenauges von Fluginsekten, jedoch bestehend aus elektrischen Photosensoren, die wiederum am unteren Ende von lichtleitenden Trichtern (ohne Linse) sitzen - oder mit jeweils einer vorgeschalteten Mikro-Linse versehen sind - oder einer Kombination aus beiden (Trichter und Mikro-Linse) (siehe z.B. EP 0813079 A2).

Der mindestens eine Sensor kann die Helligkeit des aus der mit dem Eye-Tracker bestimmten Blickrichtung des Auges auf ihn treffenden sichtbaren Lichts bestimmen. Der Regelkreis kann dann als Istwert die vom Sensor gemessene Helligkeit in Blickrichtung des Auges heranziehen.

Mit einer solchen Brille lässt sich die Helligkeit schnell und exakt an die Blendung aus der tatsächlichen Blickrichtung des Brillenträgers anpassen, etwa wenn einem Autofahrer ein anderes Auto entgegenkommt und der Autofahrer entweder in Richtung des entgegenkommenden Fahrzeugs blickt oder nicht. Da die Darstellung der Anzeige stets an die von der Brille vorgenommene Blendunterdrückung angepasst wird, wird die Lesbarkeit der Anzeige niemals beeinträchtigt.

Die Aufgabe wird ferner durch ein Verfahren gelöst, das einem bestimmungsgemäßen Betrieb des geschilderten Systems entspricht.

Codierung

Die Aufgabe wird ferner durch ein System zum Sichtverbessern durch Blendunterdrückung gelöst. Das System weist folgendes auf:

- eine Brille für einen Träger mit mindestens einem Auge, mit
- mindestens einem Brillenglas;
- wobei das mindestens eine Brillenglas eine Flüssigkristallzelle aufweist, deren Transmission durch eine geeignete Ansteuerung veränderbar ist;
- wobei die Flüssigkristallzelle derart ausgebildet ist, dass die Transmission der Flüssigkristallzelle zwischen Zuständen hoher und niedriger Transmission umgeschaltet werden kann.

Ferner umfasst die Brille Mittel zum Regeln oder Steuern der Zeiten des Zustands hoher Transmission der Flüssigkristallzelle.

Das System umfasst außerdem eine Lichtquelle mit Mitteln zum Steuern oder Regeln der Leuchtzeiten und der Leuchtintensität der Lichtquelle derart, dass diese während der Zeiten des Zustands hoher Transmission der Flüssigkristallzelle leuchtet. Dabei bleibt das zeitliche Integral des Produkts aus der Leuchtintensität der Lichtquelle und der Transmission der Flüssigkristallzelle bei einer Veränderung der Zeiten des Zustands hoher Transmission innerhalb einer vorgegebenen Toleranz konstant.

Die Regelung oder Steuerung der Flüssigkristallzelle und der Lichtquelle ist derart ausgebildet, dass die zeitliche Position der Zeiten des Zustands hoher Transmission innerhalb einer Periode aus Zeiten des Zustands hoher Transmission und Zeiten des Zustands niedriger Transmission kontinuierlich oder sprunghaft verändert werden kann. Und/oder die Dauer einer Periode aus Zeiten des Zustands hoher Transmission und Zeiten des Zustands niedriger Transmission kann kontinuierlich oder sprunghaft verändert werden.

Diese Veränderungen werden dabei durch einen geheimen Codierschlüssel bestimmt.

Alle diese Vorgänge des Umschaltens der Transmission der Flüssigkristallzelle und des Ein- und Ausschaltens der Lichtquelle sollten mit einer solchen Frequenz und Schnelligkeit stattfinden, dass für den Träger der Brille kein Flimmern oder sonstige wahrnehmbare Effekte auftreten. D.h. alle für den Träger potenziell wahrnehmbaren Effekte sollten mit mindestens 24 Hz, besser mit mindestens 60 Hz erfolgen.

Eine solche Codierung eröffnet vielfältige Möglichkeiten, insbesondere im militärischen und Sicherheits-Bereich (Polizei, Feuerwehr, etc.). Sie macht es einem gegenüber, das nicht über den Codierschlüssel verfügt, schwer, z.B. eine Blendung durch die Lichtquelle zu eliminieren.

Auch eröffnet die Codierung die Möglichkeit, dass unterschiedliche Gruppen, seien es Gegner oder andere Teams mit ähnlichem Auftrag, jeweils eine individuell geheime exklusive Sicht via codierter Eigenlichtquellen erhalten, insbesondere falls außen stehende Nutzer mit sehr ähnlichen Gesamtsystemen (Visor und Lichtquelle) nachts in der gleichen räumlichen Region agieren.

Zur automatischen Regelung der Blendunterdrückung verfügt die Brille vorzugsweise über mindestens einen Sensor zum Messen der Helligkeit des auf den Sensor treffenden sichtbaren Lichts. Der Sensor ist augenseitig des Brillenglases angeordnet und misst die Helligkeit durch das mindestens eine Brillenglas. Ferner umfasst die Brille einen geschlossenen Regelkreis für die Regelung der Transmission der Flüssigkristallzelle dergestalt, dass die Zeiten des Zustands hoher Transmission mit zunehmender Helligkeit kürzer werden (Pulsweiten-Modulation, PWM). Ein Sollwert für die Helligkeit am Auge des Brillenträgers ist vorgegeben, wobei der Regelkreis als Istwert die vom Sensor gemessene Helligkeit heranzieht.

Die Genauigkeit der Blendunterdrückung kann erhöht werden, wenn einerseits der mindestens eine Sensor ein abbildendes System mit einer Kamera oder mindestens drei Sensoren, die ein Koordinatensystem aufspannen, oder ein Facettenauge aufweist. Und andererseits die Brille ferner einen Eye-Tracker aufweist, der die Blickrichtung des Auges bestimmen kann. Denn dann kann der mindestens eine Sensor die Helligkeit des aus der mit dem Eye-Tracker bestimmten Blickrichtung des Auges auf ihn treffenden sichtbaren Lichts bestimmen. Und der Regelkreis kann als Istwert die vom Sensor gemessene Helligkeit in Blickrichtung des Auges heranziehen. Dies führt klarerweise zu einer sehr exakten Unterdrückung der tatsächlich vorliegenden Blendung.

Von besonderem Interesse für Sicherheitsanwendungen ist es, wenn entweder die Lichtquelle oder eine zusätzliche, zweite Lichtquelle zum Blenden eines Lebewesens, eines optischen Sensors oder einer Kamera geeignet ist. Zum Beispiel könnte die Lichtquelle geeignet sein, ein Nachtsichtgerät zu blenden, was schon mit geringen Intensitäten z.B. aus einer Infrarot-Lichtquelle zu erreichen ist. Militärische Nachtsichtsysteme funktionieren bei zunehmender Helligkeit nicht mehr, weil die sehr empfindlichen Empfänger / Restlichtverstärker ab bestimmten Helligkeiten "übersteuert" sind, d.h. durch zu viel Licht ihren Dienst versagen.

Klarerweise sollte auch eine zweite Lichtquelle lediglich während der Zeiten des Zustands niedriger Transmission der Flüssigkristallzelle leuchten. Dies eröffnet die Möglichkeit, einen Verbrecher oder Gegner zu blenden, ohne selber geblendet zu werden.

Die Aufgabe wird ferner durch ein Verfahren gelöst, das einem bestimmungsgemäßen Betrieb des geschilderten Systems entspricht.

Blendwaffe

Die Aufgabe wird ferner gelöst durch ein System zum Blenden eines Lebewesens, eines optischen Sensors oder einer Kamera, mit:

- einer Brille für einen Träger mit mindestens einem Auge, mit mindestens einem Brillenglas, wobei das mindestens eine Brillenglas eine Flüssigkristallzelle aufweist, deren Transmission durch eine geeignete Ansteuerung veränderbar ist. Dabei ist die Flüssigkristallzelle derart ausgebildet ist, dass die Transmission der Flüssigkristallzelle zwischen Zuständen hoher und niedriger Transmission umgeschaltet werden kann. Hinzu kommen Mittel zum Steuern der Zeiten des Zustands hoher Transmission der Flüssigkristallzelle.

- Ferner hat das System eine Lichtquelle zum Blenden eines Lebewesens, eines optischen Sensors oder einer Kamera, die während der Zeiten des Zustands niedriger Transmission der Flüssigkristallzelle leuchtet.

Der große Vorteil eines solchen Systems besteht darin, dass mithilfe der Lichtquelle z.B. ein Verbrecher oder Gegner geblendet werden kann, der Träger der Brille jedoch nicht geblendet wird, da die Lichtquelle immer nur leuchtet, wenn die Flüssigkristallzelle in der Brille das Licht blockiert.

Zudem könnte der Geblendete/das zu blendende System hinter einer spiegelnden Scheibe sitzen (z.B. in einem Fahrzeug) oder zufällig spiegelnde Gegenstände bei sich tragen oder vorsätzlich einen Spiegel benutzen, um das Blendlicht absichtlich zurück zum Sender zu werfen. Nach aktuellem Stand der Technik ist der Bediener der Blendwaffe dann ungeschützt und könnte mit seinem eigenen Licht via Rückreflexion beeinträchtigt werden. Auch Teamkollegen derselben Einsatzgruppe, die sich z.B. rechts oder links neben dem Bediener befinden, könnten nach aktuellem Stand der Technik ebenfalls durch Rückreflexionen geblendet werden. Dies gilt auch im unvorsichtigen und versehentlichen Umgang mit Blendwaffen. Mit dem vorgeschlagenen System werden diese Risiken eliminiert.

Zum Beispiel könnte die Lichtquelle geeignet sein, ein Nachtsichtgerät zu blenden, was schon mit geringen Intensitäten z.B. aus einer Infrarot-Lichtquelle zu erreichen ist. Militärische Nachtsichtsysteme funktionieren bei zunehmender Helligkeit nicht mehr, weil die sehr empfindlichen Empfänger / Restlichtverstärker ab bestimmten Helligkeiten "übersteuert" sind, d.h. durch zu viel Licht ihren Dienst versagen.

Derartige Blendwaffen werden oft auch als "Dazzler" bezeichnet, bei Verwendung eines Lasers auch als "Laser Dazzler".

Möchte man bei Sicherheitsaufgaben nicht nur den Gegner blenden, sondern z.B. in einer dunklen Nacht die Szenerie mit einem eigenen Scheinwerfer zur besseren eigenen Orientierung ausleuchten, so ergibt sich regelmäßig das Problem, dass das extrem helle Licht der Blendwaffe das eigene Licht des Scheinwerfers überblendet, so dass das Scheinwerferlicht in der Ferne nicht mehr ausreichend erkennbar ist, d.h. insbesondere die konkret geblendete Person oder das geblendete System kann wegen der Überblendung nicht hinreichend gut beobachtet werden bezüglich reaktiver Verhaltensänderungen (Ergeben, stehen bleiben, Rückzug, Umkehr etc.) oder bezüglich genereller Datenerhebung (KFZ-Nummernschilder ablesen etc.).

Darüber hinaus ist die Überblendung oft sogar so hell, dass selbst das Umfeld der geblendeten Person oder des geblendeten Systems nicht mehr ausreichend erkennbar ist, wenn mit dem Scheinwerfer das Umfeld des geblendeten Gegners ausgeleuchtet wird, um zum Beispiel verdächtige Veränderungen in der Szenerie zu beobachten (aktive Umfeld-Beobachtung).

Um hier Abhilfe zu schaffen, umfasst das System eine zweite Lichtquelle sowie Mittel zum Steuern oder Regeln der Leuchtzeiten und der Leuchtintensität der zweiten Lichtquelle derart, dass diese während der Zeiten des Zustands hoher Transmission der Flüssigkristallzelle leuchtet.

Eine solche Ergänzung ermöglicht es einem Nutzer des Systems, eine Szenerie für sich selbst auszuleuchten, während der Gegner geblendet bleibt. Die zweite Lichtquelle leuchtet zu den Zeiten, zu denen die Flüssigkristallzelle das Licht transmittiert. Die Blend-Lichtquelle beleuchtet gerade zu den komplementären Zeiten, zu denen die Flüssigkristallzelle das Licht blockiert. Der Nutzer des Systems wird also durch die Blendwaffen nicht geblendet, kann aber mithilfe des Scheinwerfers die Szenerie beleuchten und erkunden.

In einer weiteren Option ist es denkbar, dass die zweite Lichtquelle eine Anzeige ist. Der Nutzer des Systems kann dann einen Gegner blenden und selber Informationen von den Anzeigen von Instrumenten ungestört ablesen.

Um zu verhindern oder wenigstens zu erschweren, dass ein Gegner sich a) mit einem vergleichbaren System auf die Leuchtzeiten der Blendwaffe synchronisiert und genau zu diesen Zeiten seine Flüssigkristallzelle auf Blockieren schaltet (Szenario A), oder noch schlimmer, b) immer dann wenn der Dazzler aus ist, der Gegners die Vermutung hegt, dass dann die Brille des Senders offen sei und ihn genau in diesem Zeitschlitz mit eigener gegnerischen Blendwaffe blendet, kann die Regelung oder Steuerung der Flüssigkristallzelle und der Lichtquelle zum Blenden derart ausgebildet sein, dass die zeitliche Position der Zeiten des Zustands hoher Transmission innerhalb einer Periode aus Zeiten des Zustands hoher Transmission und Zeiten des Zustands niedriger Transmission kontinuierlich oder sprunghaft verändert werden kann (Phasen-Springen, "phase hopping"). Alternativ kann die Dauer einer Periode aus Zeiten des Zustands hoher Transmission und Zeiten des Zustands niedriger Transmission kontinuierlich oder sprunghaft verändert werden (Frequenz-Springen, "requency hopping"). Wichtig ist dann, dass diese Veränderungen durch einen geheimen Codierschlüssel bestimmt werden. Etwaige Muster sollten sich nicht in leicht erkennbarer Weise periodisch wiederholen.

Ein gegnerischer Selbstschutz vor Blendung (Szenario A) kann bei hinreichend schnell reagierenden Systemen (im Sinne von technologischer Waffengleichheit) mit der Codierung zwar nicht garantiert ausgeschlossen werden, da aber der Gegner hauptsächlich nur aus der "fallenden Aus-Flanke" des Dazzlers eine Vermutung* über die offene Brille ableiten kann (* = unvollständiges Wissen / Informations-Asymmetrie), kann es ihm nicht mit kontinuierlicher Sicherheit (maximaler Energie) gelingen, mit einer eigenen (gegnerischen) Blendwaffe in alle jeweils offene Zeitschlitze der Brille zu schießen, insbesondere dann nicht, wenn die Puls-Muster via Codierung nicht mehr nur synchron und komplementär sind, sondern "unlogisch springen", d.h. ein kurzer Aussetzer beim Dazzler (fallende Lichtflanke) muss eben genau nicht zwangsweise bedeuten, dass die Brille des Senders dann nachfolgend offen ist, zumal bei einem 100 Hz System immerhin 100 Zeitschlitze pro Sekunde zur Verfügung stehen, von denen nicht jeder "konsequent logisch" genutzt werden muss.

Außerdem kann der Laser Dazzler samt Lampe pro Zykluszeit T mehr als nur einen springenden "Aussetzer" bzw. "Lichtpulse" pro Zyklus produzieren (insbesondere weil Laser und LED-Lampen sich heutzutage extrem schnell modulieren lassen, z.B. Faktor 100 schneller als der LC-Shutter = 10kHz statt 100Hz). Dies führt dann zwangsläufig zu einer Täuschung und Verwirrung des Gegners, insbesondere dann wenn nicht jeder "Aussetzer" bzw. "Lichtpuls" zu einem synchronen Öffnen der Brille führt. D.h. die vorgenannte geheime Codierung kann eben auch "systemintern" angewandt werden, weil die "öffentlich ausgesendeten Informationen" (Dazzler-Aussetzer oder Scheinwerfer-Pulse) dann eben genau nicht mehr in einem logischen Zusammenhang mit der Öffnungszeit einer eigenen Brille (oder Sensors) besteht.

Das System lässt sich hervorragend kombinieren mit den weiter unten geschilderten Systemen und Verfahren zur Farbcodierung der Sicht verschiedener Personen.

Die Aufgabe wird ferner durch ein Verfahren gelöst, das einem bestimmungsgemäßen Betrieb des geschilderten Systems entspricht.

Eigenlicht-Erkennung

Die Aufgabe wird ferner gelöst durch ein System zum Sichtverbessern durch Blendunterdrückung mit einer Brille für einen Träger mit mindestens einem Auge. Die Brille hat mindestens ein Brillenglas, wobei das mindestens eine Brillenglas eine Flüssigkristallzelle aufweist, deren Transmission durch eine geeignete Ansteuerung veränderbar ist. Die Flüssigkristallzelle ist derart ausgebildet, dass die Transmission der Flüssigkristallzelle zwischen Zuständen hoher und niedriger Transmission umgeschaltet werden kann.

Das System weist ferner mindestens einen Sensor zum Messen der Helligkeit des auf den mindestens einen Sensor treffenden sichtbaren Lichts auf, wobei der mindestens eine Sensor vorzugsweise auf der vom Auge abgewandten Seite des Brillenglases angeordnet ist.

Außerdem verfügt das System über einen geschlossenen Regelkreis für die Regelung der Transmission der Flüssigkristallzelle, wobei ein Sollwert für die Helligkeit am Auge des Brillenträgers vorgegeben ist, und wobei der Regelkreis als Istwert die von dem mindestens einen Sensor gemessene Helligkeit heranzieht. Dabei ist die Regelung oder Steuerung derart ausgebildet ist, dass die Zeiten des Zustands hoher Transmission mit zunehmender Blendung kürzer werden.

Schließlich gehört zu dem System auch eine Lichtquelle mit Mitteln zum Steuern oder Regeln der Leuchtzeiten und der Leuchtintensität der Lichtquelle derart, dass diese während der Zeiten des Zustands hoher Transmission der Flüssigkristallzelle leuchtet. Dabei bleibt das zeitliche Integral des Produkts aus der Leuchtintensität der Lichtquelle und der Transmission der Flüssigkristallzelle bei einer Veränderung der Zeiten des Zustands hoher Transmission innerhalb einer vorgegebenen Toleranz konstant.

Für die Unterscheidung der Ursache des durch den mindestens einen Sensor detektierten Lichtes, also die Frage ob es sich um Licht von fremden Lichtquellen, etwa einer blendenden Lichtquelle, oder um Licht von der eigenen Lichtquelle handelt, ist es entscheidend, dass der mindestens eine Sensor die Helligkeit des auf ihn treffenden sichtbaren Lichts ausschließlich in den Zeiten des Zustands niedriger Transmission misst. Dies erlaubt die gewünschte Unterscheidung, da die gemessene Helligkeit dann nur von fremden Lichtquellen stammen kann.

Ein solches System verhindert die Blendung durch die eigene Lichtquelle.

Das System lässt sich hervorragend kombinieren mit den weiter oben geschilderten Systemen und Verfahren zur Blendunterdrückung unter Zuhilfenahme eines Eye-Trackers.

Die Aufgabe wird ferner durch ein Verfahren gelöst, das einem bestimmungsgemäßen Betrieb des geschilderten Systems entspricht.

RGB-Codierung

Die Aufgabe wird ferner durch ein System zum farblichen Kennzeichnen von Objekten im Sichtfeld von einer Mehrzahl von Brillenträgern gelöst. Das System umfasst je eine Brille pro Brillenträger mit jeweils mindestens einem Auge. Die Brillen haben jeweils mindestens ein Brillenglas, wobei das jeweilige mindestens eine Brillenglas eine Flüssigkristallzelle aufweist, deren Transmission durch eine geeignete Ansteuerung veränderbar ist. Dabei sind die Flüssigkristallzellen derart ausgebildet, dass die Transmission der Flüssigkristallzellen zwischen Zuständen hoher und niedriger Transmission umgeschaltet werden können.

Das System umfasst Mittel zum Regeln oder Steuern der Zeiten der Zustände hoher Transmission der Flüssigkristallzellen derart, dass die jeweiligen Flüssigkristallzellen zu unterschiedlichen Zeiten auf Zustände hoher Transmission eingestellt werden.

In dem System verfügt jeder Brillenträger über je eine RGB-Lichtquelle, sowie über Mittel zum Steuern oder Regeln der Leuchtzeiten, der Farbe und der Intensität der RGB-Lichtquelle derart, dass

- die RGB-Lichtquelle für einen ersten Brillenträger zu einer Zeit des Zustands hoher Transmission ($T_{on}$) der Flüssigkristallzellen (LC) seiner Brille mit einer ersten Farbe leuchtet; und
- die RGB-Lichtquelle für einen zweiten Brillenträger zu einer Zeit des Zustands hoher Transmission ($T_{on}$) der Flüssigkristallzellen (LC) der Brille des zweiten Brillenträgers mit einer zweiten, von der ersten verschiedenen Farbe leuchtet.

Auf diese Weise kann in Gruppenanwendungen mit einer Mehrzahl von Personen eine Farbcodierung von Personen oder Objektes im Sichtfeld der jeweiligen Teilnehmer durchgeführt werden, die jeweils nur der Einzelne sieht, nicht aber die anderen.

Wenn die RGB-Lichtquelle z.B. derart ausgebildet ist, dass sie geeignet ist, weißes Licht zu erzeugen, so kann dieses Licht beispielsweise in eine schnelle zeitliche Abfolge eines roten, eines grünen und eines blauen Lichtpulses zerlegt werden. Fällt nun lediglich einer dieser Lichtpulse in eine Zeit des Zustands hoher Transmission der Flüssigkristallzelle eines Teilnehmers, so sieht dieser lediglich diese Farbe. Ein Außenstehender, insbesondere jemand ohne eine Shutter-Brille würde das Licht als weiß wahrnehmen.

Mitglieder einer Gruppe, deren Zeiten des Zustands hoher Transmission der Flüssigkristallzellen miteinander synchronisiert sind, sehen die gleiche Farbe. Mitglieder einer anderen Gruppe mit anderen Öffnungszeiten der Flüssigkristallzellen sehen eine andere Farbe.

Damit die Farbcodierung geheim bzw. für Dritte unsichtbar bleibt und Außenstehender ohne Brille diese nicht wahrnehmen können, sollten in den Zeiten des Zustands niedriger Transmission der jeweiligen Brillen von den jeweils zugehörigen RGB-Lichtquellen diejenigen Farben ausgestrahlt werden, die nötig sind, um in einem zeitlichen Mittel einen weißen Farbeindruck bei denjenigen zu hinterlassen, die keine der Brillen tragen.

Um von dem Farb-Markierungen anderer Teilnehmer oder Gruppen wenigstens noch in abgeschwächter Form etwas zu sehen, kann die Flüssigkristallzelle eines ersten Brillenträgers in einer Zeit des Zustands hoher Transmission eines zweiten Brillenträgers eine abgeschwächte, aber von Null verschiedene Transmission aufweisen.

Die Farbcodierung kann dabei nicht nur in den drei Primärfarben Rot, Grün und Blau erfolgen, sondern in jeder beliebigen Farbe, die sich additiv aus Rot, Grün und Blau mischen lässt. Um die Farbe, in der die RGB-Lichtquelle für den ersten Brillenträger zu einer Zeit des Zustands hoher Transmission seiner Flüssigkristallzelle seiner Brille leuchtet, frei zu definieren, kann ein beliebiger Intensitätswert zwischen 0% und 100% eines Farbanteils jeder Primärfarbe seiner RGB-Lichtquelle zu der Zeit des Zustands hoher Transmission der Flüssigkristallzelle ausgestrahlt werden. Während zu der zugehörigen Zeit des Zustands niedriger Transmission der Flüssigkristallzellen für jede der drei Primärfarben seiner RGB-Lichtquelle der zu 100% fehlende Anteil ausgestrahlt wird.

Diese geheime farbliche Markierung kann hervorragend mit der weiter oben geschilderten Blendwaffe kombiniert werden.

Das System lässt sich ferner hervorragend kombinieren mit den weiter oben geschilderten Systemen und Verfahren zur Blendunterdrückung unter Zuhilfenahme eines Eye-Trackers.

Gleiches gilt für die weiter oben geschilderte Codierung mit einem Codierschlüssel, der es verhindern würde, dass der eventuell genutzte Farbcode von einem Gegner erkannt werden kann.

Das System lässt sich außerdem kombinieren mit den weiter oben geschilderten Systemen und Verfahren zur Verbesserung der Lesbarkeit von Anzeige-Instrumenten.

Die Aufgabe wird ferner durch ein Verfahren gelöst, das einem bestimmungsgemäßen Betrieb des geschilderten Systems entspricht.

Verstärken des räumlichen Eindrucks

Die Aufgabe wird ferner durch ein System zum Verstärken des räumlichen Eindrucks eines Objekts gelöst. Das System umfasst eine Brille für einen Träger mit mindestens zwei Augen, einem rechten und einem linken Auge. Die Brille hat je ein Brillenglas vor jedem der beiden Augen, wobei jedes Brillenglas eine Flüssigkristallzelle aufweist, deren Transmission durch eine geeignete Ansteuerung veränderbar ist. Dabei sind die Flüssigkristallzellen derart ausgebildet sind, dass die Transmission der Flüssigkristallzellen jeweils zwischen Zuständen hoher und niedriger Transmission umgeschaltet werden können. Die Brille verfügt auch über Mittel zum Regeln oder Steuern der Zeiten der Zustände hoher Transmission der Flüssigkristallzellen.

Ferner weist das System zwei Lichtquellen auf, die jeweils einem Auge zugeordnet sind, wobei die stereoskopische Basis der Lichtquellen größer als der Augenabstand ist. Hinzu kommen Mittel zum Steuern oder Regeln der Leuchtzeiten der Lichtquellen derart,

- dass die dem rechten Auge zugeordnete Lichtquelle während einer Zeit des Zustands hoher Transmission der Flüssigkristallzelle des rechten Auges leuchtet,
- während die dem linken Auge zugeordnete Lichtquelle nicht leuchtet und die Flüssigkristallzelle des linken Auges auf niedrige Transmission eingestellt ist.

Und umgekehrt.

Dieses Verfahren führt zu einer besseren 3D-Wahrnehmung, die in der Fachliteratur genau genommen als "2,5D" bezeichnet wird, da man nicht vollständig hinter das Objekt schauen kann. Die Objekte werden von einer größeren stereoskopischen Basis aus beleuchtet, und diese Beleuchtung wird von dem rechten bzw. linken Auge so wahrgenommen. Dadurch tritt der scheinbare optische Effekt auf, dass der Pupillenabstand ebenso groß wie der Abstand der beiden Lichtquellen wirkt, was die Möglichkeit der Tiefenauflösung verbessert.

Die Tatsache, dass RGB-Signale von jedem der beiden Scheinwerfer aus separat ausgesendet werden können, so dass Dritte stets Weißlicht sehen, und durch die Brille in entsprechenden zeitselektiven Ton Zeiten für jedes der beiden Auge eine bestimmte Farben sichtbar gemacht werden kann, kann das Objekt beispielsweise mit einem komplementären Farb-Saum versehen werden (z.B. rechts mit einem roten Farbsaum und links mit einem blauen Farbsaum).

Grundsätzlich muss man in der nachfolgenden Beschreibung unterscheiden zwischen physikalisch bedingten räumlichen Hervorhebungen aufgrund der erweiterten stereoskopischen Basis und sogenannten visuellen Effekten oder visuellen Hervorhebungen, die rein in der menschlichen Wahrnehmung zugrunde liegen, z.B. beschrieben durch den systemtheoretischen Übertragungskanal der visuellen Wahrnehmung. (Quelle: Systemtheorie der visuellen Wahrnehmung von Prof. Dr.-Ing. Gert Hauske, TU München, Teubner Verlag, Stuttgart, 1994).

Ein Objekt, das einen komplementären Farbsaum hat (z.B. rechts rot, links blau) kann in der visuellen Wahrnehmung etwas deutlicher hervortreten, insbesondere vor weit entfernten Hintergründen oder gar keinen Hintergründen (Objekt in freier Landschaft).

Eine weitere Verstärkung des räumlichen Eindrucks oder zumindest eine differenziertere Wahrnehmung gegen hellen Hintergrund erhält man, wenn die beiden Lichtquellen mit einer vorgegebenen, vom menschlichen Auge wahrnehmbaren Frequenz gegenphasig amplitudenmoduliert werden.

Hiermit können verschiedenen visuelle Wahrnehmungsverbesserungen erreicht werden, angefangen von a) einer einfachen visuellen "blinkenden Hervorhebung" bei hellen Hintergründen (gleichphasig und gegenphasig) - bis hin zu absichtlich evozierten visuellen Effekten, die die Räumlichkeit scheinbar verstärken, wie zum Beispiel durch den Pulfrich-Effekt (insbesondere gegenphasig bei Nacht).

Das vorgenannte Blinken (a) hat den Vorteil, dass bei Tag oder bei Dämmerung, eine zeitliche Helligkeitsvariation eines beleuchteten Objektes vor einem relativ hellen Hintergrund als kontrastverstärkend bzw. als konturverstärkend wahrgenommen wird, insbesondere wenn man sich vorstellt, dass die zwei unterschiedlichen Farbsäume des Objektes (rot rechts, blau links) wechselseitig aufblinken. Blinken ist tagsüber ohnehin stets ein gutes Mittel, um geringe Helligkeitsdifferenzen in der Wahrnehmung sichtbar zu machen - insbesondere mir der hier beschriebenen Anordnung.

Weiterhin kann insbesondere bei Dämmerung oder bei Nacht durch das gegenphasige Blinken (b) sowie andere geeignete Beeinflussungen des Übertragungskanals (rechts oder links das LC etwas mehr abdunkeln, wie beim "HDR-Sehen" oder weniger Licht auf einem Kanal senden), die "wahrgenommene Laufzeit im Gesichtskanal" (siehe oben genannte Quelle: Prof. Gert Hauske) eines Bildes oder beider Bilder verlängert werden, so dass noch zusätzlich ein Pulfrich-Effekt evoziert werden kann.

Dieses System lässt sich ohne weiteres mit der weiter oben geschilderten Farb-Codierung kombinieren.

Anstelle eines komplementären Farbsaumes (rechts rot, links blau) kann auch eine Rechts-Links-Variation einer bestimmten Hauptfarbe (z.B. rot) verwendet werden, wie oben im Abschnitt "unsichtbare Farbkodierung" erklärt - nur dass hier z.B. beim Nutzer 1 eines Teams der rechte Farbsaum hellrot und der Linke dunkelrot erscheint (oder dergleichen) - und beim Nutzer 2 eines Teams der rechte Farbsaum eines Objektes hellgrün und der linke dunkelgrün.

Zudem kann auch stets Weißlicht zur Verstärkung beigemischt werden, da alleine schon aufgrund der breiteren stereoskopischen Basis eine Hervorhebung stattfindet, insbesondere bei Objekten vor weiter hinten liegenden Hintergründen oder unendlichen Hintergründen im freien Feld.

Das System lässt sich außerdem kombinieren mit den weiter oben geschilderten Systemen und Verfahren zur Verbesserung der Lesbarkeit von Anzeige-Instrumenten und der Blendunterdrückung unter Zuhilfenahme eines Eye-Trackers.

Schließlich lässt sich das System auch kombinieren mit den weiter unten geschilderten Systemen und Verfahren zur räumlichen Separierung von Rückstreulicht (LIDAR).

Die Aufgabe wird ferner durch ein Verfahren gelöst, das einem bestimmungsgemäßen Betrieb des geschilderten Systems entspricht.

LIDAR

Die Aufgabe wird ferner durch ein System zum Verbessern der Sicht auf einen räumlich zu beobachtenden Bereich durch Blendunterdrückung gelöst. Das System umfasst eine Brille mit mindestens einem Brillenglas, wobei das mindestens eine Brillenglas eine Flüssigkristallzelle aufweist, deren Transmission durch eine geeignete Ansteuerung veränderbar ist. Dabei ist die Flüssigkristallzelle derart ausgebildet ist, dass ihre Transmission zwischen Zuständen hoher und niedriger Transmission umgeschaltet werden kann. Das System weist ferner Mittel zum Regeln oder Steuern der Zeiten hoher Transmission der Flüssigkristallzelle auf.

Zu dem System gehört auch eine gepulste Lichtquelle, die Lichtpulse aussendet. Die Lichtquelle ist derart ausgebildet, dass sie Lichtpulse erzeugen kann, deren zeitliche Dauer kürzer ist als das Licht der Lichtquelle braucht, um den räumlich zu beobachtenden Bereich in Blickrichtung des Trägers zu durchqueren.

Die Brille weist ferner Mittel zum Steuern oder Regeln der Zeiten des Zustands hoher Transmission der Flüssigkristallzelle auf, die in der Lage sind, die Zeiten des Zustands hoher Transmission der Flüssigkristallzelle zeitlich so zu legen, dass nur das Rückstreusignal des Lichtpulses aus dem räumlich zu beobachtenden Bereich von der Flüssigkristallzelle transmittiert wird.

Auf diese Weise erreicht man einen Effekt vergleichbar dem Laser basierten Messverfahren, das unter dem Namen LIDAR bekannt ist (*Light Detection And Ranging*). Der Brillenträger sieht das Rückstreulicht lediglich aus dem räumlichen Bereich, der durch die Steuerung der Brille zeitlich ausgeschnitten wurde. Damit wird das heutzutage übliche Streulicht von Nebel, Schneeflocken oder Regentropfen, die unmittelbar vor dem Scheinwerfer z.B. eines Autos sich befindenden, vermieden.

Um die Schaltzeit der Flüssigkristallzelle zu erhöhen empfiehlt es sich unter Umständen, die Fläche der Flüssigkristallzelle zu verkleinern. Eventuell bedarf es dafür eines Übergangs von einem einfachen Brillenglas zu einer Kombination aus zwei Sammellinsen, in deren Fokus eine möglichst kleine Flüssigkristallzelle angeordnet ist.

Zudem können auch spezielle Flüssigkristalle zum Einsatz kommen, wie beispielsweise Mehrfach-Schichten (Stacks) ferroelektischer oberflächenstabilisierter Kristalle (FLC), um die sehr schnellen Schaltanforderungen im Zeitbereich der Lichtgeschwindigkeit erreichen zu können.

Das System lässt sich hervorragend kombinieren mit den weiter oben geschilderten Systemen und Verfahren zur Blendunterdrückung sowie der stets ablesbaren Anzeige.

Gleiches gilt für die Verstärkung des räumlichen Sehens. Diese kann beim Autofahren zur Erhöhung der Sicherheit beitragen.

Die Aufgabe wird ferner durch ein Verfahren gelöst, das einem bestimmungsgemäßen Betrieb des geschilderten Systems entspricht.

Weitere Einzelheiten und Merkmale ergeben sich aus der nachfolgenden Beschreibung von bevorzugten Ausführungsbeispielen in Verbindung mit den Unteransprüchen. Hierbei können die jeweiligen Merkmale für sich alleine oder zu mehreren in Kombination miteinander verwirklicht sein. Die Möglichkeiten, die Aufgabe zu lösen, sind nicht auf die Ausführungsbeispiele beschränkt. So umfassen beispielsweise Bereichsangaben stets alle - nicht genannten - Zwischenwerte und alle denkbaren Teilintervalle.

Intelligente Brille mit Eye Tracker

Alle oben genannten Probleme werden mit einer "intelligenten Brille" gelöst, bestehend aus mindestens einem Brillenglas, das durch eine Flüssigkristallzelle LC realisiert ist, mit einem Closed-Loop-Echtzeit-PID-Regelkreis, vorzugsweise jedoch bestehend aus zwei völlig unabhängigen Brillengläsern und Regelkreisen des genannten Typs. Die Transmission der Flüssigkristallzelle ist durch geeignete Ansteuerung derart veränderbar, dass sie zwischen Zuständen hoher und niedriger Transmission umgeschaltet werden kann, um hierdurch einen Shutter-Effekt zu erzielen. Erfolgt dieser schnell genug, kann der Sichteindruck des jeweiligen Auges verändert werden, wobei die Trägheit der visuellen Wahrnehmung des Menschen ausgenutzt wird.

Damit sich ein Closed-Loop Regelkreis (geschlossener Regelkreis) realisieren lässt, muss pro Auge mindestens ein Fotosensor "innenliegend" sein, nämlich derart, dass er in Blickrichtung, gleichsam wie das menschliche Auge durch den Shutter hindurchschaut und somit die "tatsächliche Helligkeit" misst. Diese dient für die Regelung als "Istwert".

Definitorische Anmerkung zum vorgenannten Helligkeits-Istwert, der durch den Shutter hindurch gemessen wird, weil gegebenenfalls je nach technischer Sachverhaltsbeschreibung zwischen einem diskreten (punktuellen) Istwert auf der Zeitachse und einem Integrationsergebnis über einen kompletten Shutter-Zyklus T unterschieden werden muss:

1. Genau genommen können die heutzutage verfügbaren Fotosensoren so schnell ausgelesen werden, dass durch den Shutter hindurchtretende Lichtintensitäten auf der Zeitachse punktuell (z.B. mit Abtastfrequenzen im Mikrosekundenbereich) gemessen werden können, ähnlich wie bei einem digitalen Speicher-Oszilloskop mit optischem Messkopf, so dass ein diskreter Istwert-Verlauf in einem flüchtigen Speicher des Mikrocontrollers hinterlegt werden kann. In diesem Verlauf lässt sich genau erkennen, wann der Shutter innerhalb eines Pulsweitenmodulations(PWM)-Zyklus T jeweils geöffnet ($T_{on}$ bzw. transparent) und wann er geschlossen ($T_{off}$ bzw. intransparent) ist. Läuft das Shuttersystem beispielsweise mit einer Grundfrequenz von 100 Hz, so beträgt die zeitliche Speichertiefe 1/100 Hz = 10 ms. Der Mikrocontroller kann am Ende eines Zyklus rein mathematisch ein Integral über diesen Helligkeitsverlauf bilden und somit den "Istwert" eines Zyklus liefern.

2. Andererseits könnte derselbe Fotosensor ebenso über den gesamten Zyklus T, also über die vorgenannten 10 Millisekunden, physikalisch und elektronisch bzw. schaltungstechnisch bedingt derart integrieren, dass exakt am Ende des Zyklus T ein Messergebnis vorliegt, welches dann vom Mikrokontroller ausgelesen wird, ohne dass dieser eine mathematische Mittelung leisten muss. In der hier vorliegenden Erfindung wird zur Messung des Istwertes ein Fotosensor verwendet, der die schnelle punktuelle / diskrete Messung ermöglicht. Um Missverständnisse zu vermeiden, wird vorliegenden im Text in der Regel der Begriff "Istwert" verwendet, wenn ein über die Zykluszeit T umgerechneter oder integrierter "Grauwert" (mittlere durchtretende Helligkeit im Zyklus T) gemeint ist - zumal der Mensch ebenfalls nur Grauwerte wahrnimmt, auch wenn in Wirklichkeit nur zeitliche Verhältnisse von $T_{on}$ zu $T_{off}$ durchgelassen werden.

Der Fotosensor nimmt damit quasi die Rolle des Auges ein, misst also stellvertretend für das Auge die "echte Helligkeit", die in das Auge fällt, nicht nur irgendeine zufällig äußere Helligkeit. Das Auge wird bei einer ON-OFF-Keying-PWM insofern als Tiefpass benutzt, als dass nur im Auge bzw. nur in der menschlichen Wahrnehmung die Grauwerte entstehen, während die Brillengläser aber in Wirklichkeit niemals Grauwerte annehmen. In Analogie zu den oben aufgeführten Integrations-Szenarien zum Istwert (1. und 2.) kann streng genommen noch ein drittes Szenario definiert werden, indem der Mikrocontroller und/oder der Fotosensor so lange integriert, bis ein Grauwert erreicht wird, der auch vom Menschen tatsächlich als Grauwert wahrgenommen werden kann (z.B. nach Integration über etwa 250 bis 500 Millisekunden). Sollte dieser wahrnehmbare Istwert gemeint sein, so wird dies im Text in der Regel gesondert kenntlich gemacht.

Der Fotosensor bzw. Helligkeitssensor hat einen gewissen Abstand (typischerweise 1-3 mm) zur LC-Zelle, so dass aufgrund seines Öffnungswinkels die effektiv betrachtete LC-Fläche größer ist als die Chipfläche des Sensors. Dies führt zu einer besseren Mittelung der Helligkeit und einer genaueren/stabileren Messung im Falle von punktueller "LC-Domainbildung", oder im Falle von punktueller Verschmutzung auf der Gegenseite der LC-Zelle. Aus Sicherheitsgründen und aus thermischen Gründen ist es ohnehin zweckmäßig, ein äußeres Schutzglas, welches auch das äußere Design der Brille ausmacht, in einem Abstand von 1-3 mm vor der LC-Zelle anzubringen. Damit haben solche punktuellen Verschmutzungen (kleine Fliegen, Staubkörner, etc.) keinen Einfluss auf das LC und schon gar keinen Einfluss auf den Fotosensor mehr. Zudem werden die innenliegenden Fotosensoren (sofern es sich um klassische, also nicht transparente, Fotosensoren handelt) im äußeren LC-Randbereich bzw. Brillen-Rahmenbereich angebracht, so dass diese nicht das Sehfeld stören.

Um aber den Helligkeits-Istwert in der Mitte des LC-Shutters oder genauer, in der statistischen Ortsmitte der Pupille bei Geradeaussicht, möglichst genau zu bestimmen zu können, werden mindestens zwei, besser drei Fotosensoren pro Auge eingesetzt, deren Positionierung ein Koordinatensystem aufspannt. Beispielsweise können sie in einem Dreieckangeordnet sein, auf dessen Ecke der statistische Ortsmittelwert der Pupille zu liegen kommt, der in der Regel (d.h. bei nicht schielenden Menschen) identisch ist mit dem Punkt der Geradeaussicht. Mit Hilfe einer Triangulations-Berechnung lässt sich dann die mittlere Helligkeit in Bezug auf diesen statistischen Ortsmittelwert bzw. die Geradeaussicht berechnen und als "Istwert" für die Regelung heranziehen.

Zudem haben mehrere innenliegende Fotosensoren pro Auge den Vorteil, dass durch diese Redundanz die Mess-Sicherheit erhalten bleibt, auch bei Verschmutzung oder bei verstärktem punktuellem Lichteinfall (z.B. zufällige helle Reflexion auf nur einen von drei Fotosensoren).

Für die Regelung wird ein "Sollwert" benötigt, der zunächst mit einer Art Potentiometer oder vergleichbarem "Einsteller" so vorgegeben wird, dass das Auge konstant dunkeladaptiert bleibt, ähnlich wie bei einer relativ starken Sonnenbrille, z.B. mit Schutzstufe III (S3, 8-18% Transmission).

Der Regelkreis muss derart schnell sein, dass der Regelvorgang vom menschlichen Auge nicht mehr wahrgenommen werden kann, so dass die am Auge eintreffende Helligkeit stets konstant ist (dem Sollwert entsprechend), egal wie sich außen die Helligkeit ändert.

Es handelt sich also um einen sogenannten Echtzeit-Regelkreis, bei dem im eingeschwungenen Zustand (richtige PID-Parametrierung) das sogenannte Delta (Regelabweichung) - also die Differenz zwischen Sollwert und Istwert - stets Null ist.

Eine solche Regelung funktioniert jedoch nur, wenn die Brille absolut lichtdicht in Bezug auf Licht von außen ist. Das Brillengehäuse ähnelt daher einer Taucherbrille, Skibrille oder einer eng anliegenden Arbeitsschutzbrille mit weichen staub- und lichtdichten Augenmuscheln im Stile einer Schwimmbrille oder einer großen Brille mit breiten Seitenbügeln und Lichtschutz von oben und unten. Mit Hilfe eines elektrischen Potentiometers oder eines ähnlichen Einstellers lässt sich die Pupille des Trägers der Brille a) langsam öffnen, sogar regelrecht "aufdrehen", bis diese zu 75% über Normaldurchmesser bei Tageslicht geöffnet ist; und b) aufgrund der Echtzeitregelung konstant auf diesem Durchmesser halten, so dass sie quasi "ruhig gestellt" wird, egal wie sich die Helligkeit draußen ändern mag.

Dies geschieht für jedes Auge getrennt, wenngleich in der Startroutine zunächst jedes Auge den gleichen Sollwert eingestellt bekommen kann (z.B. 100 Lux am rechten (R) und linken (L) Auge). In der Praxis werden die Sollwerte R und L vergleichsweise langsam geändert (z.B. 2- bis 100-mal langsamer als die Helligkeitsregelung) und auch bewusst mit leichten Differenzen beaufschlagt (z.B. links 10% mehr Transparenz und rechts 10% weniger Transparenz). Die Gründe dafür werden nachfolgend erklärt.

Mindestens ein Außensensor pro Auge (OL, OR) erfasst grob und vergleichsweise langsam (z.B. binnen 1-2 Sekunden) die Tageslichtsituation im zeitlichen Mittel und stellt fest, ob ein heller Tag, bedeckter Tag oder eine Innenraumsituation vorliegt. Dies ist notwendig, weil der Dynamikumfang bezüglich der Beleuchtungsstärke tagsüber einen Bereich von 100 Lux bis 100.000 Lux umfasst, also etwa einen Faktor 10.000, während eine einfache LC-Zelle nur einen Faktor 1000 bis 5000 (Kontrastverhältnis) umfasst. Durch einen veränderlichen Sollwert, der vom Außensensor bestimmt wird (heller Tag, bedeckter Tag, ...) wird daher der "Arbeitspunkt" der LC Zelle während einer Initialisierungsroutine beim Einschalten in den richtigen Bereich geschoben (z.B. an einem sehr hellen Tag von initial 100 Lux am Auge auf 300 Lux am Auge).

Dieser vom Außensensor initiierte Sollwert wird auch dann schnell und dynamisch geändert, wenn der Regler am unteren oder oberen Anschlag ist, also die Regelabweichung nicht mehr Null sein kann, weil die Regelgröße an der LC-Zelle bzw. die Transmission einen nicht mehr steigerbaren Wert erreicht hat (d.h. ganz auf oder zu).

Dies sollte nicht regelmäßig der Fall sein, da vorgesehen ist, das Auge permanent dunkeladaptiert zu halten. Wenn sich die Lichtsituation insgesamt verändert, kann jedoch unter Berücksichtigung von elektronisch hinterlegten Erfahrungswerten sowie Informationen von den Außen- und Innensensoren, kurz vor Erreichen des Regler-Anschlags in eine bestimmte Richtung (LC ganz zu oder ganz auf) der Sollwert so geändert werden, dass der Regler weiterhin im "Regelbetrieb" bleibt und diesen Anschlag nicht wirklich erreicht, d.h. sich im weitesten Sinne logarithmisch oder ähnlich nichtlinear verhält - aber aufgrund der erhöhten durchtretenden Helligkeit (z.B. beim direkten Blick in die Sonne) ein sanftes und kontrolliertes Schließen der Iris ermöglicht. Diese Nachregelung des Sollwerts zur Erweiterung des Dynamikumfangs sollte allerdings nur in seltenen Ausnahmefällen auftreten; im Normalbetrieb wird die Pupille auf einen festen relativ dunklen Wert eingestellt (z.B. 75% über Normaldurchmesser), damit das bereits dunkeladaptierte Auge bei Eintritt in einen dunklen Raum sofort (d.h. z.B. innerhalb einer Millisekunde) zur Verfügung steht.

Zudem können die beiden Sollwerte (L und R) leichte Differenzen aufweisen, z.B. 5% bis 30% mehr Transparenz links als rechts, damit das Gehirn aus den beiden leicht unterschiedlichen Bildern in der Wahrnehmung unmerklich wieder ein Bild mit höherem Kontrastumfang (Dynamikbereich) machen kann (aus der Fotografie als HDR = "high dynamic range" bekannt - zwei unterschiedlich belichtete Fotos werde ineinander kopiert). Voraussetzung ist, dass der Kontrastunterschied nicht zu extrem wird, also für den Menschen unmerklich bleibt, z.B. 1% bis 60%, bevorzugt 5% bis 30%. Nicht ausgeschlossen werden auch höhere Werte > 30%, die aber dann zeitlich kürzer eingeblendet werden, derart, dass das Gehirn daraus trotzdem unmerklich ein neues Bild mit höherem Kontrastumfang konstruieren kann. Hierbei wird also mittels intelligenter Software-Algorithmen in die menschliche Wahrnehmung eingegriffen.

Zudem können in der Brille, wie heutzutage bei sog. "wearable Technology" und Smartphones üblich, auch Neigungs- und Beschleunigungssensoren integriert sein, so dass z.B. bei schneller Fahrt solche Helligkeits-Differenzen automatisch reduziert oder sogar abgeschaltet werden können, um beispielsweise unerwünschte Effekte zu vermeiden (z.B. Pulfrich-Effekt oder andere Wahrnehmungs-Artefakte).

Die höchste und komplexeste Form dieser Art von elektronischer Ansteuerung besteht in der Berücksichtigung der rechts-links kontralateralen Pupillenafferenz bei einer "Swinging-Flashlight-Test" (SWIFT) ähnlichen Beleuchtungssituation, die physiologisch über den überkreuzenden Links-Rechts-Nervensignalaustausch im Chiasma opticum und in nachfolgenden Teilen des Gehirns erfolgt. Konkret bedeutet dies, dass bei exakt gleich eingestellten elektronischen Sollwerten für beide Augen (L=R=const.) beim gesunden Menschen ohne Asymmetrien in der kontralateralen Pupillenafferenz (wie z.B. beim relativen afferenten Pupillendefekt RAPD) im Regelbetrieb keine neuronalen Reize über Kreuz ausgetauscht werden, da die Helligkeit auf beiden Augen stets konstant ist. Es gibt drei Arten, diesen Effekt auszunutzen:

1) Ein erhöhtes Stellsignal (z.B. verstärkte Abdunkelung) auf einem Kanal (L oder R), bei jeweils identischen Sollwerten (R=L=const.) signalisiert eine asymmetrische Beleuchtungssituation, z.B. übermäßig viel Außenlicht auf dem betreffenden Kanal. Der Mikrocontroller dieses Kanals teilt dem anderen Mikrocontroller oder der State-Machine des anderen Kanals das nahezu Erreichen oder Übersteuern des Kanals mit. Die unterbelichtete Seite kann dann aufmachen.

2) Absichtliches Betreiben in dem HDR-Differenzmodus kann dazu führen, dass derjenige Kanal, der heller (transparenter) geschaltet wird, insbesondere wenn dieser zu schnell und zu umfänglich transparent geschaltet wird (delta t, delta T relativ hoch), eine kontralaterale Pupillenkontraktion auf dem anderen Kanal hervorruft. Um diesem Effekt zu berücksichtigen (zu kompensieren = Gegenkopplung oder ggf. absichtlich zu verstärken = Mitkopplung), wird der andere Kanal in sanfter und geeigneter Weise so gesteuert, dass es zu einer verbesserten Sicht für das andere

15

Auge kommt, aber ohne dass es zu einer erneuten kontralateralen Übertragung auf den ursprünglich beeinflussten Kanal kommt. Dazu liegt eine Dämpfung vor, um ein Aufschaukeln des Systems aus beiden Pupillen und beiden softwaregesteuerten Kanälen zu verhindern. Hierbei werden die Außenlichtsituation, die Arbeitspunkte der beiden Regler, die Transienten/Beleuchtungsänderungen auf den jeweiligen Kanälen (z.B. heller Tag, bewölkter Tag, Nähe zum Regleranschlag) und die Differenz zwischen den Reglern berücksichtigt.

3) Medizinische und psycho-pathologische Indikationen:

a) Für Patienten mit einem relativen afferenten Pupillendefekt (RAPD) kann in der Software des Mikrocontrollers das rechts-links Pupillenverhaltensmuster des Patienten hinterlegt werden, so dass beim Betrieb innerhalb der beiden oben genannten Modi (1 und 2) deren Pupillenverhalten mit der jeweils korrekten LC-Transparenz derart berücksichtig wird, dass die wahrgenommenen Helligkeit immer konstant ist oder bestimmten gewünschten Sollwerten entspricht.

b) Für Patienten mit einem ärztlich verordneten rechts-links Sehtraining (z.B. nach einem Schlaganfall), kann optional eine Seite permanent oder nach bestimmten zeitlichen Mustern wechselweise dunkler oder heller geschaltet werden.

c) Für Einsatzkräfte in Stresssituationen (z.B. Soldaten im Einsatz), die einen akut erhöhten Adrenalinpegel und somit generell geweitete Pupillen haben, kann die Software per Befehl (Taster) die Transmission entsprechend verringern (leicht abdunkeln), damit die visuelle Wahrnehmung bei Helligkeit angenehmer ist.

Die innenliegenden Fotozellen sind mindestens doppelt oder gar dreifach ausgelegt. Dies dient nicht nur zur Berechnung der mittleren Helligkeit im wahrscheinlichsten Ortsmittel der Pupille (wie oben beschrieben), sondern auch aus Sicherheitsgründen. So kann die Software durch logischen Vergleich (z.B. zwei Sensoren zeigen ähnliche Helligkeit und nur einer zeigt gar keine Helligkeit) ggf. eine Verschmutzung oder einen Defekt eines bestimmten Fotosensors erkennen und als Konsequenz nur noch die beiden funktionieren Fotosensoren berücksichtigen.

Dazu enthält die Software zusätzlich zu den permanent berechnenden Regler-Komponenten auch rein logische Sicherheitsroutinen (separate State-Machines), die ständig parallel zum Regeln die Funktion der Brille sicherstellen. (In diesem Zusammenhang sei noch angemerkt, dass die fehlerunanfälligsten Brillen dieser Art, die für automotive Anwendungen gedacht sind, entsprechend der ASIL-Norm zugelassene Dual- oder Tri-Core Prozessoren beinhalten, die sowohl Hardware als auch Software auf Fehler prüfen.

Eye Tracker einfacher Art

In Analogie zu den oben genannten Fotosensoren oder Kameratypen, die ein menschliches Auge simulieren, wird im inneren der Brille, wo sich dieser Sensor befindet, ein zweiter Sensor platziert, der das Auge beobachtet. Dieser könnte z.B. auf der Rückseite des vorgenannten Sensors oder auch leicht versetzt daneben montiert sein. Es können diverse Arten von Sensoren eingesetzt werden, z.B. relativ einfache und kostengünstige Fotosensoren, oder Facettensensoren, oder höher auflösende abbildende Kamerasysteme. Im einfachsten Fall wird die Blickrichtung nur grob erfasst. Insbesondere die links-rechts-Bewegung des Auges lässt sich sogar am weißen Teil des Auges (Lederhaut) einfach detektieren, indem eine codierte Infrarot-Lichtschranke eingesetzt wird. Infrarotlicht wird nicht vom Auge wahrgenommen, aber je nach Blickrichtung unterschiedlich reflektiert. Eine Codierung der IR-Quelle ist notwendig, damit es nicht zur Verwechslung mit anderen Lichtquellen und Reflexionen auf der Empfängerseite kommt. Diese Codierung kann im einfachsten Fall zyklisch sein (z.B. 10 kHz Rechteck mit bekannter Frequenz und Phasenlage). Aus der Frequenz und insbesondere der Phasenlage kann ein Phase-Sensitive Detektor (PSD, auch Boxcar-Amplifier genannt) nach Tiefpass-Integration über ca. 10 Zyklen, also mit immerhin ca. 1 kHz eine sehr genaue Amplitudenmessung bezüglich des Sendersignals machen, selbst dann wenn dieses gegenüber dem "Rauschen" anderer IR Signale sehr schwach ist.

Dies ist nur ein Beispiel für einen einfachen Eye Tracker. Man kann auch die Pupillenstellung mit einem sehr ähnlichen Verfahren ermitteln - ebenfalls in Reflexion, aber dann bezüglich der Absorption in der dunklen Pupille anstelle der Reflexion an der weißen Lederhaut. Da Reflexions-Lichtschranken sehr kostengünstig sind, können durchaus sowohl innen am Auge (Nasennähe) als auch außen am Auge (Schläfennähe) solche Sensoren angebracht werden, ggf. auch noch mittig unter dem Auge (Blick nach oben/unten erfassend) - also durchaus 2 bis 3 Sensoren. Mehrere solcher Sensoren erhöhen die Messgenauigkeit bezüglich der Blickrichtung.

Idealerweise wird jedoch ein Eye Tracker verwendet, der eine winzige hochauflösende abbildende Kamera verwendet, ähnlich wie sie in Smartphones oder Notebooks verwendet werden. Diese Kamera erfasst die Pupillenstellung bezüglich Blickrichtung und somit aller Winkel.

Korrelationsberechnung aus Fotosensoren und Eye Tracker

Die Richtungs- und Helligkeitsinformationen der Fotozellen/Kamera werden mit der durch den Eye Tracker bestimmten Blickrichtung per Software mathematisch korreliert. Dies bedeutet beispielsweise, dass zunächst die Blickrichtung als Ausgangsgröße genommen wird, während zeitgleich (d.h. in Echtzeit) die einfallende Helligkeit unter dem exakt gleichen Winkel gemessen und konstant geregelt wird. Da es sich um einen Echtzeit-PID-Regelkreis handelt, bei dem die Regelabweichung stets Null ist, wird die Helligkeit in Blickrichtung stets konstant sein - nämlich dem eingestellten Sollwert entsprechend.

Wenn diese Regelung sehr exakt funktioniert, was unter Einsatz von Hochtechnologie möglich wäre, würde die Pupille auf der Hauptachse niemals einen Helligkeitsunterschied erfahren. Dieser Regelmodus kann je nach Anwendung (z.B. Sport, Automotive, Industrie, Medizin, Militär) gewählt werden, z.B. durch einen Schalter oder sonstigen Befehl (z.B. über ein Smartphone, welches über Bluetooth o.ä. mit der Brille verbunden ist).

Andererseits könnte dieser extrem schnelle und präzise Regelmodus je nach Anwendung auch zu unerwünschten Artefakten in der Wahrnehmung führen. Daher ist ein alternativer Modus einstellbar, in dem die Software absichtlich etwas verlangsamt wird bzw. die Helligkeit nur in leichten Winkelabstufungen nachgeregelt wird. Beispielsweise würde erst dann, wenn der Anwender wirklich genau in eine eher seitlich liegende Blendquelle (z.B. Autogegenverkehr) schaut, sofort auf konstante Helligkeit geregelt, ansonsten, wenn sich die Pupille nur in geringem Maße in der Mitte hin und her bewegt und aus der Region kein Gegen-Blendlicht rührt, wird nur auf diese Helligkeit konstant geregelt.

Außerdem wird die individuelle und altersabhängige Blendempfindlichkeitsfunktion, welche in der Software als Formel oder Look-Up-Table (LUT) hinterlegt sein kann, wie eine Schablone (z.B. mit multiplikativer Gewichtung) über das Signal des nach vorne schauenden Helligkeitssensors gelegt. Obwohl sich dieser Sensor nicht wie ein Augapfel bewegt, sondern starr geradeaus montiert ist, wird aufgrund des Eye-Tracker-Signals diese Schablone gemäß der Augapfel-Bewegung mitverschoben. Damit ist praktisch ein künstliches Auge geschaffen, welches die individuelle blickwinkelabhängige Blendempfindlichkeit berücksichtigt, welche dem Echtzeit-PID-Regelkreis als Führungsgröße (inwendig ebenfalls "Istwert" genannt) dient. Dabei bleibt es dem Fachmann überlassen, die Algorithmen je nach vorgesehener Anwendung sanfter oder stärker auszuprägen. Alternativ kann vorgesehen sein, dass der Anwender dazu eine Auswahl trifft.

allgemeines System zur Blendunterdrückung

Hier geht es um ein System zur Sichtverbesserung durch Blendunterdrückung (auch Anti-Blend-System genannt), das intelligente und sicherheitsrelevante Mehrkanal-Echtzeit-Regelungen zur Sichtverbesserung realisiert, wobei das linke und rechte Auge getrennt behandelt werden, und/oder welches mehrere Nutzer für Gruppenanwendungen einbeziehen kann.

Um ein konsistentes Gesamtsystem, bei dem das Visor und die Scheinwerfer kontinuierlich und analog derart zusammenwirken, dass ein Anwendungsbereich von völliger Dunkelheit (0 Lux) bis hin zu Dämmerung (z.B. 100 Lux) nahtlos abgedeckt wird, und welches gleichzeitig alle lichttechnischen Normen (z.B. konstante integrale Helligkeit der Scheinwerfer) erfüllt, zu erhalten, wird eine in Echtzeit auf konstante Helligkeit geregelte Brille, wie sie oben beschrieben wurde, benötigt, wobei in vielen Fällen eine etwas vereinfachte Ausführung (ohne Eye Tracker) ausreichend ist. Eine solche Brille ermöglicht, Blendung durch konstantes Regeln auf einen Helligkeitswert zu unterdrücken. Zudem wird das Auge permanent ziemlich dunkeladaptiert gehalten (d.h. es wird eine relativ große Pupille eingestellt), so dass der Anwender beim Durchqueren eines Hell-Dunkel-Sprungs (z.B. Einfahrt in einen dichten Wald) sofort und unmerklich (in Echtzeit) dunkeladaptiert ist, was sonst bis zu einer Minute oder länger dauert. Problematisch ist jedoch, dass mit zunehmender Verdunklung durch die Brillengläser (d.h. zunehmende Helligkeit draußen) der Kontrastumfang bzw. der Quotient aus Nutzsignal und Störsignal abnimmt.

Um dieses zu beheben, wird noch ein synchron laufender Scheinwerfer benötigt (der also mit derselben Frequenz wie die Brille arbeitet). Die Pulsenergie soll dabei pro ausgesendeten Lichtpuls weitgehend konstant bleiben. Dazu wird das zeitliche Integral des Produkts aus der Leuchtintensität des Scheinwerfers und seiner Leuchtdauer während eines Zyklus möglichst konstant gehalten.

System zur Blendunterdrückung mit Anzeige

Der Einsatz- und Anwendungsbereich von Anti-Blend-Systemen lag bisher hauptsächlich bei der motorisierten Fortbewegung (Autos, Motorräder, Züge, usw.) oder bei schneller Fortbewegung aus Eigenkraft (Fahrräder etc.), weil in der Regel davon ausgegangen wurde, dass die Blendung in erster Linie durch die Scheinwerfer entgegenkommender Fahrzeuge oder durch tief stehende Sonne oder sonstige störende Lichtquellen verursacht wird. In solchen Szenarien wird davon ausgegangen, dass das Störsignal (z.B. Gegenverkehr oder Sonne) und das Nutzsignal (eigene Scheinwerfer) aus völlig anderen Richtungen kommen (Sonne in der Ferne, Scheinwerfer am Auto). Eine etwas andere Situation ergibt sich, wenn das Störsignal (Sonne) genau an der Stelle reflektiert wird, wo ein Nutzsignal entsteht, z.B. auf einer reflektierenden BildschirmOberfläche.

Beide Situationen haben jedoch gemeinsam, dass das Summensignal am Auge stets aus Störsignal und Nutzsignal bestehen. In Bezug auf die Kombination Brille-Auge ändert sich also physikalisch gesehen nichts, da in Richtung menschlicher Wahrnehmung stets ein Nutzsignal von einem Störsignal durch Zeitmultiplex unterschieden und das integrale Verhältnis von Nutzsignal zu Störsignal verbessert werden soll. Außerdem kann in beiden Fällen das Störsignal auch aus einer anderen Richtung als der Blickrichtung des Nutzers kommen, aber auch ein solches Störsignal kann so stark blenden, dass die Sicht im Betrachtungspunkt beeinträchtigt ist.

Unter Anzeigesysteme fallen alle Arten von Bildschirmen, Displays (PC, Notebook, SmartPhone, TV, ...), Armaturen oder sonstige visuelle Mensch-Maschine-Schnittstellen, z.B. Cockpit-Armaturen aller Art, z.B. im Auto, Flugzeug, Schiff, Motorrad, etc., oder sonstige selbstleuchtende Anzeigetafeln, Warnschilder, Tachos, Uhren, Geokoordinaten-Navigationssysteme, Head-Up-Displays usw.

Abhilfe wird erreicht, indem die Anzeigenbeleuchtung so moduliert wird, als wäre es der vorgenannte Eigenlicht-Scheinwerfer. D.h. immer dann, wenn die Flüssigkristallzelle der Brille in einem kurzen Zeitschlitz geöffnet ist (z.B. in nur 5% der Zyklus-Zeit T), wird auch die Hintergrundbeleuchtung des Displays kurz und pulsartig, vorzugsweise mit höherer Leuchtintensität als normal, eingeschaltet.

Der vorgegebene Wert der Helligkeit des Displays, der benötigt wird, damit ein Nutzer die auf dem Display angezeigten Informationen lesen kann, ergibt sich einerseits aus der Helligkeit, auf die die Brille das auf das Auge treffende Licht regelt, z. B. 400 lx, und andererseits aus der üblichen Helligkeit dieses Displays. Da die Brille typischerweise auf 400 lx regelt, also einen eher dunklen Zustand, liegt der vom Produkt aus $T_{on}$ und der Helligkeit des Displays möglichst zu erreichende Wert im Allgemeinen unterhalb der gewöhnlichen Helligkeitseinstellung des Displays. Dies lässt Raum für die benötigte pulsartige Überhöhung. Diese ist immer dann problemlos möglich, wenn die Hintergrundbeleuchtung aus schnell reagierenden Lichtquellen (z.B. Weißlicht- oder RGB-LEDs) besteht, die sich wiederum per a) Software oder per b) OEM-Hardwarelösung ansteuern lassen.

a) Im einfachsten Fall kann bereits eine aus dem Internet herunter geladene Software (z.B. eine sog. App) die Display-Hintergrundbeleuchtung eines Smartphones oder ähnlichen Geräts, wie z.B. Tablet oder Notebook oder ein außerhalb der Brille befindliches Head-Up-Display, in ihrer Helligkeit derart modulieren, dass das oben beschriebe Anti-Blendsystem realisiert wird.

b) Ansonsten kann erwartet werden, dass sich Endgerätehersteller von Smartphones und Tablets mittelfristig (d.h. bereits nach ein paar Jahren) auf ein solches System einstellen, indem sie spezielle kurz überpulsbare Hintergrundbeleuchtungen in ihre Geräte einbauen werden. Nachdem ohnehin etwa im Jahresrhythmus neue Consumer-Endgeräte im Markt erscheinen, kann diese Möglichkeit als realistisch angesehen werden. Und im Falle von speziellen Anzeigen für Nicht-Consumer-Geräte (Flugzeug-Cockpit etc.) ist es ohnehin naheliegend, über Kooperationen solche Spezialsysteme problemlos in die nächste Generation von Anzeigen einzubauen.

Durch ein solches System wird z.B. über 95% des Blendlichtes unterdrückt, während die Lichtpulse des Bildschirms genau in den offenen Zeitschlitz der Brille und damit auf das dunkeladaptierte Auge fallen. Dadurch werden die angezeigten Informationen trotz erheblicher Sonneneinstrahlung (oder sonstiger Störlichteinstrahlung) deutlich sichtbar, während die Anzeige ohne solch ein System nicht lesbar wäre.

System zur Blendunterdrückung mit Blendwaffe

Begriffsdefinition:

Das hier allgemein verwendete Wort "Blendwaffe" oder (Englisch) "Dazzler" dient hier nur nur als Oberbegriff, d.h. es ist unerheblich, um welche lichttechnische Realisierung (Lampe, Laser etc.), Wellenlänge oder Intensität es sich handelt, so dass auch LASER-Dazzler mit sehr hoher Strahlintensität oder LASER mit variabler Wellenlänge (multicolor) oder sonstige hoch intensive Lichtquellen - auch im Randbereich zum Infrarot (IR) oder Ultraviolett (UV) - hier einbezogen werden. Allen Blendwaffen gemeinsam ist die Idee zur explizit aggressiven taktischen Blendung und Störung von Gegnern (egal ob es sich um eine Einzelperson oder eine Gruppe handelt), oder auch zur Blendung und Störung von durch den Gegner eingesetzten optoelektronischen Systemen (z.B. Sensorsysteme auf Panzern o.ä.).

Nach aktuellem Stand der Technik überblendet das extrem helle Licht einer eigenen Blendwaffe das eigene Scheinwerferlicht, so dass dieses in der Ferne nicht mehr ausreichend erkennbar ist, und zwar selbst dann nicht, wenn mit dem/den Scheinwerfern das Umfeld des geblendeten Gegners ausgeleuchtet wird, um zum Beispiel verdächtige Veränderungen in der Szenerie zu beobachten (jenseits des bereits erfolgreich Geblendeten, sog. aktive Umfeld-Beobachtung).

Das vorliegende Blendunterdrückungssystem kann mit einer solchen Blendwaffe (Dazzler) kombiniert werden. Bei dieser Ausgestaltung wird der Blendwaffe ein bzgl. der Öffnungsdauer $T_{on}$ der Brille antizyklisches bzw. invertiertes Einschaltsignal zugeführt. Die Blendwaffe wird also immer nur für den jeweils sehr kurzen Zeitschlitz (z.B. 5% der cw-Dauerstrichzeit des Dazzler) ausgeschaltet, in dem der Suchscheinwerfer eingeschaltet und die Brille synchron dazu kurz offen ist. Sobald die Brille wieder schließt (intransparent geschaltet wird), geht die Blendwaffe wieder an. Dies ermöglicht insgesamt einen getrennten Zweikanalbetrieb (von Scheinwerfer und Dazzler).

Wird der Dazzler in den kurzen offenen Zeitschlitzen der Brille komplett ausgeschaltet, kann dies Nachteile haben, da er dann visuell nicht mehr verfolgbar ist. Deshalb kann der Dazzler für diese Zeiten auf eine frei einstellbare, niedrige Leuchtintensität in Höhe von beispielsweise 0,5-5% seiner maximalen Intensität festgelegt werden, damit er für den Anwender gut sichtbar bleibt und nicht versehentlich so stark unterdrückt wird, dass nicht mehr ausreichend erkennbar ist, wohin die Blendwaffe strahlt.

Mit dem einem solchen Zweikanal- oder sogar Mehrkanalbetrieb, bestehend aus individueller Eigenlichtquelle und mindestens einer individuellen Blendwaffe, ist es möglich, gegnerische Personen oder deren optische Ausrüstung (z.B. Sensoren auf einem Panzer) zu blenden, aber auch gleichzeitig die benachbarte Umgebung in Blickrichtung mit der separaten Eigenlichtquelle auszuleuchten/auszukundschaften

In Kombination mit weiter unten beschriebenen Ausgestaltungen des erfindungsgemäßen Systems ist es sogar möglich, gegnerische Ziele für Mitglieder eines Teams (und nur für diese) farblich zu markieren und das System verschlüsselt zu betreiben.

System zur Blendunterdrückung mit Codierung

Insbesondere für den Einsatz bei Behörden und Organisationen mit Sicherheitsaufgaben (BOS) oder beim Militär ist eine Gruppenanwendung vorgesehen. Diese muss sicherstellen, dass die Akteure sich nicht versehentlich gegenseitig bzw. untereinander blenden. Dazu sind die Komponenten des Systems miteinander synchronisiert. Da nicht ausgeschlossen werden kann, dass es außenstehende Nutzer ähnlicher Systeme gibt (egal ob es sich dabei um Gegner oder um andere Teams mit ähnlichem Auftrag handelt), ist vorgesehen, die Komponenten des Systems (z.B. Brille und Lichtquellen) so zu modulieren, dass die ausgesendeten kurzen schmalen Lichtpulse und die entsprechend synchronen kurzen Öffnungszeiten der Brille keinem zyklischen bzw. periodischen Muster mehr entsprechen, sondern sich ihre zeitliche Abfolge gemäß eines geheimen Codier-Schlüssels ständig ändert. Diese Änderung kann grundsätzlich bezüglich aller denkbaren freien Modulationsparameter erfolgen, vorzugsweise aber bezüglich ihrer Phasenlage, Puls-Position (Phase- and Pulse-Position Hopping), Frequenz (Frequency-Hopping), Amplitude (AM) oder einer Kombinationen aus diesen Modulationsverfahren.

Eine solche Codierung kann selbstverständlich auch auf die vorstehend beschriebene Ausgestaltung des Systems mit Blendwaffen angewendet werden. In einem solchen Fall "springt" auch die Blendwaffe entsprechend mit den geheim codierten Zeitschlitzen der Brille und der Eigenlichtquelle auf der Zeitachse hin und her - nur eben jeweils invertiert.

Denkbar sind auch Abstufungen von Codier-Schlüsseln, z.B. könnten mittels eines Sub-Schlüssels (ggf. vererbt vom Team-Schlüssel) pro Person oder pro Team noch eine oder mehrere weitere Blendwaffen (Dazzler) gesondert verschlüsselt werden, ohne dass man sich versehentlich gegenseitig blendet.

Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente. Im Einzelnen zeigt:

Fig. 1     eine schematische Darstellung in einer Schnitt-Aufsicht der elektronischen Brille;
Fig. 2     ein Diagramm der sog. Transmission der Brille eines Blendunterdrückungssystems über die Zeit, wobei das System mit einer Blendwaffe ausgerüstet ist;
Fig. 3     eine schematische Darstellung der Situation, wenn ein Blendsignal (Sonne) auf einer Anzeige bzw. Bildschirmoberfläche reflektiert wird;
Fig. 4     die Situation aus Fig. 3, mit einer Zusatzvorrichtung für nicht modulierbare Displays;
Fig. 5     eine schematische Darstellung der Situation mit einem sog. "innenliegenden HUD";
Fig. 5B    eine Ausführung als Schutzbrille für völlige Dunkelheit, ohne Eigenlichtquelle (Arbeitsschutz);
Fig. 6     ein Diagramm der Transmission für ein Blendunterdrückungssystem mit RGB-Farbcodierung;
Fig. 7     ein Diagramm, welches das Verhalten der unterschiedlichen Transmissionsgrade TR (Ch#1, 2, 3) bei einem Blendunterdrückungssystem mit RGB-Farbcodierung verdeutlicht;
Fig. 8     eine schematische Darstellung eines Systems zum Verstärken des räumlichen Eindrucks;
Fig. 9     eine schematische Darstellung eines Systems zum Verbessern der Sehweite durch Ausblendung von Reflexionen im Nahbereich bei Partikelniederschlag nach dem LIDAR-Prinzip;
Fig. 10    ein Diagramm zur Eigenlichtunterdrückung eines Blendunterdrückungssystems; und
Fig. 11    ein weiteres Diagramm zur Eigenlichtunterdrückung, das die Initialisierungsphase zeigt.

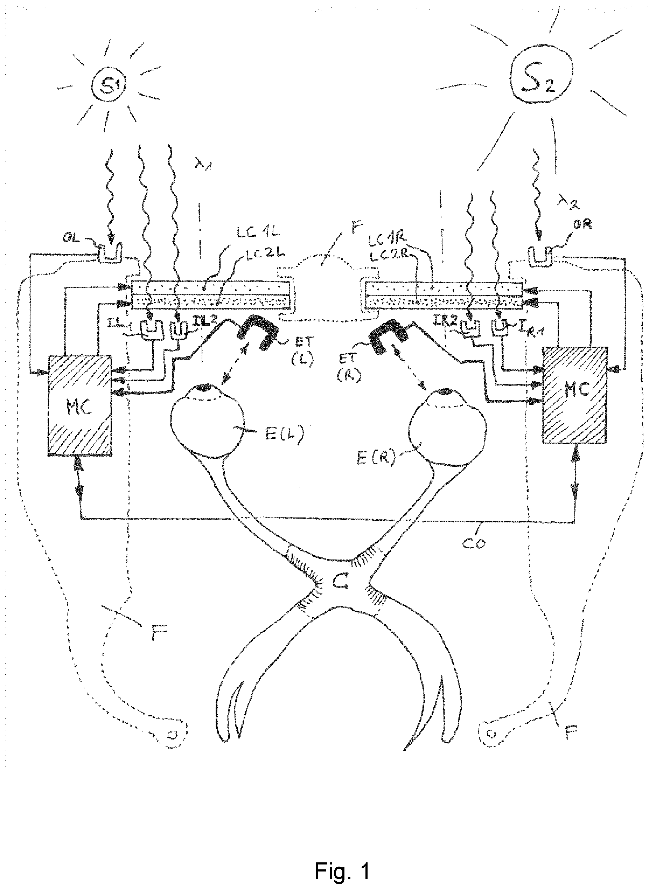

Im Folgenden wird teilweise auf Fig. 1 Bezug genommen.

Alles Nachfolgende gilt immer für ein Auge (rechts oder links, auch als "Kanal" bezeichnet). Ein Kanal besteht aus mindestens einer LC-Zelle (es können aber auch zwei oder mehrere LC-Zellen hintereinander geschaltet werden), welche je nach Anwendung geeignetes schnelles und kontrastreiches LC-Material (TN, STN, Fe-LC) beinhaltet.

Die vom menschlichen Körper entferntere Zelle wird als "distal" bezeichnet, die am Auge befindliche als "proximal". Hinter der proximalen Zelle befinden sich in einem gewissen Abstand (typ. 1-3 mm) ein bis drei komplexe Fotosensoren IL1, IR1, die in Blickrichtung den Lichteinfall durch die LC-Zelle LC 1L, LC 2L, LC 1R, LC 2R aufnehmen, wobei ein einzelner Fotosensor wiederum aus mindestens 3 Sensoren besteht, die ein orthogonales x-y-z-Koordinatensystem aufspannen - wobei der Vektor (1,1,1) sinnvollerweise in Blickrichtung zeigt.

Alternativ zu einem solchen x-y-z-Fotosensor ist es möglich, ein Fotosensor-Array zu verwenden, welches - ähnlich einem Facettenauge - über deutlich mehr als nur 3 orthogonale Kanäle verfügt. Jeder Kanal kann die Helligkeit in einem großen Dynamikbereich messen, so dass dem Mikroprozessor ein "grobes Bild" übermittelt wird.

Alternativ zu einem solchen "groben Bild" kann auch ein mit optischen Linsen abbildendes System (Kamera) mit deutlich höherer Auflösung vorgesehen sein (z.B. 5 Megapixel Kamera) bei gleicher Miniaturbaugröße, die wenige Quadratmillimeter nicht überschreitet, ähnlich wie sie bereits in Smartphones und Notebooks eingesetzt werden. Das von solch einer Kamera an den Prozessor übermittelte Bild ist feiner aufgelöst: Der Dynamikumfang und die Linearität zur Messung der Helligkeit wird durch den Einsatz hochdynamischer Chipmaterialien sichergestellt, ähnlich wie in der analytischen medizinischen Fotographie.

Aus reinen Sicherheitsgründen werden pro Auge E(L), E(R) (Kanal) mindestens 3 solcher komplexen Fotosensoren verwendet (x-y-z, oder Facetten oder Kamera).

Alle genannten Fotosensoren können z.B. als Fotodioden, Fototransistoren, Fotozellen, usw. ausgeführt sein - jedenfalls ist allen gemeinsam, dass sie farbneutral reagieren, indem sie die Farbempfindlichkeitskurve des Auges (sog. V-Lambda-Funktion nach DIN 5031) mit einrechnen. Derartige Fotozellen werden beispielsweise in der Fotographie zur farbneutralen Belichtungsmessung eingesetzt. Per Software kann, je nach Umgebungshelligkeit (gemessen durch einen Außensensor OL, OR, oder abgeleitet aus Stellgröße und Sollwert des Reglers MC) bei hauptsächlicher Dunkelheit ein Look-Up-Table (LUT) in den Rechenalgorithmus einbezogen werden, der die V' Werte für Nachtsicht beinhaltet, so dass der sog. Purkinje-Effekt (erhöhte Blauempfindlichkeit bei Nacht) berücksichtigt wird. Ferner kann die individuelle, altersabhängige Blendempfindlichkeit berücksichtigt werden - dazu gibt es empirische Studien, insbesondere winkelabhängig und altersabhängig (z.B. Adrian and Bhanji 1991 - Illumination Engineering Society of North America).

Freiform-Linse/Kanal oder Software mit Kamera

Die physikalische Umsetzung der o.g. Augenempfindlichkeitsformel kann durch eine entsprechende Freiform-Linse aus transparentem Material (z.B. Glas, Kunststoff, Flüssigkeit, etc.) erfolgen, die derart vor einem Fotosensor angebracht ist, dass er wie das menschliche Auge zur richtungsempfindlichen Messung von Helligkeit eingesetzt werden kann. Es wird also ein "künstliches Auge" kreiert, welches sich ebenso blendungsempfindlich über den Einfallswinkel zeigt wie ein menschliches Auge. Hierzu müssen zwei Faktoren berücksichtigt werden: 1. die V-Lambda- und V'-Lambda-Funktionen (Purkinje-Effekt bei Nacht); 2. die winkelabhängige Blendungsempfindlichkeit.

Anstelle dieser Linse kann vereinfachend auch ein entsprechend per Freiformberechnung geformter schwarzer Kanal (d.h. im Wesentlichen eine Bohrung) verwendet werden, an dessen Ende sich die Fotozelle befindet, so dass diese einen Öffnungswinkel erhält, der der Empfindlichkeit des menschlichen Auges entspricht.

Alternativ kann die Formel zur Blendungsempfindlichkeit rein als Algorithmus bzw. in die Software implementiert werden, die auch das hochauflösende/hochdynamische Bild der Kamera empfängt, da in dem Kamerabild auch die Richtungsinformation und Helligkeit pro Pixel enthalten ist. Das Kamerabild kann dann mit individuellen (altersabhängigen) Bewertungsformeln gewichtet werden, zumal man sein persönliches Alter oder sonstige individuelle Präferenzen oder ärztliche Indikationen/Empfehlungen bezüglich Blendungsempfindlichkeit über eine beliebige Mensch-Maschine-Schnittstelle (z.B. Tasten an der Brille, USB-PC-Software-Schnittstelle, Smartphone-App via (Bluetooth)-Funk) eingeben kann.

Eye Tracker

Die Richtungs- und Helligkeitsinformationen der Fotozellen/Kamera können zusätzlich mit der Blickrichtung, die durch einen Eye Tracker ET(L), ET(R) bestimmt werden kann, mathematisch korreliert werden.

Die individuelle und altersabhängige Blendempfindlichkeitsfunktion, welche in der Software als Formel oder Look-Up-Table (LUT) hinterlegt sein kann, kann dann wie eine Schablone (z.B. mit multiplikativer Gewichtung) über das Signal des nach vorne schauenden Helligkeitssensors gelegt werden. Dieser Sensor ist starr an der Brille montiert. Aufgrund des Eye-Tracker-Signals wird diese Schablone jedoch gemäß der Augapfel-Bewegung mitverschoben, wodurch die Funktionalität eines künstlichen Auges erreicht wird, welches die individuelle blickwinkelabhängige Blendempfindlichkeit berücksichtigt,

Puls-Shaping bei den LC-Zellen

Hier gibt es drei Möglichkeiten:

1. Bei beiden LC-Zellen handelt es sich um Zellen, die im spannungslosen Zustand jeweils transparent sind, um im Falle eines System- oder Spannungsausfalls in jedem Falle normale Sicht zu ermöglichen.

2. Für Hochsicherheitsanwendungen, bei denen von einer permanenten Blendungsgefahr im Arbeitsbereich auszugehen ist (z.B. LASER-Labor oder Lichtbogenschweißen) können LC-Zellen eingesetzt werden, die genau umgekehrt arbeiten, d.h. im spannungslosen Zustand komplett dunkel geschaltet sind und nur durch Drücken eines Sicherheitsschalters oder dergleichen transparent geschaltet werden können.

3. Mischung von Zellen der o.g. Typen, also eine im spannungslosen Zustand durchlässige und eine undurchlässige Zelle. Diese Anordnung kann dazu genutzt werden, die Flankensteilheit bei sowohl der aufsteigenden als auch der fallenden Flanke eines optischen Pulses zu verbessern, im Sinne von Transparentschaltung für einen Bruchteil einer Sekunde in der Form eines Rechteckpulses auf der Zeitachse (Rechteck mit hoher Flankensteilheit auf dem optisch messenden Oszilloskop-Bild). Der Vorteil besteht in geringerem Übersprechen und sonstigen kontrastvermindernden Artefakten (Crosstalk) in Synchron-Anwendungen mit einer Eigenlichtquelle oder mehreren Teilnehmern.

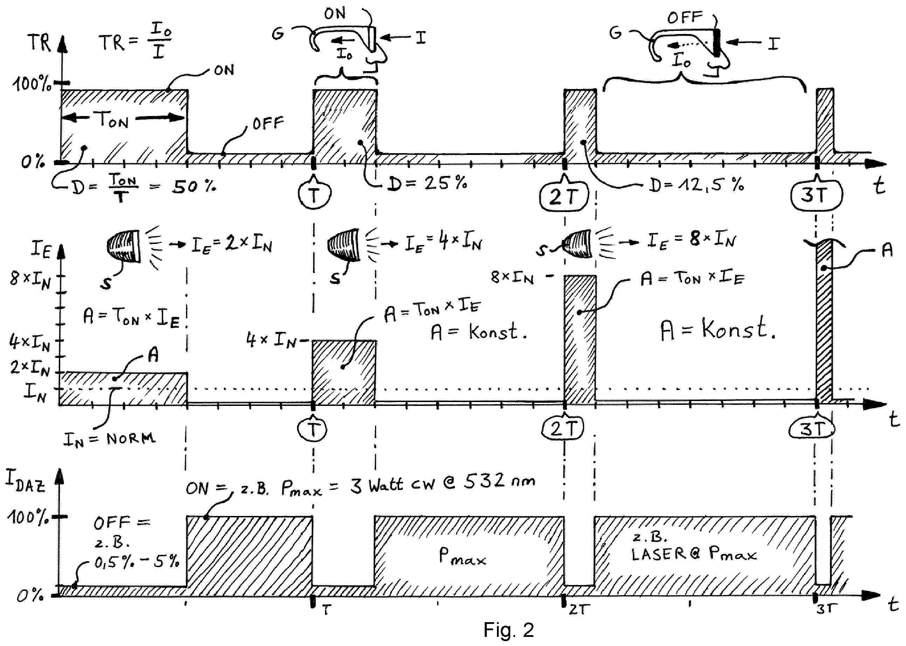

Eine wie vorstehend beschriebene Brille kann als Teil eines Blendunterdrückungssystems Verwendung finden. Fig. 2 zeigt die sogenannte Transmission (TR) einer solchen Brille über die Zeit. Die Transmission ist dabei der Quotient aus der durch die Flüssigkristallzelle LC gelassenen Intensität $I_o$ und der einfallenden Intensität I.

In der Zeit $T_{on}$ ist die Brille geöffnet, d.h. transparent geschaltet. In der restlichen Zeit (Periodenzeit T minus $T_{on}$) ist die Brille geschlossen, d.h. nicht transparent.

Um nahtlose und analoge Grauwerte zu erhalten, wird das Signal in Fig. 2 (erste Zeile) als analoge Pulsweiten-Modulation PWM realisiert, d.h. in Fig. 2 sind von Zyklus T zu den Zyklen 2T und 3T lediglich beispielhaft verschiedene sprunghafte Zustände der PWM abgebildet. Diese Zustände lassen sich auch als prozentuales Puls-Zykluszeit-Verhältnis D (Duty-Cycle) beschreiben.

Um das "Rauschverhältnis" SNR ("Signal to Noise Ratio") zu verbessern, wird die Pulsenergie pro ausgesendeten Lichtpuls in gewissen Grenzen konstant gehalten. Insbesondere soll die Fläche A in der mittleren Zeile von Fig. 2, die sich ergibt aus der aktiven Pulsweitenzeit $T_{on}$ multipliziert mit der jeweiligen emittierten Lichtintensität IE (I = Intensität, E=Emittiert) eines Pulses, weitgehend konstant gehalten werden.

In der Praxis kann dies durch Anlegen einer höheren Spannung bzw. durch Einprägen eines höheren Stroms in ein geeignetes Leuchtmittel, welches für solch hohe Energien ausgelegt ist, erfolgen. Es bleibt dem Fachmann überlassen, sicherzustellen, dass das vorhandene Leuchtmittel hierfür geeignet ist.

Zudem muss die Lichtintensität IE stets dem normierten und bereits von Behörden (TÜV etc.) zugelassenen Intensitätswert I Norm entsprechen, allerdings multipliziert mit dem Kehrwert des Hundertstel des Duty-Cycle D.

Beispiel:

$$\text{Puls-Pause-Verhältnis = Duty-Cycle = 50\% = 0,5}$$

Kehrwert von 0,5 = Faktor 2

$$\text{IE} = 2 \times \text{I Norm}$$

Dieses Verfahren ist notwendig, damit die über ein langes Zeitintegral gemessene Intensität im zeitlichen Mittel immer einer konstanten I Norm entspricht. Selbst wenn das zeitliche Messintervall bei den Behörden nur 1 Sekunde betrüge, so wären bei einem 70 Hz Scheinwerfer bereits so viele unterschiedliche Pulshöhen bzw. Puls-Zyklen zeitlich gemittelt worden, dass sich immer der geforderte konstante Lichtwert I Norm ergibt. Integriert man das Signal IE in der mittleren Zeile von Fig. 2 von t = 0 bis zum Zyklus-Ende T3, so wird das Prinzip deutlich.

Darüber hinaus wird bei sehr schmalen Zeitschlitzen, in denen die Brille offen und transparent ist (z.B. 5%), bei entsprechend dunkel eingestelltem Sollwert des Regelkreises, d.h. bei konstant und weit geöffneter Augenpupille, das Auge so lichtempfindlich, dass bereits kleine Leistungen von IE (d.h. IE dividiert durch $T_{on}$) ausreichen, um eine sichtbare Verbesserung der betrachteten Szenerie zu erreichen, während ca. 100 - 5% = 95% des störenden Fremdlichtes unterdrückt werden.

Das vorliegende Blendunterdrückungssystem kann mit einer Blendwaffe (Dazzler) kombiniert werden. Die unterste Zeile von Fig. 2 bezieht sich auf diese Situation und zeigt, wie der Dazzler ein bezüglich der Öffnungsdauer $T_{on}$ der Brille antizyklisches bzw. invertiertes Einschalt-Signal erhält. Zudem ist zu erkennen, dass der Dazzler auf einen (frei einstellbaren) von Null verschiedenen OFF-Wert in Höhe von z.B. 0,5-5% seiner maximalen Intensität I DAZ festgelegt werden kann, damit er für den Anwender visuell gut verfolgbar bleibt.

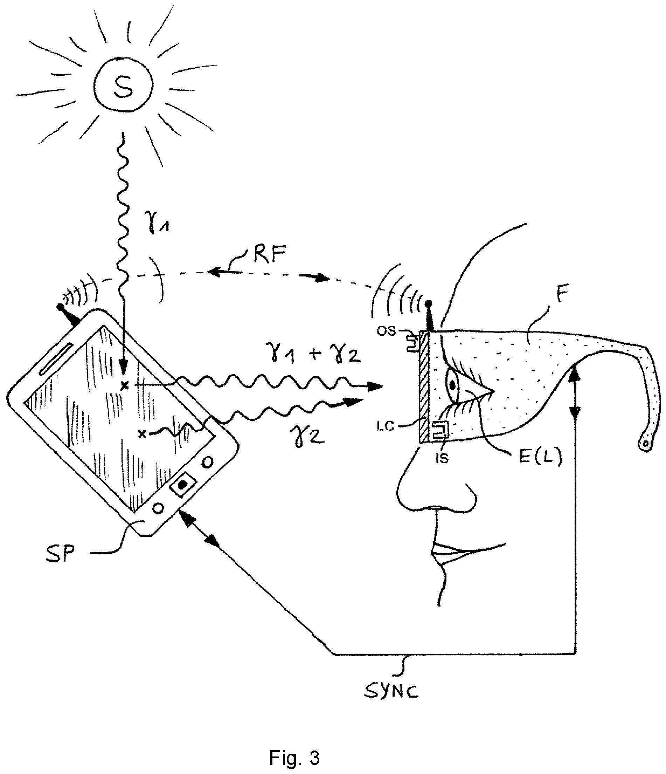

Fig. 3 zeigt, wie das Anti-Blend-System mit einer Anzeige kombiniert werden kann, um Blendungen durch Reflexion an der Anzeige zu unterdrücken und gleichzeitig die Ablesbarkeit der Anzeige sicherzustellen. Hierbei ist das Summensignal Gamma 1 + 2 am Auge stets aus Störsignal und Nutzsignal zusammengesetzt. Im einfachsten Fall kann bereits eine aus dem Internet herunter geladene Software (z.B. eine sog. App) die Display-Hintergrundbeleuchtung eines Smartphones SP oder ähnlichen Geräts, wie z.B. Tablet oder Notebook oder ein außerhalb der Brille befindliches Head-Up-Display, in ihrer Helligkeit derart modulieren, dass das oben beschriebe Anti-Blendsystem realisiert wird. Über 95% des Sonnenlichtes S und Gamma 1 können so unterdrückt werden, während die Lichtpulse des Bildschirms genau in den offenen Zeitschlitz der Brille und auf das dunkeladaptierte Auge fallen.

Die Synchronisierung der Brille mit der Anzeige kann auf verschiedene Weisen geschehen:

1) In einem Falle ist das Elektronik-Gerät der "Master", der einfach gepulstes Licht aussendet, worauf sich die Brille mit Hilfe ihrer Lichtsensoren (Außen = OS, Innen = IS) rein optisch synchronisieren kann.
2) Optional kann über eine Funkverbindung RF zwischen Brille und Endgerät eine Synchroninformation ausgetauscht werden. Typischerweise werden dabei bereits serienmäßig vorhandene Funksysteme, wie z.B. Bluetooth, genutzt. Welches Gerät "Master" ist, kann hier offen bleiben und ist nur eine Frage der Programmierung.
3) Im Übrigen kann auch mit Hilfe eines Kabels (z.B. USB) oder auch auf jede andere erdenkliche Art eine Synchroninformation SYNC zwischen Endgerät und Brille übermittelt werden. Wer von beiden "Master" ist, kann auch hier offen bleiben und ist nur eine Frage der Programmierung.

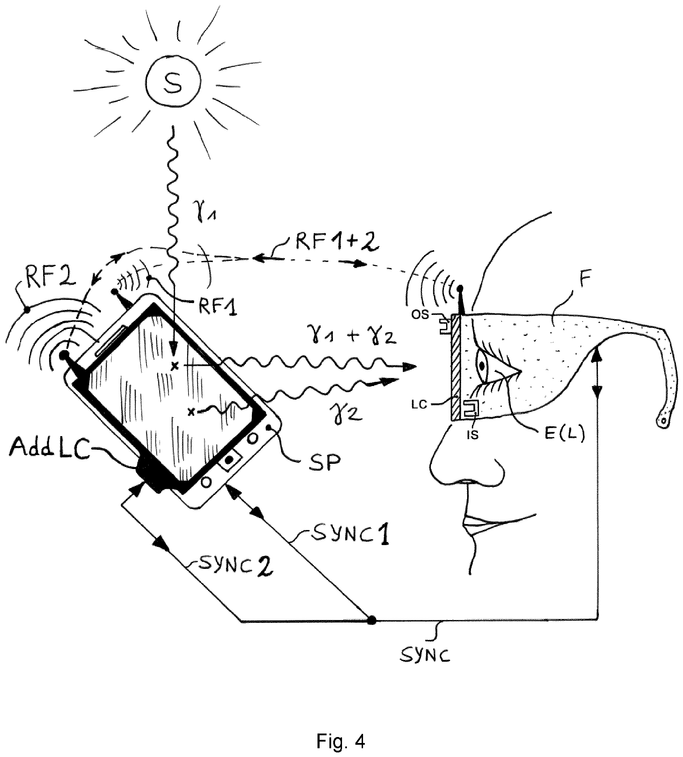

Im Folgenden wird auf Fig. 4 Bezug genommen.

Auch für Displays und Anzeigen, die eine Modulation der Hintergrundbeleuchtung nicht ohne weiteres zulassen, ist eine Lösung möglich. Für Anzeigen, die zumindest eine gleichförmige Hintergrundbeleuchtung besitzen (z.B. papierähnliche Displays mit "elektronischer Tinte" zum Lesen von Büchern), kann ein weiterer Flüssigkristall-Shutter AddLC auf dieses Display aufgelegt oder geklemmt werden. Dieser zusätzliche Shutter moduliert dann das ansonsten gleichmäßige (DC), aber maximale (oder per Eingriff auch übermaximale) Hintergrundlicht des Displays entsprechend der Zeitschlitze der Brille. Sofern die gleichmäßige Hintergrundbeleuchtung sehr hell eingestellt werden kann, ergeben sich mit dieser Anordnung die bereits oben beschriebenen Vorteile der Blendungsunterdrückung von fremden Störlichtquellen S, einschließlich der beschriebenen Verbesserung der Lesbarkeit. Der zusätzliche Shutter verfügt über eigene Schnittstellen zur Synchronisation mit der Brille, z.B. Funk RF2 oder einen Kabelanschluss (z.B. USB) oder einen beliebigen sonstigen Zugang SYNC2.

Im Übrigen kann auch eine geeignete Kombination aus den genannten Informationskanälen zum Einsatz kommen, z.B. eine Software ("App") zum Einschalten der Hintergrundbeleuchtung über Funk RF1, und zur Synchronisation mit der Brille die Funkverbindung RF2 oder das Kabel SYNC2. Auch eine rein optische Synchronisation durch die optischen Sensoren OS, IS der Brille ist möglich.

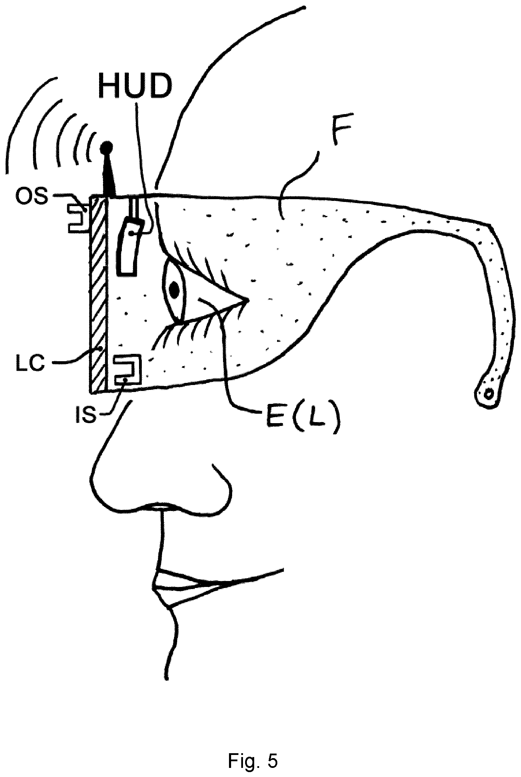

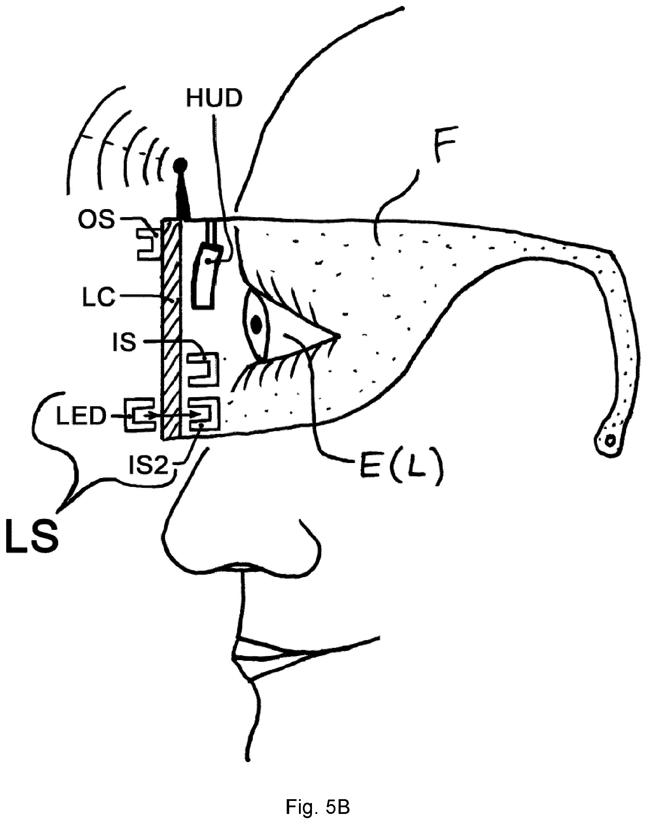

Im Gegensatz zum außerhalb der Brille liegenden Head-Up-Display (HUD) stellt das "innerhalb der Brille liegende HUD" einen Sonderfall dar, der in Fig. 5 dargestellt ist (Durchsicht-HUD, ähnlich wie bei "Google Glass" oder Samsung "Gear Glass" etc.). Hier ergibt sich eine Ableseverbesserung durch Blendunterdrückung, was z.B. beim versehentlichen Blick in die Sonne wichtig ist (Shutter wird kurzfristig ganz oder nahezu geschlossen). Zudem ergibt sich eine Verbesserung dadurch, dass die Brille über einen sehr großen Dynamikbereich stets auf exakt die gleiche Helligkeit regelt (weitgehend konstanter Sollwert), wodurch wiederum das innenliegende Durchsicht-HUD stets die optimale Hintergrundhelligkeit und/oder den optimalen Kontrast erhält, egal wie sich außerhalb die Helligkeit ändert. Das innenliegende HUD ist dadurch jederzeit optimal ablesbar.

Das Folgende bezieht sich auf Fig. 5B.

Es gibt im Rahmen des Arbeitsschutzes sehr einfach arbeitenden Blendschutzbrillen, die hauptsächlich im Dunkeln getragen werden, z.B. in Forschungs- und Entwicklungslaboren, in denen es zur Durchführung der Arbeit dunkel sein muss (Licht- und LASER-Experimente, Bio-Tech), von Hautärzten während einer Therapie mit Lichtpulsen hoher Intensität (Intense pulsed light - IPL Therapy) oder dergleichen. Diese Schutzbrillen sind jedoch für die Arbeitsausführung oft ungeeignet, weil sie nur zwei Zustände kennen, nämlich auf und zu, und zudem falsch reagieren, da zu wenige Fotosensoren außen angebracht sind, die die Flüssigkristall-Gläser lediglich ansteuern, aber nicht in Echtzeit regeln (vgl. z.B. DE 10 2014 107 587). Zudem bleibt der Transmissions-Zustand der Gläser (auf oder zu) bei Dunkelheit unbekannt, da weder eine Steuerung noch eine Regelung zuverlässige "Istwerte" liefern kann. Sogar eine Regelung hätte in völliger Dunkelheit (z.B. um Null Lux herum) das Problem, dass der Istwert zu klein sein kann, um zuverlässige und sicherheitsrelevante Aussagen über die korrekte Funktion der Flüssigkristallzellen zu machen.

Für solche Situationen wird pro Augenglas (also links und rechts) eine aktive Lichtschranke LS vorgesehen, bestehend aus einen aktiven Lichtemitter LED und einem gegenüberliegenden, weiteren Innen-Sensor IS2, womit durch die Flüssigkristallzellen hindurch deren Transmission konkret über einen weiten analogen Dynamikbereich gemessen werden kann - selbst bei völliger Dunkelheit.

System zur Blendunterdrückung mit RGB-Codierung

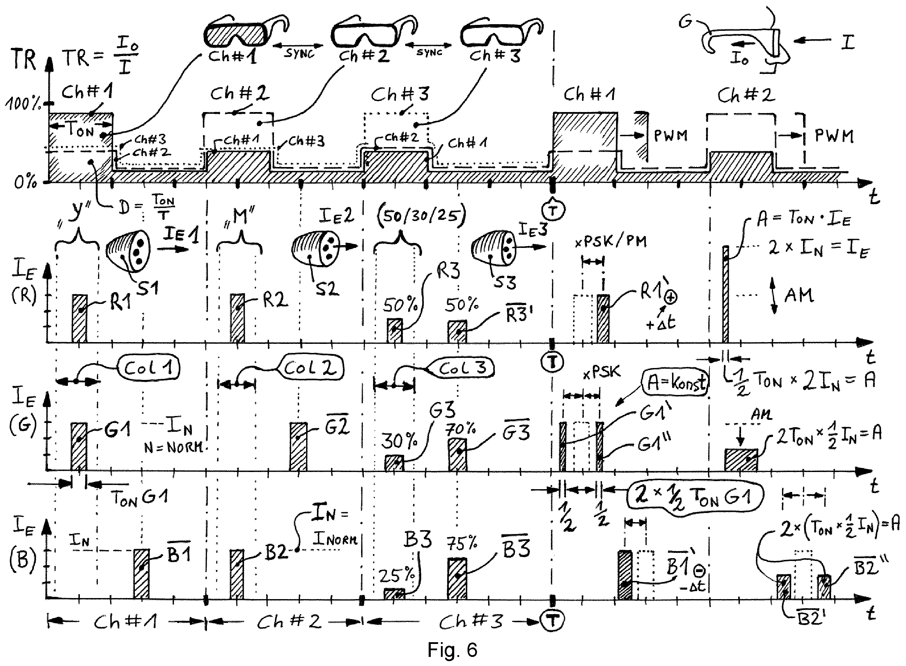

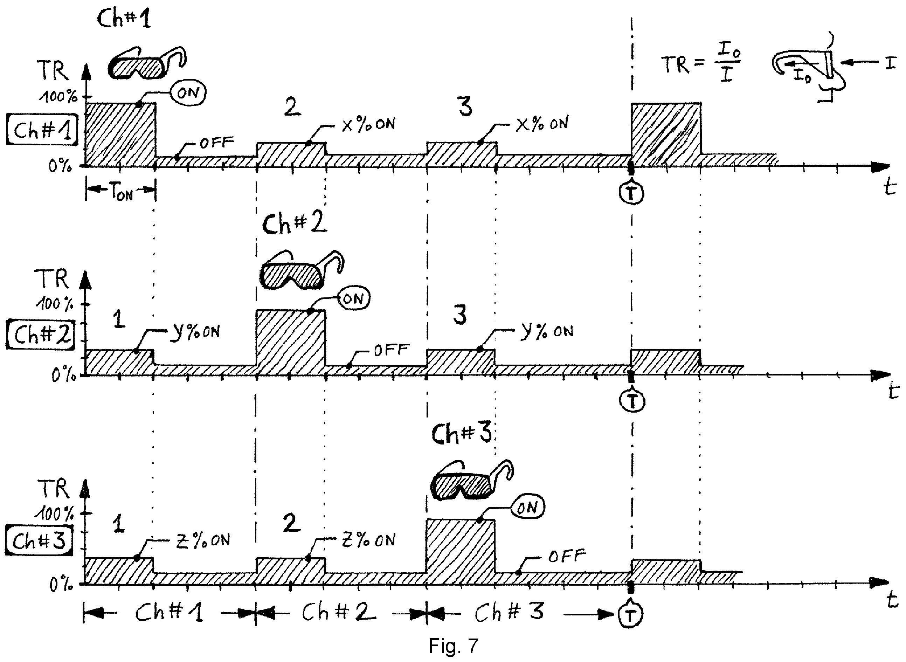

Im Folgenden wird auf die Figuren 6 und 7 Bezug genommen.

Insbesondere bei Blendunterdrückungssystemen, die für Gruppenanwendungen für den Einsatz bei Behörden und Organisationen mit Sicherheitsaufgaben (BOS) oder beim Militär vorgesehen sind, kann eine Ausführungsform zum Einsatz kommen, die ermöglicht, z.B. verschiedenen Teilnehmern oder verschiedenen beleuchteten Objekten (z.B. markierte Ziele) jeweils eine frei wählbare Lichtfarbe zuzuordnen, die beispielsweise nur ein Teammitglied in aller Deutlichkeit zu sehen bekommt - und in abgeschwächter Form auch seine Gruppenmitglieder, während das verwendete Licht für Außenstehende weiß erscheint.

Hierzu werden Eigenlichtquellen verwendet, die nicht nur in ihrer Amplitude bzw. Leuchtintensität moduliert werden können, sondern auch in ihrer Farbe (Wellenlänge). Neben Wellenlängen-durchstimmbaren Lichtquellen wie Oszillatoren (z.B. OPO, OPA-Laser etc.) bieten sich im einfachsten Falle leistungsstarke RGB-LASER oder RGB-LED an, die typischerweise über 3 separat ansteuerbare Kanäle verfügen - nämlich für die sogenannten Primär-Farben "rot, grün und blau", gemäß RGB-Farbmodell, die in entsprechender Überlagerung weißes Licht ergeben. Auch andere Arten und Kombinationen von Primärfarben in der Nähe des RGB-Farbmodells sind denkbar, solange sie in der Summe weißes Licht ergeben.

Die Farben R=Rot, G=Grün, B=Blau des ersten Kanals Ch#1, jeweils getrennt dargestellt in den unteren 3 Diagrammen von Fig. 6 (IE von R, G, B), werden nicht notwendigerweise absolut zeitgleich ausgesendet, sondern es kann beispielsweise Blau auch mit einer leichten zeitlichen Verzögerung nach Rot und Grün ausgesendet werden, jedoch so kurz danach (wenige Millisekunden), dass das menschliche Gehirn dies nicht als Flackern wahrnimmt, sondern stets gemeinsam als weißes Licht.

Der Unterschied für den Träger der Brille mit der Kanalbezeichnung besteht jedoch darin, dass in dem Zeitschlitz $T_{on}$, in dem die Brille geöffnet ist (also TR nahe 100%), die beiden Farben Rot und Grün von der Eigenlichtquelle ausgesendet werden, die Farbe Blau aber erst dann, wenn die Brille bereits wieder geschlossen ist (TR nahe 0% = OFF). In Fig. 6 ist dieser Blau-Puls bezeichnet mit "B1 und Oberstrich", wobei der Strich über dem Buchstaben "negiert" bedeutet. In diesem Kontext heißt B1 negiert "Blau, unsichtbar für Kanal 1". In Fig. 6 ist dies symbolisch mit Y oberhalb der geschweiften Klammer angedeutet, weil die Summe aus Rot und Grün die Mischfarbe Gelb (Engl. Yellow) ergibt. Der Träger der Brille Ch#1 sieht also gelbes Licht. Es wird also wenigstens ein Mehrkanal-Zeitmultiplex-Verfahren bezüglich der 3 Farbkanäle RGB und der jeweiligen Brillen verwendet.

In Fig. 6 ist im Kanal 2 bzw. erkennbar, dass sich hier die Farben Rot und Blau R+B in dem Zeitschlitz $T_{on}$ mischen, in dem die Brille offen ist, angedeutet durch die geschweifte Klammer mit M (für Magenta, da diese Farbe aus der Mischung von Rot und Blau entsteht). Der Träger der Brille Ch#2 sieht also magentafarbenes Licht.

Damit der Träger der eine Idee davon bekommt, welches Ziel sein Nachbar mit Kanal gerade anleuchtet (z.B. zwecks geheimer Markierung), wird die Brille Ch#1 im Zeitschlitz des Kanal nur ein wenig aufgehen, z.B. von nahe 0% (Brille geschlossen) auf (beispielsweise und frei einstellbare) 25% Transmission, so dass auch der Träger die Farbe Magenta des Trägers von Brille Ch#2 sieht. Da aber hiervon nur 25% sichtbar sind, kann sich der Träger von Brille Ch#1 besser auf sein eigenes Licht konzentrieren. Je nach spezieller Anwendung lässt sich der Grad dieser Abschwächung frei verändern zwischen 0% (andere Teammitglieder ausblenden) und 100% (alle anderen genauso hell sehen wie die eigene Farblichtquelle).

Eigentlich überlappen sich die "zeitgleichen Signalflanken" (durchgezogene, gestrichelte und gepunktete Linien in Fig. 6). Zur besseren Erkennbarkeit wurden diese in Fig. 6 jedoch nicht überlappend gezeichnet, sondern minimal versetzt. Die korrekte Situation, ohne diesen Versatz, wird in Fig. 7 verdeutlicht. Dort ist zu erkennen, dass jede Brille bzw. jeder Kanal Ch#1 bis Ch#3 in Wirklichkeit ungefähr gleich breit ist (gleiches $T_{on}$), und dass im Zeitschlitz der anderen Kanäle die jeweilige Brille ganz leicht öffnet (z.B. etwa 25%). Fig. 7 stellt also dieselbe Situation dar wie Fig. 6, allerdings mit separaten Kanälen. Die Variablen x%, y%, z% sollen hierbei darstellen, dass jeder Nutzer den Grad der Erkennbarkeit der anderen Teilnehmer bzw. Farben frei einstellen kann - je nach seiner Rolle im Team oder nach persönlichen Präferenzen.

In Fig. 6 sind nach Ablauf der Zykluszeit T diverse beispielhafte Modulationsverfahren für die RGB-Lichtquellen dargestellt: In Analogie zu der eingangs beschriebenen Methode der konstanten Energie pro Puls (konstante Pulsfläche A) kann auch eine RGB-Lichtquelle so moduliert werden, dass die einzelnen Farbkanäle zeitlich schmaler, aber dafür in der Intensität höher werden und/oder umgekehrt. Dies ist problemlos möglich, weil RGB-LED oder RGB-LASER relativ schnell, insbesondere deutlich hochfrequenter als die Brille, in Phase und Amplitude moduliert werden können. Somit kann die exakte Phase (zeitliche Position) eines einzelnen RGB-Pulses innerhalb der Öffnungzeit $T_{on}$ der Brille problemlos variiert werden, sei es von Gesamt-Zyklus zu Gesamt-Zyklus (ca. 70-140 Hz), oder sogar extrem schnell (>> 1 kHz) innerhalb eines Zyklus. Durch eine solche extrem schnelle Phasenvariation kann man eine Phasenmodulation oder eine PSK auf jeden einzelnen RGB-Kanal aufbringen, die von anderen Brillen oder sonstigen Empfängern erkannt werden und z.B. auch zur "optischen Synchronisation" der Brillen herangezogen werden kann - die Außen- und Innensensoren OS, IS der Brillen sind stets schnell genug dafür. Damit lässt sich eine Synchronisierung der Brillen innerhalb eines Teams auch ohne Funkkontakt herstellen (z.B. falls dieser unerwünscht ist oder ausfällt).

Abgesehen von der Farbmarkierung von Objekten kann diese Phasenmodulation zusätzlich mit einem geheimen Schlüssel und geheimen Informationsinhalten derart codiert sein, dass auch sonstige Informationen (z.B. um welche Art von Objekt es sich handelt, Name, etc.) im Sinne einer vollständigen Markierung ("full information designation") auf ein Ziel oder Objekt aufgebracht werden. Diese vollständige Information kann wiederum von den Außen- und Innensensoren OS, IS oder auch von gesonderten Empfangs- und Dekodier-Einheiten entschlüsselt werden.

In Fig. 6 ist auf dem dritten Zeitstrahl von oben (IE Grün) rechts, jenseits der Periodendauer T die Aufspaltung des Grün-Impulses in zwei zeitlich halb so breite Pulse G1' und G1" zu sehen (d.h. $2 \times \frac{1}{2} T_{on}$), rechts oberhalb bezeichnet mit A = konstant, was dem schon erklärten Prinzip der konstanten Energie pro Puls entspricht. Darüber steht zudem "xPSK", was bedeutet, dass mit zwei getrennten Pulsen, ähnlich wie "Di-Bits", welche in Phasenrelation zueinander oder auch in Relation zur Zeitachse variieren und springen können, nahezu x-beliebige PhasenModulationsverfahren möglich sind - theoretisch auch QPSK und ähnliche Verfahren.

Auf dem unteren Zeitstrahl (IE Blau) ist ganz rechts außen die Aufspaltung des Blau-Pulses in B2' und B2" (jeweils oben negiert) zu sehen, allerdings nur in halber Höhe, d.h. Amplitude 0,5 I Norm. Auch in diesem Beispiel wird deutlich, dass die Fläche A (also die Energie des Pulspaares) konstant bleibt. Die Amplitudeninformation kann, wie bei einer Amplitudenmodulation AM ebenfalls zur Informationsübertragung genutzt werden, ggf. auch mit einem geheimen Schlüssel kodiert. Auch Mischformen aus beliebigen FSK-, x-PSK- und AM-Verfahren sind daher möglich.

Die Synchronisierung der Brillen und Eigenlichtquellen erfolgt in der Regel über Funksignale, kann aber auch optisch erfolgen. Die Synchronisierung kann dabei nach einem bestimmten Hierarchie-System erfolgen, wonach ein Teilnehmer stets "Master" ist und alle anderen stets "Slave" (bei Ausfall des Master könnte nach einem einprogrammierten Rangprinzip ein bestimmter anderer "Slave" zum neuen "Master" werden usw.). Diese Hierarchie kann z.B. im Rahmen einer gemeinsamen Initialisierungsroutine (also zeitlich vor einem Einsatz) festgelegt werden, aber auch mittendrin im Geschehen (z.B. über Funk oder optisch, aufgrund einer einprogrammierten codierten Erkennung, ähnlich wie man es von Multi-User-IT-Systemen wie LAN, WLAN, Token-Ring etc. bereits kennt).

Zudem kann dieses Multi-User-Gesamtsystem auf Kosten einer etwas geringeren Kanalanzahl so betrieben werden, dass der Pulsweiten-Modulationshub der Brille etwas erweitert wird (siehe Fig. 6 oben rechts im Diagramm TR, rechts jenseits der Periode T, gekennzeichnet mit gestrichelter Flanke und PWM. Diese Erweiterung des PWM-Modulationshubes hat den Vorteil, dass die Brille bei leichter Dämmerung (z.B. 0 Lux bis 100 Lux) weiterhin mit analogen Graustufen geregelt werden kann. Selbst bei einer Multikanal-Gruppenanwendung mit unsichtbarer Farbmarkierung kann die Brille zwecks analogen Graustufenregelbetriebs nahtlos in Richtung einer Tagfahrbrille (wie weiter oben beschrieben) betrieben werden.

Die Eigenlichtquelle muss nicht notwendigerweise ausschließlich aus leistungsstarken RGB-LEDs oder RGB-LASERn bestehen, sondern kann auch aus Hochleistungs-Weißlicht-LEDs bestehen, die beispielsweise den Hauptanteil des Eigenlichts ausmachen, während die rot-grün-blau-Komponenten nur additiv zwecks Farbgebung hinzugemischt werden. Dies kann erreicht werden, indem sich im Scheinwerfer/Reflektor neben den Weißlicht-LEDs auch mindestens eine oder mehrere RGB-LEDs/LASER befinden.

In dem kurzen Zeitschlitz $T_{on}$, in dem die eigene Brille offen ist, wird von der Eigenlichtquelle neben dem bereits in Fig. 2 (mittlere Zeile) dargestellten Weißlichtimpuls gleicher Fläche auch eine bestimmte Farbe ausgesendet, d.h. die beiden Modulations-Verfahren (Weißlicht und unsichtbare Farbmarkierung) lassen sich kombinieren, so dass es sich nach wie vor um ein nahtlos funktionierendes Gesamtsystem handelt. Auch eine (weiter oben beschriebene) Blendwaffe kann nach wie vor parallel zu der hier beschriebenen unsichtbaren Farbmarkierung benutzt werden, da diese nur dann eingeschaltet ist, wenn die Brillen sämtlicher Kanäle (Ch#1, 2, 3, etc.) jeweils geschlossen sind (minimale Transmission).

Optional dazu kann die Eigenlichtquelle, wie schon weiter oben beschrieben, nach wie vor mit einem geheimen Puls-Sprungverfahren versehen werden, so dass z.B. gegnerische Einheiten die Farben nicht dekodieren können und auch das Gesamtsystem (Scheinwerfer mit Brille) nicht stören können. Ein solches Gesamtsystem kann selbstverständlich auch mit der verbesserten Ablesbarkeit von Displays kombiniert werden (Figuren 3 bis 5).

Verstärken des räumlichen Eindrucks

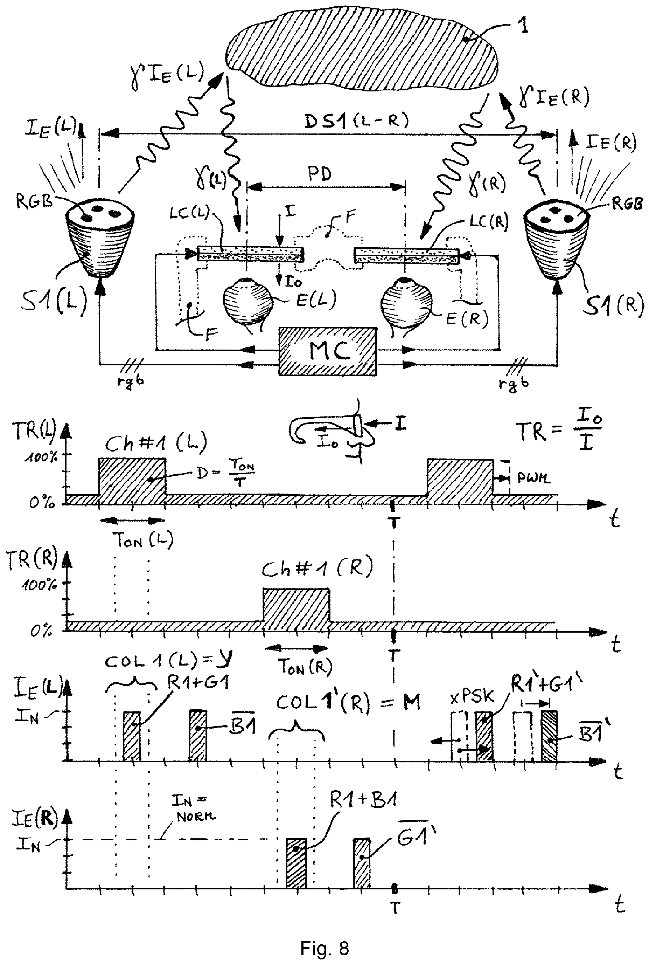

Objekte in großen Entfernungen erscheinen aufgrund des begrenzten menschlichen Augenabstandes zunehmend eindimensional, wodurch ihre Erkennbarkeit eingeschränkt wird. Eine Ausführungsform des erfindungsgemäßen Gesamtsystems, welche hier Abhilfe schaffen kann, ist in Fig. 8 dargestellt. Dort ist der Augenabstand bzw. die Pupillendistanz PD zu sehen, sowie ein beliebiges Objekt 1, welches sich z.B. einige hundert Meter entfernt befindet (auch wenn es sich aufgrund der begrenzten Zeichnungsgröße unmittelbar vor der Brille F zu befinden scheint) - je nach Reichweite der beiden separierten Eigenlichtquellen S1(L) und S1(R). Wie weiter oben beschrieben, kann die Brille F für beide Augen die Helligkeit völlig getrennt voneinander (d.h. zwei voneinander getrennte Kanäle/Regelungen) in Echtzeit regeln, dies sogar unter Berücksichtigung von absichtlichen Helligkeitsdifferenzen (HDR-Sehen) und/oder physiologischen Besonderheiten. Nun ist allerdings vorgesehen, dass der Mikrokontroller MC auch zwei getrennte Eigenlichtquellen ansteuern kann. Diese sind rechts und links vom Träger eines solchen Systems angeordnet, allerdings in einem größeren Abstand DS1(L-R) als der Pupillenabstand PD des Trägers.

Die Arbeitsweise entspricht dabei im Wesentlichen der weiter oben beschriebenen RGB-Codierung. Die Flüssigkristalle der Brille werden dabei wechselseitig nacheinander, aber niemals gleichzeitig geöffnet, so wie im Diagramm TR(L) und TR(R) dargestellt. Da es sich nach wie vor um ein Zeitmultiplex-Verfahren handelt, geht dies auf Kosten der freien Kanäle (Nutzer), so dass das System in etwa nur halb so viele Nutzer in einer Gruppenanwendung verarbeiten kann, sofern alle Teilnehmer die 3D-Verstärkung nutzen wollten. Im Unterschied zur weiter oben beschriebenen RGB-Codierung wird hierbei allerdings pro Auge eine deutlich voneinander unterscheidbare Farbe verwendet, z.B. Gelb Y links und Magenta M rechts.

Aus Platzgründen wurde in Fig. 8 nicht jeder einzelne RGB-Kanal aufgezeichnet, sondern direkt die Farbsumme pro Augenkanal L, R, erkennbar z.B. an der Bezeichnung R1+G1 im linken Kanal IE(L). Im Totzeitschlitz (beide Brillengläser sind geschlossen), folgt der Lichtpuls B1 (negiert), damit für außen stehende Dritte das Gesamtsystem in neutralem Weißlicht erscheint. Auf dem rechten Augenkanal IE(R) addieren sich beispielhaft R1+B1 zu M (Magenta), im Totzeitschlitz (beide Augengläser geschlossen) gefolgt von einem Grünpuls G1 (negiert). Das Grundprinzip ist also prinzipiell identisch mit der RGB-Codierung, auf deren Beschreibung weiter oben zur weiteren Vertiefung verwiesen sei. In Fig. 8 sind außerdem rechts, jenseits der Periodendauer T bereits beschriebenen Phasenmodulationsverfahren und xPSK-Verfahren angedeutet.

Insgesamt führt dieses Verfahren zu einer besseren 3D-Wahrnehmung, die in der Fachliteratur allerdings häufig als "2,5D" bezeichnet wird, da man nicht vollständig hinter das Objekt schauen kann.

Das Verfahren funktioniert auch mit einem Gemisch aus moduliertem Weißlicht und RGB-Licht, so dass das System zum weiter oben genannten Mischen von hochfrequenten RGB-LED/LASER-Modulen mit etwas langsameren Weißlicht-LEDs kompatibel ist.

Die Verwendung von reinem Weißlicht (d.h. ohne RGB-Quellen) ist ebenfalls möglich, insbesondere indem der Abstand der Quellen DS1(L-R) noch mehr vergrößert wird und/oder indem man den linken und rechten Kanal wechselseitig deutlich wahrnehmbar aufblinken oder wechselseitig hin und her blinken lässt (z.B. mit 2 bis 10 Hz), was durch entsprechende Ansteuerung der Eigenscheinwerfer und der Brille möglich ist.

LIDAR

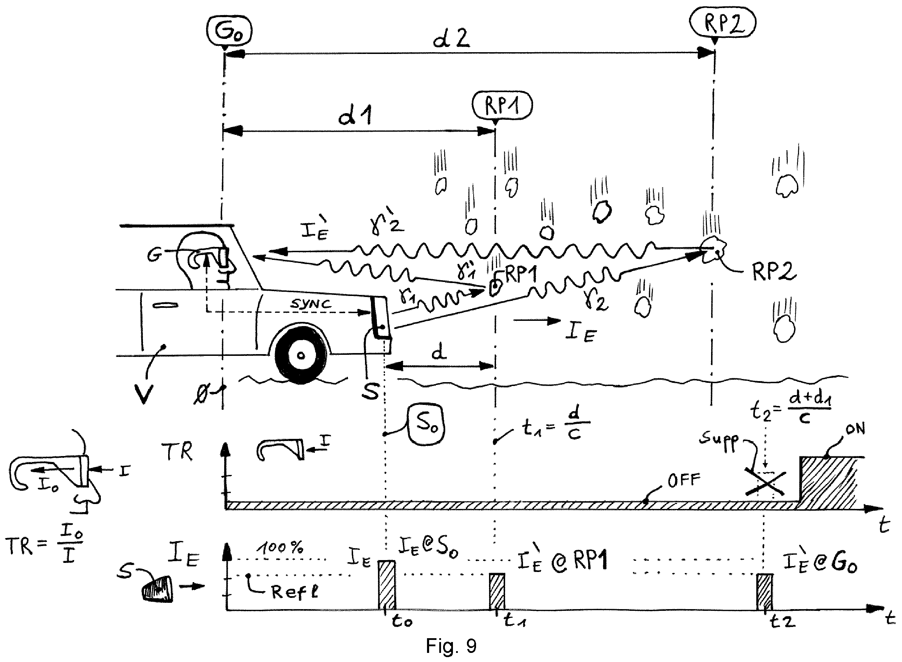

Das bisher beschriebene System kann derart erweitert werden, dass Lichtreflexionen von herunterfallenden oder aufsteigenden Partikeln im Nahbereich des Nutzers ausgeblendet werden. Das Problem tritt beispielsweise beim nächtlichen Autofahren bei Schneetreiben auf, wobei die Schneeflocken unmittelbar vor den Scheinwerfern aufgrund der höheren Leuchtdichte besonders hell erscheinen, ja geradezu blenden, so dass die Sicht auf größere Entfernung, in die Raumtiefe hinein, behindert wird. Diese Situation ist in Fig. 9 dargestellt: In einer Entfernung d1 reflektiert ein Reflexionspartikel RP1 das Licht Gamma1 in Richtung Fahrer.

Werden mit speziellen LASER- oder LED-basierten Scheinwerfern ultrakurze Pulse mit Pulsweiten von wenigen Nanosekunden erzeugt, so können diese nach dem (aus dem Stand der Technik bekannten) LIDAR/LaDAR-Prinzip über ihre Laufzeit mit Hilfe eines ebenso schnellen Shutters für den Nutzer aus- oder eingeblendet werden. Dazu wird die Shutterbrille so angesteuert, dass sie erst zu der (späteren) Zeit t2 öffnet, nachdem die Reflexion des Eigen- bzw. Scheinwerferlichtes an dem räumlich nahen Partikel RP1 zeitlich abgelaufen ist. Die Zeitachse in Fig. 9 ist dabei auch wie eine räumliche Achse zu verstehen, da nach Multiplikation mit der konstanten Lichtgeschwindigkeit c sich die Strecken ergeben (d = ct), und umgekehrt sich die entsprechend Zeiten t ergeben, wenn man die Summe der vom Licht zurückgelegten Strecken durch die konstante Lichtgeschwindigkeit c dividiert (t2 = (d + d1)/c). Nachdem das Licht die Strecken d (Scheinwerfer bis naher Partikel) und d1 (naher Partikel bis Brille) durchlaufen hat, ist also die Zeit t2 verstrichen. Wenn der Shutter der Brille aber erst nach Verstreichen der Zeit t2 öffnet, so wie in Fig. 9 mit TR (= on) dargestellt, so wird der Lichtreflex unterdrückt (supp in Fig. 9) und ist dementsprechend nicht sichtbar.

Schneeflocken oder sonstige Partikel (oder auch Nebel) werden dadurch nicht wirklich unsichtbar - sie erscheinen vielmehr als schwarze Punkte -, aber die Gesamtsicht in die Raumtiefe hinein wird aufgrund verminderter Blendung deutlich verbessert.

Eigenlichterkennung bzw. -Unterdrückung

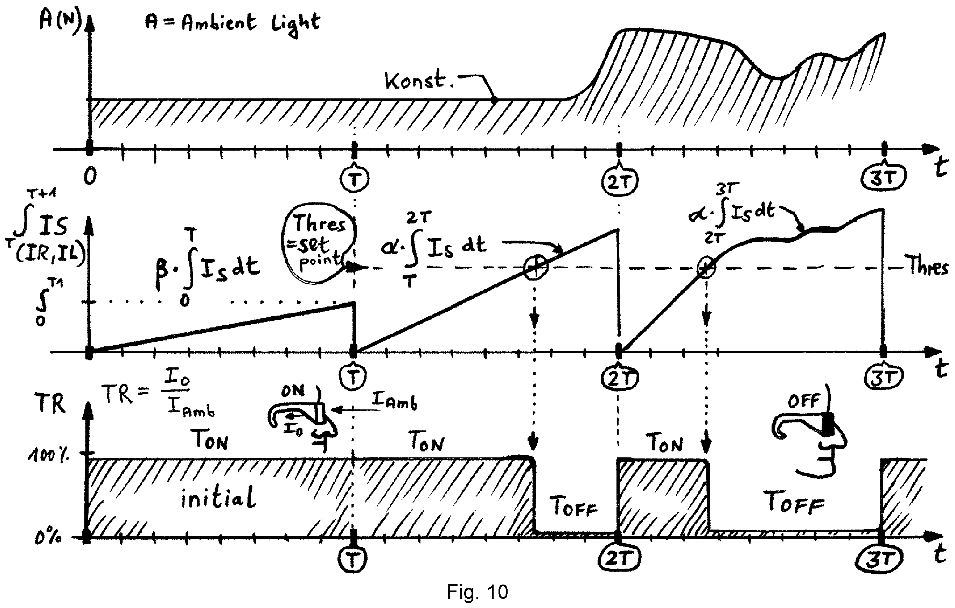

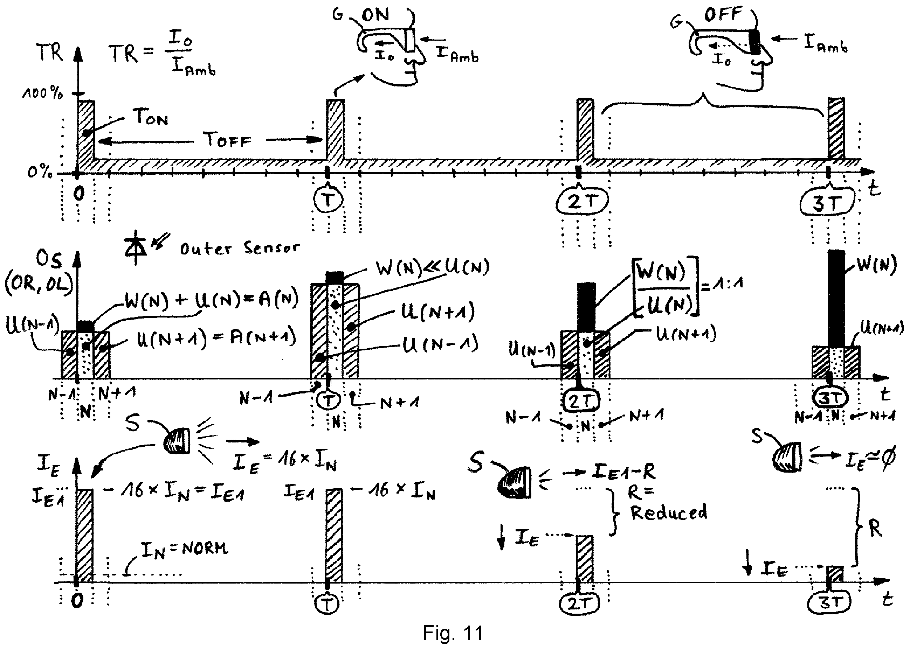

Im Folgenden wird auf die Figuren 10 und 11 Bezug genommen. A bedeutet Umgebungslicht (ambient), U Fremd- oder Störlicht (unwanted, z.B. Sonnenlicht), und W Eigenlicht (wanted). Die Unterscheidung zwischen U und W erfolgt wie nachfolgend beschrieben:
Da der Microkontroller die Zeitpunkte kennt, in denen er den eigenen Scheinwerfer W einschaltet, kann er den äußeren Fotosensor, der mehr als hinreichend schnell ist, in einem Zeitschlitz kurz vor (oder kurz nach) dem ausgesendeten Lichtpuls abfragen - dargestellt in Fig. 10 durch N-1 oder N+1, wobei N den Nten Zeitschlitz des ausgesendeten Lichtpulses beschreibt. Es gilt:

$$A\,(t) = U\,(t) + W\,(t) \qquad (1),$$

beziehungsweise diskret abgefragt, mit N = Mittelwert aus einem Zeitschlitz N gemäß Fig. 10:

$$A\,(N) = U\,(N) + W\,(N)$$

Es wird unterstellt, dass sich das Störlicht zeitlich "kurz vor oder kurz nach" dem Lichtpuls nicht wesentlich ändert, da der Zeitraum zwischen N-1 und N und N+1 sehr klein ist.

$$U\,(N) = U\,(N\text{-}1) = U\,(N\text{+}1) \qquad (3)$$

Es können auch andere, z.B. komplexere, erfahrungsbasierte Mittelungsverfahren gewählt werden, oder auch das einfache arithmetische Mittel. Jedenfalls wird davon ausgegangen, dass mit dieser Methodik der Störlichtwert Wert U (N) im Zeitschlitz N mit sehr hoher Genauigkeit ermittelt werden kann, sofern das Umgebungslicht sich nicht sehr schnell ändert und nicht seinerseits gepulst ist. Geht man nun davon aus, dass das Zusatzlicht vom Eigenscheinwerfer sich zum Umgebungslicht addiert gemäß Formel (1), dann gilt für A (N), dass diese stets grösser ist als das Umgebungslicht in den Nachbarzeitschlitzen:

$$A\,(N) > A\,(N\text{-}1)\ \text{und}\ A\,(N) > A\,(N\text{+}1)$$

Weiterhin gilt, dass die normale Eigenlicht-Rück-Reflexion von entfernten und nicht sehr spiegelnden Gegenständen, d.h. von einer normalen Szenerie / Umwelt (Strasse, Wald, Feld, im Haus mit großen Räumen), eher gering ist, verglichen mit einem massiven Störlicht wie einer tiefstehenden Sonne, so dass im extremen Einsatzfall der massiven Blendunterdrückung gilt:

$$W\,(N) << U\,(N) \qquad (6)$$

Oft spricht man bei sehr kleinen Größen auch von einem "Delta", welches hinzukommt oder wegfällt, so dass hier die Formel (1) auch geschrieben werden kann als:

$$Delta\,(N) = W\,(N) = A\,(N) - U\,(N) \qquad (7)$$

Da bei einem 70 Hz System binnen einer Sekunde immerhin 70 Mal ein Delta (N) gemessen wird, können diese Werte ihrerseits gemittelt werden, z.B. über einen sinnvollen kleinen Zeitraum, der schnell genug ist, um das Auge hinreichend zu schützen, bezüglich potentiellem Notausschalten oder Herunterregeln der Eigenscheinwerfer beim versehentlichen Blick in diesen Scheinwerfer, z.B. über einen Zeitraum von einem Drittel oder einem Achtel einer Sekunde (x = z.B. 125 ms bis 300ms):
Mittelwert: MDelta (N) = MW (N) = z.B. fließender arithmetischer Mittelwert von allen W (N) in Zeitraum T = t bis t+x
Dieser Wert kann dann einer Schwellwert-Abschaltung zugeführt werden - oder für eine gleichmäßigere (analoge) Herunterregelung der Eigenscheinwerfer herangezogen werden.

Beispiel:

S = Entscheidungsschwelle zur Notausschaltung der Eigenscheinwerfer
W (N) < S Eigenscheinwerfer läuft normal weiter
W (N) >= S Eigenscheinwerfer wird abgeschaltet
Als Faustformel kann gelten, dass ein um ein empirisch ermitteltes Vielfaches M (Multiplikator) heller
erscheinendes Licht als Schwelle dient:

$$S = M * U (N) \quad (8.1)$$

Oder - falls man sich nicht auf U (N) beziehen will, d.h. sich von sogenannten "Szenarien", wie
massive oder keine Blendung unabhängig machen will - dann formuliert man einfach selbstbezüglich
durch Vielfache von W(N), z.B.:

$$S = 50\% \text{ bis } 500\% \text{ vom üblichen Erfahrungswert von W(N)} \quad (8.2).$$

In Fig. 10 wird angenommen, dass die Brille sich im "Nachtmodus" am Regelanschlag befindet, so dass alle $T_{ON}$ Zeiten gleich schmal sind (z.B. 5% von der Zykluszeit T). Als voll ausgefüllter schwarzer Balken ist in dem Graph in der Bildmitte das erwünschte Licht W(N) dargestellt. Da eine Rückreflektion von einem Objekt in Normalfall nur sehr schwach ist, ist der schwarze Balken für die ersten beiden Zyklen sehr klein dargestellt. Gleichgültig wieviel sonstiges Störlicht U hinzukommt, beispielhaft dargestellt im Zyklus T, das unten dargestellte Scheinwerferlicht bleibt auf konstanter Intensität IE1, d.h. der Scheinwerfer hat mit 16 x IN beispielsweise schon sein Intensitätsmaximum erreicht, welches nicht weiter gesteigert werden kann. Verändert sich jedoch das Verhältnis von Wanted zu Unwanted erheblich, wie in 2T dargestellt (1:1), so wird die Scheinwerferintensität reduziert R. Im Extremfall 3T kann bei direktem Blick in den Eigenscheinwerfer auch abgeschaltet werden (IE nahe Null).

Messung mit dem Innensensor IS - in Verbindung mit kurzen einmaligen Eigenlichtaussetzern

Außerdem kann man alternativ zum obigen Verfahren oder zu Testzwecken in einem Zyklus T wie oben beschrieben das Delta, also W(N), messen, um dann ausnahmsweise und ausschließlich nur in dem Folge-Zyklus 2T die Lampe S anstelle des erwarteten Lichtpulses aus lässt, also einen Lichtpuls als Schlupf bewusst aussetzt. Weil solch ein vereinzelter "Aussetzer" im Zyklus 2T nur ein einziger von insgesamt 70 Lichtpulsen pro Sekunde ist (bei einem 70 Hz System), wird dies vom Nutzer und von außenstehenden Dritten nicht bemerkt.

Liegt DC Störlicht vor oder läuft die Brille synchron zu einem AC Störlicht, kann man sogar voraussetzen, dass sich das Störlicht nicht nur in dem sehr kurzen Zeit-Intervall N-1, N, N+1 kaum ändert, sondern sogar von einem Zyklus T bis zum nächsten Zyklus 2T weitgehend konstant bleibt:

$$U(N, T) = U(N, 2T) \quad (9)$$

Der Innen-Sensor IS kann dann im Zyklus T das Delta W(N) messen, während im Zyklus 2T dieses Delta W(N) wegen des ausgeschalteten Eigenscheinwerfers nicht mehr erscheint. Somit kann im gleichen Zeitschlitz N, mittels Innensensor, eine Zusatzmessung von W(N) erfolgen, ohne dass man auf die oben erklärte Messung mit dem Außensensor (in den Zeitschlitzen N-1, N, N+1) angewiesen ist. Wendet man beide Verfahren (also Innensensor mit einmalig ausgeschalteter Lichtquelle und Außensensor mit Messung von A (N-1, N, N+1) gleichzeitig an, so kann mit dieser Redundanz die Genauigkeit und Verlässlichkeit der W(N) Messung noch erhöht werden. Widersprüchliche oder unlogische Messungen können bei gleichzeitiger Anwendung beider Verfahren durch den Mikrocontroller erfasst und entsprechend korrigiert werden.

Keine DC Gegenlichtquelle, aber versehentlicher Blick in die Eigenlichtquelle

Es kann im Extremfall angenommen werden, dass bei tief schwarzer Nacht und störungsfreier Sicht (z.B. völlig allein im Wald) gilt:

$$U (N) = 0$$

Daraus folgt mit obiger Formel (2) = A (N) = U (N) + W (N), dass gilt:

$$A\,(N) = W\,(N)$$

Die Brille kann in diesem Falle auch komplett offen/transparent sein, und der Scheinwerfer auch dauerhaft an bzw. scheinbar oder weitgehend dauerhaft an (z.B. vereinzelte Messpulse alle 300ms), so dass weiterhin die oben beschriebenen Delta-Messungen stattfinden können. Erst mit plötzlichem Auftreten von Störlicht wird die Brille automatisch wieder in den üblichen PWM-Modulationsmodus überführt.

Starke AC-Gegenlichtquelle, wie z.B. elektrische Kunstlichtquelle, die z.B. aus dem 50/60Hz Niederspannungsnetz gespeist wird

Der Aussensensor OS bzw. OL, OR verfügt über drei wesentliche Eigenschaften:

1) Er ist vergleichsweise wesentlich schneller als industrielles Kunstlicht (100 - 120 Hz) und kann dieses elektronisch auflösen und mittels Mikrokontroller problemlos erkennen.

2) Er ist zudem wie ein Messgerät genormt (kann Werte in Lux oder vergleichbaren Lichttechnischen Einheiten bzw. in zugehörigen Spannungsäquivalenten Werten ausgeben) und ist mit der menschlichen Augenempfindlichkeitskurve gewichtet, so dass er auch Lichtstärke messen kann.

3) Er ist vorzugsweise, aber nicht notwendigerweise, baugleich mit dem Innen-Sensor IS, so dass der Micro-Controller ohne aufwendige Umrechnung, sofort in Echtzeit "Vergleichsmessungen" zwischen Innen (durch das LCD hindurch) und Außen (am LCD vorbei) machen kann.

Gibt es nur eine einzelne dominante Kunstlichtquelle, so dass eine zyklische 100 / 120 Hz Schwingung vom Außen-Sensor detektiert werden kann, so legt dieser den Startzeitpunkt $T_{Null}$ der Grundfrequenz der PWM der Brille fest - sowie die Frequenz der PWM - so dass beide Systeme derart synchron laufen, dass das Helligkeitsmaximum der externen Lichtquelle immer genau am Anfang eines Zyklus steht und sofort vom Außen-Sensor OS und auch vom Innensensor IS gemessen werden kann. Der Innensensor IS kann diese maximale Helligkeit der Kunstlichtquelle ebenfalls messen, weil am Anfang eines Zyklus die Brille stets "offen" ist, d.h. die Flüssigkeitskristallzelle transparent ist. Somit messen der Außensensor OS und der Innensensor IS im Grunde das gleiche Licht, nur mit dem kleinen Unterschied, dass sich vor dem Innensensor IS das transparente LCD befindet, so dass IS etwas weniger Licht abbekommt - nämlich abzüglich der temperaturabhängigen und alterungsabhängigen Transmission im Durchlasszustand - z.B. 50% weniger bei gekreuzten Polfiltern (Polarisator-Analysator-Stellung).

Weiterhin sind die Innen- und Außen-Sensoren IS1 und OS1 auch räumlich sehr nah angeordnet, auf einer gedachten Achse z.B. maximal 3 mm auseinander liegend - auch "Messpärchen No.1" (MP1) genannt. Somit können auch Ortsfrequenzen OF (i.w.S. "Streifenmuster") von OF > 3 mm zu keinerlei Messfehler führen. Zudem existiert ein weiteres Messpärchen MP2 bestehend aus IS2 und OS2 jeweils orthogonal zum vorgenannten Messpärchen MP1, so dass Schachbrettmuster, also Ortsfrequenzen, die senkrecht zu vorgenannten Ortsfrequenzen verlaufen, entsprechend erfasst werden können, sofern diese mehr als 3 mm betragen. Beide Messpärchen (MP1 und MP2) liefern Werte, die vom Mikrocontroller derart ausgewertet werden können, dass "geometrische Mittelwerte" gemäß dem gedachten Dreieck zwischen Pupillen-Mittelpunkt-Stellung und Sensoranordnung gebildet werden können.

Integration binnen eines Zyklus

Der Innensensor IS misst das durch das LC auftreffende Licht und integriert dieses Licht im Rahmen einer Leerlauf-Initialisierungs-Phase im allerersten Zyklus von 100 oder 120 Hz in dem die Brille komplett offen bleibt (siehe Fig. 11). Da es nur ein einziger Zyklus aus einem synchronen 100 oder 120 Hz System ist (also von weiteren folgenden 109 oder 119 geregelten Zyklen), nimmt das menschliche Auge dies nicht wahr. Es liegt aber ein erstes Integrationsergebnis vom Zyklus T vor.

Bildet der Sensor IS dabei ein Integral über beispielsweise eine Konstante (DC Störlicht), so ergibt sich eine gerade aufsteigende Linie (siehe Fig.11), die nach Überschreiten einer Sollwert-Schwelle (Sollwert-Trigger) das komplette Schließen der Brille veranlasst (hartes on-off Keying via PWM). Dies hat zudem den Vorteil, dass noch binnen eines jeweiligen Zyklus T eine Entscheidung und Reaktion erfolgt, ohne dass man den T+1 oder T-1 oder sonstige weitere Zyklen mit einbeziehen muss, so wie es bei einer "analog mathematischen Berechnung in der Frequenzdomain eines PIDReglers" normalerweise nötig wäre. Es ist also keinerlei Fourier-Transformation nötig - weder FFT noch FT, DFT, usw.

Die sogenannte Regelung ist also in diesem Falle "hart" ausgelegt und reagiert in Echtzeit bereits im Zyklus T auf einen Sollwert - auch "mikroskopische Regelung" genannt.

Die sogenannte "Makroskopische Regelung":

Davon unbenommen kann aber dieser mikroskopische Integrationswert aus dem N-ten Zyklus in einem flüchtigen Zwischenspeicher abgelegt werden, so dass er für weitere nachfolgende Integrationswerte als "fließender/gleitender Mittelwert Korrekturwert", d.h. als Makroskopischer Integrationswert zur Verfügung steht - etwa binnen eines Viertels oder Drittels eines 100 Hz oder 120 Hz Zyklus (also binnen unmerklichen Sekundenbruchteilen).

Somit reagiert die Regelung im Falle von schwankendem Kunstlicht immer richtig. Fig. 11 zeigt hierzu die Initialisierungsphase mit noch unbekanntem Ausgang bzw. unbekannter Außenhelligkeit (Gewichtungsfaktor Beta), danach im Zyklus 2 gefolgt von einem auf 1 bzw. maximale Helligkeit und Modulationshub normierten Zyklus (Gewichtungsfaktor Alpha), in dem das Integral bei Erreichen eines Sollwertes Thres das Schließen der Brille veranlasst ($T_{OFF}$).

Im dritten Zyklus ist beispielsweise gezeigt, wie die Außenhelligkeit sich erhöht hat und zudem schwankt. Das entsprechende Integral (Graph in der Bildmitte) läuft nun steiler an, so dass der Sollwert Thres schneller erreicht wird und dementsprechend die Brille zeitlich früher schließt - $T_{OFF}$ ist also länger als im Zyklus zuvor. Die Integralwerte werden am Ende eines jeden Zyklus auf null gesetzt, so dass jeder Zyklus in Echtzeit in seiner Durchlässigkeit TR geregelt wird.

Szenario: Mehrere starke AC Gegenlichtquellen, wie z.B. elektrische Kunstlichtquellen, die z.B. aus diversen Netzen gespeist werden, so dass Frequenzgemische vorliegen.

Ein Gemisch von diversen sich überlagernden Frequenzen kann dazu führen, dass der Außensensor nicht mehr auf eine bestimmte Störfrequenz synchronisieren kann. Dies kann aber auch Vorteile haben, da sich ein Gemisch im Oszillogramm wie "Rauschen" darstellt, welches kaum mehr Täler und Aussetzer von Fremdlicht aufweist, da es aufgrund von Überlagerungen ein stabiles "Grundrauschen" gibt. In diesem Falle wird die Brille bzw. der Mikrocontroller den Versuch der Synchronisation abbrechen und einfach auf eine typische voreingestellte Arbeitsfrequenz schalten, z.B. auf 70 Hz, um dort unbeirrt zu arbeiten, gemäß obigem Integrationsschema.

Szenario: Mehrere starke gepulste Gegenlichtquellen, wie z.B. elektrische LED Kunstlichtquellen, z.B. wie das vorliegende oder ähnliche Systeme

Aufgrund der sofortigen Integration innerhalb eines Zyklus kann die Brille entsprechend zu machen, sobald ein Schwellwert erreicht ist. Da der Dynamikbereich und die Messgeschwindigkeit der Außen- und Innensensoren stets schneller und besser als das menschliche Auge sind, können auch extreme Intensitäten und schädliche Leistungen schützend abgewendet werden, wie z.B. extrem kurze Lichtpulse hoher Energie, wie sie z.B. von gepulsten Q-Switch-Lasern oder überpulsten LED stammen können.

Das menschliche Auge kann ab einer bestimmten anwachsenden Intensität bei gleichzeitig immer kürzer werdenden Pulsen dies nicht mehr vernünftig wahrnehmen und reagieren, so dass die Hornhaut und Netzhaut in Gefahr sind, Schaden zu nehmen.

Reaktion der Brille im Zweifelsfall:

Die Brille macht daher im Zweifelsfall bei hohen Intensitäten eher "zu" (Augenschutz) - während sie bei geringen Intensitäten, aber chaotischen, nicht näher interpretierbaren Frequenzmustern, auf die auch nicht synchronisiert werden kann, tendenziell eher "auf" gemacht wird, und versucht wird eine Art "mittlere Helligkeit" zu bestimmen, indem über mehrere Zyklen T integriert und gemittelt wird (z.B. über 300ms), so als wäre es Rauschen bzw. eine nahezu gleichförmige Quelle - während sie sich aber grundsätzlich im PWM Nachtsicht- und Dämmerungs-Betrieb befindet (5% bis ca. 20% offener PWM-Zeitschlitz mit entsprechend gepulstem Scheinwerfer).

Es wird also eine Brille vorgeschlagen. Die Brille hat ein Brillenglas mit einer Flüssigkristallzelle LC, deren Transmission TR zwischen transmittierend und blockierend umgeschaltet werden kann. Ferner weist die Brille einen Eye-Tracker ET auf, der die Blickrichtung des Auges bestimmen kann. Ferner mindestens einen Sensor IL, IR zum Messen der Helligkeit des auf ihn treffenden sichtbaren Lichts, wobei der Sensor augenseitig des Brillenglases angeordnet ist und die Helligkeit durch das mindestens eine Brillenglas ortsaufgelöst misst. Der Sensor kann die Helligkeit des aus der mit dem Eye-Tracker bestimmten Blickrichtung des Auges auf ihn treffenden sichtbaren Lichts bestimmen. Die Brille verfügt ferner über einen geschlossenen Regelkreis für die Regelung der Transmission der Flüssigkristallzelle, wobei ein Sollwert für die Helligkeit am Auge vorgegeben ist, und wobei der Regelkreis als Istwert die vom Sensor gemessene Helligkeit in Blickrichtung des Auges heranzieht.

Die Gegenstände der Erfindung können auch durch die folgenden Klauseln beschrieben werden:

1. Brille für einen Träger mit mindestens einem Auge, mit

1.1 mindestens einem Brillenglas;

1.1.1 wobei das mindestens eine Brillenglas eine Flüssigkristallzelle (LC) aufweist, deren Transmission (TR) durch eine geeignete Ansteuerung veränderbar ist;

1.2 einem Eye-Tracker (ET), der die Blickrichtung des Auges bestimmen kann;

1.3 mindestens einem Sensor (IL, IR) zum Messen der Helligkeit des auf ihn treffenden sichtbaren Lichts;

1.3.1 wobei der mindestens eine Sensor (IL, IR) augenseitig des Brillenglases angeordnet ist;

1.3.2 wobei der mindestens eine Sensor (IL, IR) die Helligkeit durch das mindestens eine Brillenglas messen kann;

1.3.3 wobei der mindestens eine Sensor (IL, IR)

1.3.3.1 ein abbildendes System mit einer Kamera oder

1.3.3.2 mindestens drei Sensoren, die ein Koordinatensystem aufspannen oder

1.3.3.3 ein Facettenauge

aufweist;

1.3.4 wobei der mindestens eine Sensor (IL, IR) die Helligkeit des aus der mit dem Eye-Tracker (ET) bestimmten Blickrichtung des Auges auf ihn treffenden sichtbaren Lichts bestimmen kann; und

1.4 einem geschlossenen Regelkreis (MC) für die Regelung der Transmission der Flüssigkristallzelle (LC);

1.4.1 wobei ein Sollwert für die Helligkeit am Auge vorgegeben ist;

1.4.2 wobei der Regelkreis als Istwert die von dem mindestens einen Sensor (IL, IR) gemessene Helligkeit in Blickrichtung des Auges heranzieht.

2. Brille nach der vorhergehenden Klausel,
**dadurch gekennzeichnet,**
dass die Flüssigkristallzelle (LC) derart ausgebildet ist, dass sie ihre Transmission von 90% auf 10% und von 10% auf 90% in maximal 10 ms ändern kann.

3. Brille nach der vorhergehenden Klausel,
**dadurch gekennzeichnet,**

3.1 dass die Flüssigkristallzelle (LC) derart ausgebildet ist, dass die Transmission (TR) der Flüssigkristallzelle (LC) zwischen Zuständen hoher und niedriger Transmission umgeschaltet werden kann;
3.2 dass Mittel zum Regeln oder Steuern der Zeiten des Zustands hoher ($T_{on}$) und niedriger ($T_{off}$) Transmission der Flüssigkristallzelle (LC) sowie des Wechsels zwischen diesen beiden Zuständen vorhanden sind;
3.2.1 wobei die Regelung oder Steuerung und der geschlossene Regelkreises (MC) derart ausgebildet sind, dass die Zeiten des Zustands hoher Transmission ($T_{on}$) mit zunehmender Helligkeit des auf den mindestens einen Sensor (IL, IR) treffenden sichtbaren Lichts kürzer werden;
3.2.2 wobei der Wechsel zwischen den Zuständen hoher ($T_{on}$) und niedriger ($T_{off}$) Transmission mit einer zeitlichen Frequenz stattfindet, die das menschliche Auge nicht auflösen kann.

4. Brille nach einer der vorhergehenden Klauseln,
**dadurch gekennzeichnet,**
dass der Regelkreis derart ausgebildet ist, dass er bei der Bestimmung der Helligkeit aus der Blickrichtung des Auges eine nutzerspezifische Augen/Netzhaut-Empfindlichkeitskurve zur Gewichtung der Helligkeit berücksichtigen kann.

5. Brille nach einer der vorhergehenden Klauseln,
**gekennzeichnet durch**

ein Brillengestell (F); wobei

das Brillengestell das dem mindestens einen Brillenglas zugeordnete Auge lichtdicht gegenüber dem Umgebungslicht abdichtet.

6. Brille nach einer der vorhergehenden Klauseln,
**dadurch gekennzeichnet,**
dass der Sollwert des Regelkreises eine Helligkeit am Auge von 20 bis 400 lx vorgibt.

7. Brille nach einer der vorhergehenden Klauseln,
**dadurch gekennzeichnet,**
dass die Helligkeit des Umgebungslichts aus dem Sollwert und einem Stellsignal des Regelkreises erschlossen wird.

8. Brille nach einer der vorhergehenden Klauseln,
**gekennzeichnet durch**
mindestens einen weiteren Helligkeits-Sensor (OL, OR), der auf der vom Auge abgewandten Seite der Brille angeordnet ist (Außensensor) und die Helligkeit des Umgebungslichts bestimmt.

9. Brille nach einer der beiden vorhergehenden Klauseln,
**dadurch gekennzeichnet,**

    9.1 dass der Sollwert des Regelkreises abhängig von der Helligkeit des Umgebungslichts verändert werden kann; und
    9.2 dass die Veränderung des Sollwerts um mindestens einen Faktor 10 langsamer ist als die Regelung der Transmission der Flüssigkristallzelle.

10. Brille nach der vorhergehenden Klausel,
**dadurch gekennzeichnet,**

    10.1 dass die Veränderung des Sollwerts in vorgegebenen Stufen erfolgt;
    10.2 wobei die stufenweise Veränderung des Sollwerts um mindestens einen Faktor 100 langsamer ist als die Regelung der Transmission der Flüssigkristallzelle.

11. Brille nach der vorhergehenden Klausel,
**dadurch gekennzeichnet,**
dass die Regelung derart ausgebildet ist, dass sie auf extreme Helligkeitswerte innerhalb von 10 μs bis einer Sekunde derart reagiert, dass die Flüssigkristallzelle (LC) auf den Zustand niedriger Transmission eingestellt wird.

12. Brille nach einer der vorhergehenden Klauseln,
**gekennzeichnet durch**

    12.1 zwei Brillengläser für zwei Augen eines Brillenträgers;
    12.2 je einen augenseitigen Sensor für jedes Brillenglas zum Messen der Helligkeit des auf das jeweilige Auge treffenden sichtbaren Lichts; und durch
    12.3 je einen Regelkreis für jedes Brillenglas.

13. Brille nach der vorhergehenden Klausel,
**dadurch gekennzeichnet,**
dass die Sollwerte für die beiden Augen um 1% bis 60% voneinander abweichen.

14. Brille nach Klausel 12,
**dadurch gekennzeichnet,**
dass bei der Regelung der Helligkeit des auf ein Auge treffenden sichtbaren Lichts die Regelung der Helligkeit für das andere Auge berücksichtigt wird.

15. Brille nach Klausel 1,
**gekennzeichnet durch**

    15.1 mindestens eine Lichtquelle (S), die an der vom Auge abgewandten Seite der Brille angeordnet ist;

15.2 wobei die Lichtquelle abhängig von der Blickrichtung des Brillenträgers gesteuert werden kann.

16. Brille nach der vorhergehenden Klausel,
**gekennzeichnet durch**

16.1 Mitteln zum Steuern oder Regeln der Leuchtzeiten und der Leuchtintensität der Lichtquelle (S) derart, dass diese während der Zeiten des Zustands hoher Transmission ($T_{on}$) der Flüssigkristallzelle (LC) leuchtet;
16.2 wobei das zeitliche Integral des Produkts aus
16.2.1 der Leuchtintensität der Lichtquelle (S) und
16.2.2 der Transmission (TR) der Flüssigkristallzelle (LC)
bei einer Veränderung der Zeiten des Zustands hoher Transmission ($T_{on}$) innerhalb einer vorgegebenen Toleranz konstant bleibt.

17. Brille nach Klausel 1,
**gekennzeichnet durch**

- eine Lichtquelle zum Blenden eines Lebewesens, eines optischen Sensors oder einer Kamera, und/oder
- eine Anzeige an der vom Auge abgewandten Seite des Brillenglases und/oder
- eine Anzeige augenseitig des Brillenglases und/oder
- ein Head-Up-Display (HUD).

18. Brille nach einer der vorhergehenden Klauseln,
**dadurch gekennzeichnet,**
dass die Messwerte der Sensoren und/oder die Sollwerte der Regelkreise und/oder die davon abgeleitete Helligkeit der Umgebung mit einem Geokoordinaten-Signal eines Geokoordinaten-Empfängers verbunden und aufgezeichnet werden.

19. Brille nach einer der vorhergehenden Klauseln,
**dadurch gekennzeichnet,**

19.1 dass das mindestens eine Brillenglas eine weitere Flüssigkristallzelle aufweist, deren Transmission durch eine geeignete Ansteuerung veränderbar ist,
19.2 wobei die weitere Flüssigkristallzelle in Blickrichtung hinter oder vor der Flüssigkristallzelle (LC) angeordnet ist.

20. Brille nach einer der vorhergehenden Klauseln in Kombination mit dem System nach einer der Klauseln 69 bis 73.

21. Verfahren zum Regeln der Helligkeit des auf mindestens ein Auge treffenden sichtbaren Lichts, mit folgenden Schritten:
21.1 eine Brille wird zur Verfügung gestellt, wobei die Brille folgendes aufweist:

21.1.1 mindestens ein Brillenglas;
21.1.2 wobei das mindestens eine Brillenglas eine Flüssigkristallzelle (LC) aufweist, deren Transmission (TR) durch eine geeignete Ansteuerung veränderbar ist;
21.2 einen Eye-Tracker (ET), der die Blickrichtung des Auges bestimmt;
21.3 mindestens ein Sensor (IL, IR) zum Messen der Helligkeit des auf den Sensor treffenden sichtbaren Lichts wird zur Verfügung gestellt;
21.3.1 wobei der mindestens eine Sensor (IL, IR) augenseitig des Brillenglases angeordnet ist;
21.3.2 wobei der mindestens eine Sensor (IL, IR) die Helligkeit durch das mindestens eine Brillenglas misst;
21.3.3 wobei der mindestens eine Sensor (IL, IR)
21.3.3.1 ein abbildendes System mit einer Kamera oder
21.3.3.2 mindestens drei Sensoren, die ein Koordinatensystem aufspannen oder
21.3.3.3 ein Facettenauge
aufweist;
21.3.4 wobei der mindestens eine Sensor (IL, IR) die Helligkeit des aus der mit dem Eye-Tracker (ET) bestimmten Blickrichtung des Auges auf ihn treffenden sichtbaren Lichts bestimmt;
21.4 ein geschlossener Regelkreis (MC) für die Regelung der Transmission der Flüssigkristallzelle (LC) wird zur Verfügung gestellt;

21.4.1 wobei ein Sollwert für die Helligkeit am Auge vorgegeben ist;

21.4.2 wobei der Regelkreis als Istwert die vom Sensor gemessene Helligkeit in Blickrichtung des Auges heranzieht.

22. System zum Sichtverbessern durch Blendunterdrückung mit:

22.1 einer Brille für einen Träger mit mindestens einem Auge, mit

22.1.1 mindestens einem Brillenglas;

22.1.1.1 wobei das mindestens eine Brillenglas eine Flüssigkristallzelle (LC) aufweist, deren Transmission durch eine geeignete Ansteuerung veränderbar ist;

22.1.1.1.1 wobei die Flüssigkristallzelle (LC) derart ausgebildet ist, dass die Transmission (TR) der Flüssigkristallzelle (LC) zwischen Zuständen hoher und niedriger Transmission umgeschaltet werden kann; und mit

22.1.2 Mitteln zum Regeln oder Steuern der Zeiten des Zustands hoher Transmission ($T_{on}$) der Flüssigkristallzelle (LC);

22.1.3 mindestens einem Sensor (IL, IR) zum Messen der Helligkeit des auf ihn treffenden sichtbaren Lichts;

22.1.3.1 wobei der mindestens eine Sensor (IL, IR) augenseitig des Brillenglases angeordnet ist;

22.1.3.2 wobei der mindestens eine Sensor (IL, IR) die Helligkeit durch das mindestens eine Brillenglas misst; und mit

22.1.4 einem geschlossenen Regelkreis (MC) für die Regelung der Transmission der Flüssigkristallzelle (LC);

22.1.4.1 wobei die Regelung derart ausgebildet ist, dass die Zeiten des Zustands hoher Transmission ($T_{on}$) mit zunehmender Blendung kürzer werden;

22.1.4.22 wobei ein Sollwert für die Helligkeit am Auge des Brillenträgers vorgegeben ist;

22.1.4.3 wobei der Regelkreis als Istwert die vom Sensor gemessene Helligkeit heranzieht; und mit

22.2 einer Anzeige mit

22.2.1 Mitteln zum Steuern oder Regeln der Leuchtzeiten und der Leuchtintensität der Anzeige derart, dass diese während der Zeiten des Zustands hoher Transmission ($T_{on}$) der Flüssigkristallzelle (LC) leuchtet;

22.2.2 wobei das zeitliche Integral des Produkts aus

22.2.2.1 der Leuchtintensität der Anzeige und

22.2.2.2 der Transmission (TR) der Flüssigkristallzelle (LC)

bei einer Veränderung der Zeiten des Zustands hoher Transmission ($T_{on}$) innerhalb einer vorgegebenen Toleranz konstant bleibt.

23. System nach der vorhergehenden Klausel,
**dadurch gekennzeichnet,**
dass die Anzeige

- eine Anzeige an der vom Auge abgewandten Seite des Brillenglases und/oder
- eine Anzeige augenseitig des Brillenglases und/oder
- ein Head-Up-Display (HUD)

umfasst.

24. System nach einer der Klauseln 22 bis 23,
**dadurch gekennzeichnet,**
dass die Flüssigkristallzelle (LC) derart ausgebildet ist, dass sie ihre Transmission von 90% auf 10% und von 10% auf 90% in maximal 10 ms ändern kann.

25. System nach einer der Klauseln 22 bis 24,
**dadurch gekennzeichnet,**

25.1 dass die Brille ein Brillengestell aufweist, wobei

25.2 das Brillengestell das mindestens eine Auge des Brillenträgers lichtdicht gegenüber dem Umgebungslicht abdichtet.

26. System nach einer der Klauseln 22 bis 25,
**dadurch gekennzeichnet,**
dass der Sollwert des Regelkreises eine Helligkeit am Auge von 20 bis 400 lx vorgibt.

27. System nach einer der Klauseln 22 bis 26,
**dadurch gekennzeichnet,**
dass die Helligkeit des Umgebungslichts aus dem Sollwert und einem Stellsignal des Regelkreises erschlossen wird.

28. System nach einer der Klauseln 22 bis 27,
**gekennzeichnet durch**
mindestens einen weiteren Helligkeits-Sensor (OL, OR), der auf der vom Auge abgewandten Seite der Brille angeordnet ist (Außensensor) und die Helligkeit des Umgebungslichts bestimmt.

29. System nach einer der Klauseln 22 bis 28,
**dadurch gekennzeichnet,**

29.1 dass der Sollwert des Regelkreises abhängig von der Helligkeit des Umgebungslichts verändert werden kann; und
29.2 dass die Veränderung des Sollwerts um mindestens einen Faktor 10 langsamer ist als die Regelung der Transmission der Flüssigkristallzelle.

30. System nach der vorhergehenden Klausel,
**dadurch gekennzeichnet,**

30.1 dass die Veränderung des Sollwerts in vorgegebenen Stufen erfolgt;
30.2 wobei die stufenweise Veränderung des Sollwerts um mindestens einen Faktor 100 langsamer ist als die Regelung der Transmission der Flüssigkristallzelle.

31. System nach einer der Klauseln 22 bis 30,
**dadurch gekennzeichnet,**
dass die Regelung derart ausgebildet ist, dass sie auf extreme Helligkeitswerte innerhalb von 10 $\mu$s bis einer Sekunde derart reagiert, dass die Flüssigkristallzelle (LC) auf den Zustand niedriger Transmission eingestellt wird.

32. System nach einer der Klauseln 22 bis 31,
**dadurch gekennzeichnet,**
dass die Brille

32.1 zwei Brillengläser für zwei Augen eines Brillenträgers;
32.2 je einen augenseitigen Sensor für jedes Brillenglas zum Messen der Helligkeit des auf das jeweilige Auge treffenden sichtbaren Lichts; und durch
32.3 je einen Regelkreis für jedes Brillenglas
aufweist.

33. System nach der vorhergehenden Klausel,
**dadurch gekennzeichnet,**
dass die Sollwerte für die beiden Augen um 1% bis 60% voneinander abweichen.

34. System nach Klausel 31,
**dadurch gekennzeichnet,**
dass bei der Regelung der Helligkeit des auf ein Auge treffenden sichtbaren Lichts die Regelung der Helligkeit für das andere Auge berücksichtigt wird.

35. System nach einer der Klauseln 22 bis 34,
**dadurch gekennzeichnet,**

35.1 dass ein Eye-Tracker (ET) vorhanden ist, der die Blickrichtung des Auges bestimmen kann;
35.2 wobei der mindestens eine Sensor (IL, IR)
35.2.1 ein abbildendes System mit einer Kamera oder
35.2.2 mindestens drei Sensoren, die ein Koordinatensystem aufspannen oder
35.2.3 ein Facettenauge
aufweist;
35.3 wobei der mindestens eine Sensor (IL, IR) die Helligkeit des aus der mit dem Eye-Tracker (ET) bestimmten Blickrichtung des Auges auf ihn treffenden sichtbaren Lichts bestimmen kann; und

35.4 wobei der Regelkreis (MC) als Istwert die vom Sensor gemessene Helligkeit in Blickrichtung des Auges heranzieht.

36. System nach einer der Klauseln 22 bis 35,
**dadurch gekennzeichnet,**
dass der Regelkreis derart ausgebildet ist, dass er bei der Bestimmung der Helligkeit aus der Blickrichtung des Auges eine nutzerspezifische Augen/Netzhaut-Empfindlichkeitskurve zur Gewichtung der Helligkeit berücksichtigen kann.

37. System nach einer der Klauseln 22 bis 36,
**dadurch gekennzeichnet,**
dass die Messwerte der Sensoren und/oder die Sollwerte der Regelkreise und/oder die davon abgeleitete Helligkeit der Umgebung mit einem Geokoordinaten-Signal eines Geokoordinaten-Empfängers verbunden und aufgezeichnet werden.

38. System nach einer der Klauseln 22 bis 37,
**dadurch gekennzeichnet,**

38.1 dass das mindestens eine Brillenglas eine weitere Flüssigkristallzelle aufweist, deren Transmission durch eine geeignete Ansteuerung veränderbar ist,
38.2 wobei die weitere Flüssigkristallzelle in Blickrichtung hinter oder vor der Flüssigkristallzelle (LC) angeordnet ist.

39. Verfahren zum Sichtverbessern durch Blendunterdrückung mit folgenden Schritten:
39.1 eine Brille für einen Träger mit mindestens einem Auge wird zur Verfügung gestellt, wobei die Brille derart ausgebildet wird, dass sie folgendes aufweist:

39.1.1 mindestens ein Brillenglas;
39.1.1.1 wobei das mindestens eine Brillenglas eine Flüssigkristallzelle (LC) aufweist, deren Transmission durch eine geeignete Ansteuerung veränderbar ist;
39.1.1.1.1 wobei die Flüssigkristallzelle (LC) derart ausgebildet ist, dass die Transmission (TR) der Flüssigkristallzelle (LC) zwischen Zuständen hoher und niedriger Transmission umgeschaltet werden kann;
39.1.2 Mittel zum Regeln oder Steuern der Zeiten des Zustands hoher Transmission ($T_{on}$) der Flüssigkristallzelle (LC);
39.1.3 mindestens einen Sensor (IL, IR) zum Messen der Helligkeit des auf ihn treffenden sichtbaren Lichts;
39.1.3.1 wobei der mindestens eine Sensor (IL, IR) augenseitig des Brillenglases angeordnet ist;
39.1.3.2 wobei der mindestens eine Sensor (IL, IR) die Helligkeit durch das mindestens eine Brillenglas misst;
39.1.4 einen geschlossenen Regelkreis (MC) für die Regelung der Transmission der Flüssigkristallzelle (LC);
39.1.4.1 wobei die Regelung derart ausgebildet wird, dass die Zeiten des Zustands hoher Transmission ($T_{on}$) mit zunehmender Blendung kürzer werden;
39.1.4.2 wobei ein Sollwert für die Helligkeit am Auge des Brillenträgers vorgegeben wird;
39.1.4.3 wobei der Regelkreis als Istwert die vom Sensor gemessene Helligkeit heranzieht;
39.2 eine Anzeige wird zur Verfügung gestellt, wobei die Anzeige derart ausgebildet wird, dass sie folgendes aufweist:

39.2.1 Mittel zum Steuern oder Regeln der Leuchtzeiten und der Leuchtintensität der Anzeige derart, dass diese während der Zeiten des Zustands hoher Transmission ($T_{on}$) der Flüssigkristallzelle (LC) leuchtet;
39.2.2 wobei das zeitliche Integral des Produkts aus
39.2.2.1 der Leuchtintensität der Anzeige und
39.2.2.2 der Transmission (TR) der Flüssigkristallzelle (LC)
bei einer Veränderung der Zeiten des Zustands hoher Transmission ($T_{on}$) innerhalb einer vorgegebenen Toleranz konstant bleibt.

40. System zum Sichtverbessern durch Blendunterdrückung mit:

40.1 einer Brille für einen Träger mit mindestens einem Auge, mit
40.1.1 mindestens einem Brillenglas;

40.1.1.1 wobei das mindestens eine Brillenglas eine Flüssigkristallzelle (LC) aufweist, deren Transmission (TR) durch eine geeignete Ansteuerung veränderbar ist;

40.1.1.1.1 wobei die Flüssigkristallzelle (LC) derart ausgebildet ist, dass die Transmission (TR) der Flüssigkristallzelle (LC) zwischen Zuständen hoher und niedriger Transmission umgeschaltet werden kann; und mit

40.1.2 Mitteln zum Regeln oder Steuern der Zeiten des Zustands hoher Transmission ($T_{on}$) der Flüssigkristallzelle (LC);

40 und mit

40.2 einer Lichtquelle (S) mit

40.2.1 Mitteln zum Steuern oder Regeln der Leuchtzeiten und der Leuchtintensität der Lichtquelle (S) derart, dass diese während der Zeiten des Zustands hoher Transmission ($T_{on}$) der Flüssigkristallzelle (LC) leuchtet;

40.2.2 wobei das zeitliche Integral des Produkts aus

40.2.2.1 der Leuchtintensität der Lichtquelle (S) und

40.2.2.2 der Transmission (TR) der Flüssigkristallzelle (LC)

bei einer Veränderung der Zeiten des Zustands hoher Transmission ($T_{on}$) innerhalb einer vorgegebenen Toleranz konstant bleibt;

40.3 wobei die Regelung oder Steuerung der Flüssigkristallzelle (LC) und der Lichtquelle (S) derart ausgebildet ist,

40.3.1 dass die zeitliche Position der Zeiten des Zustands hoher Transmission ($T_{on}$) innerhalb einer Periode aus Zeiten des Zustands hoher Transmission ($T_{on}$) und Zeiten des Zustands niedriger Transmission ($T_{off}$) kontinuierlich oder sprunghaft verändert werden kann; und/oder

40.3.2 dass die Dauer einer Periode aus Zeiten des Zustands hoher Transmission ($T_{on}$) und Zeiten des Zustands niedriger Transmission ($T_{off}$) kontinuierlich oder sprunghaft verändert werden kann;

40.3.3 wobei die Veränderungen durch einen geheimen Codierschlüssel bestimmt werden.

41. System nach der vorhergehenden Klausel,
**dadurch gekennzeichnet**

41.1 dass die Brille ferner mindestens einen Sensor (IL, IR) zum Messen der Helligkeit des auf den Sensor treffenden sichtbaren Lichts aufweist;

41.1.1 wobei der mindestens eine Sensor (IL, IR) augenseitig des Brillenglases angeordnet ist;

41.1.2 wobei der mindestens eine Sensor (IL, IR) die Helligkeit durch das mindestens eine Brillenglas misst;

41.2 dass die Brille ferner einen geschlossenen Regelkreis (MC) für die Regelung der Transmission der Flüssigkristallzelle (LC) umfasst;

41.2.1 wobei ein Sollwert für die Helligkeit am Auge des Brillenträgers vorgegeben ist;

41.2.2 wobei der Regelkreis als Istwert die vom Sensor gemessene Helligkeit heranzieht.

42. System nach der vorhergehenden Klausel,
**dadurch gekennzeichnet,**

42.1 dass der mindestens eine Sensor (IL, IR)

42.1.1 ein abbildendes System mit einer Kamera oder

42.1.2 mindestens drei Sensoren, die ein Koordinatensystem aufspannen, oder

42.1.3 ein Facettenauge

aufweist;

42.2 dass die Brille ferner einen Eye-Tracker (ET) aufweist, der die Blickrichtung des Auges bestimmen kann;

42.3 dass der mindestens eine Sensor die Helligkeit des aus der mit dem Eye-Tracker (ET) bestimmten Blickrichtung des Auges auf ihn treffenden sichtbaren Lichts bestimmen kann; und

42.4 dass der Regelkreis als Istwert die vom Sensor gemessene Helligkeit in Blickrichtung des Auges heranzieht.

43. System nach einer der vorhergehenden Klauseln,
**gekennzeichnet durch**

43.1 eine zweite Lichtquelle zum Blenden eines Lebewesens, eines optischen Sensors oder einer Kamera,

43.2 die während der Zeiten des Zustands niedriger Transmission ($T_{off}$) der Flüssigkristallzelle (LC) leuchtet.

44. System nach Klausel 40,
**dadurch gekennzeichnet**

dass die Lichtquelle eine Lichtquelle zum Blenden eines Lebewesens, eines optischen Sensors oder einer Kamera ist.

45. System nach einer der vorhergehenden Klauseln,
**dadurch gekennzeichnet,**
dass die Lichtquelle eine Anzeige ist.

46. Verfahren zum Sichtverbessern durch Blendunterdrückung mit folgenden Schritten:

46.1 eine Brille für einen Träger mit mindestens einem Auge wird zur Verfügung gestellt,

46.1.1 wobei die Brille mindestens ein Brillenglas aufweist;

46.1.2 wobei das mindestens eine Brillenglas eine Flüssigkristallzelle (LC) aufweist, deren Transmission (TR) durch eine geeignete Ansteuerung veränderbar ist;

46.1.2.1 wobei die Flüssigkristallzelle (LC) derart ausgewählt wird, dass die Transmission (TR) der Flüssigkristallzelle (LC) zwischen Zuständen hoher und niedriger Transmission umgeschaltet werden kann;

46.1.2.1.1 wobei die Zeiten des Zustands hoher Transmission ($T_{on}$) der Flüssigkristallzelle (LC) geregelt oder gesteuert werden;46und

46.2 eine Lichtquelle (S) wird zur Verfügung gestellt;

46.2.1 wobei Leuchtzeiten und Intensität der Lichtquelle (S) derart gesteuert oder geregelt werden, dass diese während der Zeiten des Zustands hoher Transmission ($T_{on}$) der Flüssigkristallzelle (LC) leuchtet, wobei

46.2.2 das zeitliche Integral des Produkts aus

46.2.2.1 der Intensität der Lichtquelle (S) und

46.2.2.2 der Transmission (TR) der Flüssigkristallzelle (LC)

bei einer Veränderung der Zeit hoher Transmission ($T_{on}$) innerhalb einer vorgegebenen Toleranz konstant gehalten wird;

46.3 wobei die Regelung oder Steuerung der Flüssigkristallzelle (LC) und der Lichtquelle (S) derart ausgebildet wird,

46.3.1 dass die zeitliche Position der Zeiten des Zustands hoher Transmission ($T_{on}$) innerhalb einer Periode aus Zeiten des Zustands hoher Transmission ($T_{on}$) und Zeiten des Zustands niedriger Transmission ($T_{off}$) kontinuierlich oder sprunghaft verändert werden kann; und/oder

46.3.2 dass die Dauer einer Periode aus Zeiten des Zustands hoher Transmission ($T_{on}$) und Zeiten des Zustands niedriger Transmission ($T_{off}$) kontinuierlich oder sprunghaft verändert werden kann;

46.3.3 wobei die Veränderungen durch einen geheimen Codierschlüssel bestimmt werden.

47. System zum Blenden eines Lebewesens, eines optischen Sensors oder einer Kamera, mit:

47.1 einer Brille für einen Träger mit mindestens einem Auge, mit

47.1.1 mindestens einem Brillenglas;

47.1.1.1 wobei das mindestens eine Brillenglas eine Flüssigkristallzelle (LC) aufweist, deren Transmission durch eine geeignete Ansteuerung veränderbar ist;

47.1.1.1.1 wobei die Flüssigkristallzelle (LC) derart ausgebildet ist, dass die Transmission (TR) der Flüssigkristallzelle (LC) zwischen Zuständen hoher und niedriger Transmission umgeschaltet werden kann; und mit

47.1.2 Mitteln zum Steuern der Zeiten des Zustands hoher Transmission ($T_{on}$) der Flüssigkristallzelle (LC);

47.2 einer Lichtquelle zum Blenden eines Lebewesens, eines optischen Sensors oder einer Kamera,

47.2.1 die während der Zeiten des Zustands niedriger Transmission ($T_{off}$) der Flüssigkristallzelle (LC) leuchtet;

47.3 wobei die Regelung oder Steuerung der Flüssigkristallzelle (LC) und der Lichtquelle zum Blenden derart ausgebildet ist,

47.3.1 dass die zeitliche Position der Zeiten des Zustands hoher Transmission ($T_{on}$) innerhalb einer Periode aus Zeiten des Zustands hoher Transmission ($T_{on}$) und Zeiten des Zustands niedriger Transmission ($T_{off}$) kontinuierlich oder sprunghaft verändert werden kann; und/oder

47.3.2 dass die Dauer einer Periode aus Zeiten des Zustands hoher Transmission ($T_{on}$) und Zeiten des Zustands niedriger Transmission ($T_{off}$) kontinuierlich oder sprunghaft verändert werden kann;

47.3.3 wobei die Veränderungen durch einen geheimen Codierschlüssel bestimmt werden.

48. System nach der vorhergehenden Klausel,
**gekennzeichnet durch**

48.1 eine zweite Lichtquelle (S);

48.2 Mitteln zum Steuern oder Regeln der Leuchtzeiten und der Leuchtintensität der zweiten Lichtquelle (S) derart, dass diese während der Zeiten des Zustands hoher Transmission ($T_{on}$) der Flüssigkristallzelle (LC) leuchtet.

49. System nach der vorhergehenden Klausel,
**dadurch gekennzeichnet,**
dass die zweite Lichtquelle eine Anzeige ist.

50. System nach einer der vorhergehenden Klauseln,
**dadurch gekennzeichnet**

50.1 dass die Brille ferner mindestens einen Sensor (IL, IR) zum Messen der Helligkeit des auf den mindestens einen Sensor treffenden sichtbaren Lichts aufweist;
50.1.1 wobei der mindestens eine Sensor augenseitig des Brillenglases angeordnet ist;
50.1.2 wobei der mindestens eine Sensor die Helligkeit durch das mindestens eine Brillenglas misst;
50.2 dass die Brille ferner einen geschlossenen Regelkreis (MC) für die Regelung der Transmission der Flüssigkristallzelle (LC) umfasst;
50.2.1 wobei ein Sollwert für die Helligkeit am Auge des Brillenträgers vorgegeben ist;
50.2.2 wobei der Regelkreis als Istwert die vom Sensor gemessene Helligkeit heranzieht.

51. System nach der vorhergehenden Klausel,
**dadurch gekennzeichnet,**

51.1 dass der Sensor (IL, IR)
51.1.1 ein abbildendes System mit einer Kamera oder
51.1.2 mindestens 3 Sensoren, die ein Koordinatensystem aufspannen, oder
51.1.3 ein Facettenauge
aufweist;
51.2 dass die Brille ferner einen Eye-Tracker (ET) aufweist, der die Blickrichtung des Auges bestimmen kann;
51.3 dass der Sensor die Helligkeit des aus der mit dem Eye-Tracker (ET) bestimmten Blickrichtung des Auges auf ihn treffenden sichtbaren Lichts bestimmen kann; und
51.4 dass der Regelkreis als Istwert die vom Sensor gemessene Helligkeit in Blickrichtung des Auges heranzieht.

52. System nach einer der vorhergehenden Klauseln,
**dadurch gekennzeichnet,**
dass die Flüssigkristallzelle (LC) derart ausgebildet ist, dass sie ihre Transmission von 90% auf 10% und von 10% auf 90% in maximal 10 ms ändern kann.

53. System nach einer der vorhergehenden Klauseln,
**dadurch gekennzeichnet,**
dass das Brillengestell das mindestens eine Auge des Brillenträgers lichtdicht gegenüber dem Umgebungslicht abdichtet.

54. System nach einer der vorhergehenden Klauseln,
**dadurch gekennzeichnet,**
dass der Sollwert des Regelkreises eine Helligkeit am Auge von 20 bis 400 lx vorgibt.

55. System nach einer der vorhergehenden Klauseln,
**dadurch gekennzeichnet,**
dass die Helligkeit des Umgebungslichts aus dem Sollwert und einem Stellsignal des Regelkreises erschlossen wird.

56. System nach einer der vorhergehenden Klauseln,
**gekennzeichnet durch**
mindestens einen weiteren Helligkeits-Sensor (OL, OR), der auf der vom Auge abgewandten Seite der Brille angeordnet ist (Außensensor) und die Helligkeit des Umgebungslichts bestimmt.

57. System nach einer der beiden vorhergehenden Klauseln,

57.1 wobei der Sollwert des Regelkreises abhängig von der Helligkeit des Umgebungslichts verändert werden kann; und

57.2 wobei die Veränderung des Sollwerts um mindestens einen Faktor 10 langsamer ist als die Regelung der Transmission der Flüssigkristallzelle.

58. System nach der vorhergehenden Klausel,
**dadurch gekennzeichnet,**

58.1 dass die Veränderung des Sollwerts in vorgegebenen Stufen erfolgt;
58.2 wobei die stufenweise Veränderung des Sollwerts um mindestens einen Faktor 100 langsamer ist als die Regelung der Transmission der Flüssigkristallzelle und
58.3 in ihrem Verlauf eine Hysterese aufweist.

59. System nach der vorhergehenden Klausel,
**dadurch gekennzeichnet,**
dass die Regelung derart ausgebildet ist, dass sie auf extreme Helligkeitswerte innerhalb von 10 μs bis einer Sekunde derart reagiert, dass die Flüssigkristallzelle (LC) auf den Zustand niedriger Transmission eingestellt wird.

60. System nach einer der vorhergehenden Klauseln,
**gekennzeichnet durch**

60.1 zwei Brillengläser für zwei Augen eines Brillenträgers;
60.2 zwei augenseitige Sensoren zum Messen der Helligkeit des auf das jeweilige Auge treffenden sichtbaren Lichts; und durch
60.3 je einen Regelkreis für jedes Auge.

61. System nach der vorhergehenden Klausel,
**dadurch gekennzeichnet,**
dass die Sollwerte für die beiden Augen 1% bis 60% voneinander abweichen.

62. System nach Klausel 60,
**dadurch gekennzeichnet,**
dass bei der Regelung der Helligkeit des auf ein Auge treffenden sichtbaren Lichts die Regelung der Helligkeit für das andere Auge berücksichtigt wird.

63. System nach Klausel 47,
**gekennzeichnet durch**

63.1 Lichtquellen, die an der vom Auge abgewandten Seite der Brille angeordnet sind;
63.2 wobei die Lichtquellen abhängig von der Blickrichtung des Brillenträgers gesteuert werden.

64. System nach einer der vorhergehenden Klauseln,
**dadurch gekennzeichnet,**
dass die Messwerte der Sensoren und/oder Sollwerte der Regelkreise und/oder die davon abgeleitete Helligkeit der Umgebung mit einem Geokoordinaten-Signal eines Geokoordinaten-Empfängers verbunden und aufgezeichnet werden.

65. System nach einer der vorhergehenden Klauseln,
**dadurch gekennzeichnet,**

65.1 dass das mindestens eine Brillenglas eine weitere Flüssigkristallzelle aufweist, deren Transmission durch eine geeignete Ansteuerung veränderbar ist,
65.2 wobei die weitere Flüssigkristallzelle in Blickrichtung hinter oder vor der Flüssigkristallzelle angeordnet ist.

66. System nach Klausel 47 bis 65 in Kombination mit dem System nach einer der Klauseln 73 bis 84.

67. Verfahren zum Blenden eines Lebewesens, eines optischen Sensors oder einer Kamera, mit folgenden Schritten:

67.1 eine Brille für einen Träger mit mindestens einem Auge zur Verfügung gestellt;

67.1.1 wobei die Brille mindestens ein Brillenglas aufweist;

67.1.1.1 wobei das mindestens eine Brillenglas eine Flüssigkristallzelle (LC) aufweist, deren Transmission durch eine geeignete Ansteuerung veränderbar ist;

67.1.1.1.1 wobei die Flüssigkristallzelle (LC) derart ausgebildet ist, dass die Transmission (TR) der Flüssigkristallzelle (LC) zwischen Zuständen hoher und niedriger Transmission umgeschaltet werden kann;

67.1.2 wobei die Brille ferner Mittel zum Steuern der Zeiten des Zustands hoher Transmission ($T_{on}$) der Flüssigkristallzelle (LC) aufweist;

67.2 eine Lichtquelle zum Blenden eines Lebewesens, eines optischen Sensors oder einer Kamera wird zur Verfügung gestellt,

67.2.1 die während der Zeiten des Zustands niedriger Transmission ($T_{off}$) der Flüssigkristallzelle (LC) leuchtet;

67.3 wobei die Regelung oder Steuerung der Flüssigkristallzelle (LC) und der Lichtquelle derart ausgebildet wird,

67.3.1 dass die zeitliche Position der Zeiten des Zustands hoher Transmission ($T_{on}$) innerhalb einer Periode aus Zeiten des Zustands hoher Transmission ($T_{on}$) und Zeiten des Zustands niedriger Transmission ($T_{off}$) kontinuierlich oder sprunghaft verändert werden kann; und/oder

67.3.2 dass die Dauer einer Periode aus Zeiten des Zustands hoher Transmission ($T_{on}$) und Zeiten des Zustands niedriger Transmission ($T_{off}$) kontinuierlich oder sprunghaft verändert werden kann;

67.3.3 wobei die Veränderungen durch einen geheimen Codierschlüssel bestimmt werden.

68. System zum Sichtverbessern durch Blendunterdrückung mit:

68.1 einer Brille für einen Träger mit mindestens einem Auge, mit

68.1.1 mindestens einem Brillenglas;

68.1.1.1 wobei das mindestens eine Brillenglas eine Flüssigkristallzelle (LC) aufweist, deren Transmission durch eine geeignete Ansteuerung veränderbar ist;

68.1.1.1.1 wobei die Flüssigkristallzelle (LC) derart ausgebildet ist, dass die Transmission (TR) der Flüssigkristallzelle (LC) zwischen Zuständen hoher und niedriger Transmission umgeschaltet werden kann; und mit

68.1.2 mindestens einem Sensor (IL, IR) zum Messen der Helligkeit des auf den mindestens einen Sensor treffenden sichtbaren Lichts; und mit

68.1.3 einem geschlossenen Regelkreis (MC) für die Regelung der Transmission der Flüssigkristallzelle (LC);

68.1.3.1 wobei ein Sollwert für die Helligkeit am Auge des Brillenträgers vorgegeben ist;

68.1.3.2 wobei der Regelkreis als Istwert die von dem mindestens einen Sensor (IL, IR) gemessene Helligkeit heranzieht;

68.1.3.3 wobei die Regelung oder Steuerung derart ausgebildet ist, dass die Zeiten des Zustands hoher Transmission ($T_{on}$) mit zunehmender Blendung kürzer werden; und mit

68.2 einer Lichtquelle (S) mit

68.2.1 Mitteln zum Steuern oder Regeln der Leuchtzeiten und der Leuchtintensität der Lichtquelle (S) derart, dass diese während der Zeiten des Zustands hoher Transmission ($T_{on}$) der Flüssigkristallzelle (LC) leuchtet;

68.2.2 wobei das zeitliche Integral des Produkts aus

68.2.2.1 der Leuchtintensität der Lichtquelle und

68.2.2.2 der Transmission (TR) der Flüssigkristallzelle (LC)

bei einer Veränderung der Zeiten des Zustands hoher Transmission ($T_{on}$) innerhalb einer vorgegebenen Toleranz konstant bleibt;

68.3 wobei der mindestens eine Sensor (IL, IR) die Helligkeit des auf ihn treffenden sichtbaren Lichts ausschließlich in den Zeiten des Zustands niedriger Transmission ($T_{off}$) misst.

69. System nach der vorhergehenden Klausel,
**dadurch gekennzeichnet,**

69.1 dass der Sensor die Helligkeit des auf ihn treffenden sichtbaren Lichts zusätzlich in den Zeiten des Zustands hoher Transmission ($T_{on}$) misst;

69.2 wobei diese Messungen getrennt von den Messungen in den Zeiten des Zustands niedriger Transmission ($T_{off}$) erfolgen.

70. System nach der vorhergehenden Klausel,
**gekennzeichnet durch**
eine Steuerung der Lichtquelle (S) derart, dass für den Fall, dass die gemessene Helligkeit in den Zeiten des Zustands hoher Transmission ($T_{on}$) um mehr als eine vorgegebene Schwelle über der Helligkeit in den Zeiten des Zustands niedriger Transmission ($T_{off}$) liegt, die Lichtquelle (S) ausgeschaltet oder ihre Leuchtintensität reduziert wird.

71. System nach Klausel 68 bis 70 in Kombination mit dem System nach einer der Klauseln 1 bis 21.

72. Verfahren zum Sichtverbessern durch Blendunterdrückung mit folgenden Schritten:

72.1 eine Brille für einen Träger mit mindestens einem Auge wird zur Verfügung gestellt;
72.1.1 wobei die Brille derart ausgebildet wird, dass sie mindestens ein Brillenglas aufweist;
72.1.1.1 wobei das mindestens eine Brillenglas eine Flüssigkristallzelle (LC) aufweist, deren Transmission (TR) durch eine geeignete Ansteuerung veränderbar ist;
72.1.1.1.1 wobei die Flüssigkristallzelle (LC) derart ausgebildet ist, dass die Transmission (TR) der Flüssigkristallzelle (LC) zwischen Zuständen hoher und niedriger Transmission umgeschaltet werden kann;
72.1.2 mindestens ein Sensor (IL, IR) zum Messen der Helligkeit des auf den Sensor treffenden sichtbaren Lichts wird zur Verfügung gestellt;
72.1.2.1 wobei der mindestens eine Sensor (IL, IR) augenseitig des Brillenglases angeordnet wird;
72.1.2.2 wobei der mindestens eine Sensor (IL, IR) die Helligkeit durch das mindestens eine Brillenglas misst;
72.1.3 die Transmission der Flüssigkristallzelle (LC) wird geregelt;
72.1.3.1 wobei ein Sollwert für die Helligkeit am Auge des Brillenträgers vorgegeben ist;
72.1.3.2 wobei der Regelkreis als Istwert die von dem mindestens einen Sensor (IL, IR) gemessene Helligkeit heranzieht;
72.1.3.3 wobei die Regelung derart ausgebildet ist, dass die Zeiten des Zustands hoher Transmission ($T_{on}$) mit zunehmender Blendung kürzer werden;
72.2 ferner wird eine Lichtquelle (S) zur Verfügung gestellt;
72.2.1 die Leuchtzeiten und die Leuchtintensität der Lichtquelle (S) wird derart gesteuert, dass diese während der Zeiten des Zustands hoher Transmission ($T_{on}$) der Flüssigkristallzelle (LC) leuchtet;
72.2.2 wobei das zeitliche Integral des Produkts aus
72.2.2.1 der Leuchtintensität der Lichtquelle (S) und
72.2.2.2 der Transmission (TR) der Flüssigkristallzelle (LC)
bei einer Veränderung der Zeiten des Zustands hoher Transmission ($T_{on}$) innerhalb einer vorgegebenen Toleranz konstant bleibt;
72.3 wobei der mindestens einer Sensor (IL, IR) die Helligkeit des auf ihn treffenden sichtbaren Lichts ausschließlich in den Zeiten des Zustands niedriger Transmission ($T_{off}$) misst.

73. System zum farblichen Kennzeichnen von Objekten im Sichtfeld von einer Mehrzahl von Brillenträgern mit:

73.1 je einer Brille pro Brillenträger, mit
73.1.1 jeweils mindestens einem Brillenglas;
73.1.2 wobei das jeweilige mindestens eine Brillenglas eine Flüssigkristallzelle (LC) aufweist, deren Transmission durch eine geeignete Ansteuerung veränderbar ist;
73.1.2.1 wobei die Flüssigkristallzellen (LC) derart ausgebildet sind, dass die Transmission (TR) der Flüssigkristallzellen (LC) zwischen Zuständen hoher und niedriger Transmission umgeschaltet werden kann; und mit
73.1.3 Mitteln zum Regeln oder Steuern der Zeiten der Zustände hoher Transmission ($T_{on}$) der Flüssigkristallzellen (LC) derart, dass die jeweiligen Flüssigkristallzellen (LC) zu unterschiedlichen Zeiten auf Zustände hoher Transmission ($T_{on}$) eingestellt werden; und mit
73.2 je einer RGB-Lichtquelle (S1, S2, S3) pro Brillenträger;
73.2.1 Mitteln zum Steuern oder Regeln der Leuchtzeiten, der Farbe und der Intensität der RGB-Lichtquelle (S1, S2, S3) derart, dass
73.2.1.1 die RGB-Lichtquelle (S1) für einen ersten Brillenträger zu einer Zeit des Zustands hoher Transmission ($T_{on}$) der Flüssigkristallzelle (LC) seiner Brille mit einer ersten Farbe leuchtet; und dass
73.2.2.2 die RGB-Lichtquelle (S2) für einen zweiten Brillenträger zu einer Zeit des Zustands hoher Transmission ($T_{on}$) der Flüssigkristallzelle (LC) der Brille des zweiten Brillenträgers mit einer zweiten, von der ersten verschiedenen Farbe leuchtet.

74. System nach der vorhergehenden Klausel,
**dadurch gekennzeichnet,**
dass in den Zeiten des Zustands niedriger Transmission ($T_{off}$) der jeweiligen Brillen von den jeweils zugehörigen RGB-Lichtquellen (S1, S2, S3) diejenigen Farben ausgestrahlt werden, die nötig sind, um in einem zeitlichen Mittel einen weißen Farbeindruck bei denjenigen zu hinterlassen, die keine der Brillen tragen.

75. System nach einer der beiden vorhergehenden Klauseln,
**dadurch gekennzeichnet,**
dass die Flüssigkristallzelle (LC) eines ersten Brillenträgers in einer Zeit des Zustands hoher Transmission ($T_{on}$) eines zweiten Brillenträgers eine abgeschwächte, aber von Null verschiedene Transmission aufweist.

76. System nach einer der Klauseln 73 bis 75,
**dadurch gekennzeichnet,**

76.1 dass die Farbe, in der die RGB-Lichtquelle für den ersten Brillenträger zu einer Zeit des Zustands hoher Transmission ($T_{on}$) der Flüssigkristallzelle (LC) seiner Brille leuchtet, dadurch frei definiert werden kann, dass ein beliebiger Intensitätswert zwischen 0% und 100% eines Farbanteils jeder Primärfarbe seiner RGB-Lichtquelle (S1) ausgestrahlt wird;
76.2 während zu der zugehörigen Zeit des Zustands niedriger Transmission ($T_{off}$) der Flüssigkristallzelle (LC) für jede der drei Primärfarben seiner RGB-Lichtquelle (S1) der zu 100% fehlende Anteil ausgestrahlt wird.

77. System nach einer der Klauseln 73 bis 76 in Kombination mit dem System nach einer der Klauseln 40 bis 46.

78. System nach einer der Klauseln 73 bis 76,
**dadurch gekennzeichnet,**
dass die Brillen

78.1 jeweils mindestens einen Sensor (IL, IR) zum Messen der Helligkeit des auf sie treffenden sichtbaren Lichts aufweisen;
78.1.1 wobei der jeweilige mindestens eine Sensor (IL, IR) augenseitig des jeweiligen Brillenglases angeordnet ist;
78.1.2 wobei der jeweilige mindestens eine Sensor (IL, IR) die Helligkeit durch das mindestens eine Brillenglas misst;
78.2 und mit je einem geschlossenen Regelkreis (MC) für die Regelung der Transmission der jeweiligen Flüssigkristallzelle (LC);
78.2.1 wobei jeweils ein Sollwert für die Helligkeit am Auge des jeweilige Brillenträgers vorgegeben ist;
78.2.2 wobei der Regelkreis (MC) als Istwert die von dem mindestens einen Sensor (IL, IR) gemessene Helligkeit heranzieht.

79. System nach der vorhergehenden Klausel,

**gekennzeichnet durch**
eine zusätzliche LED, die den Sensor ansprechen kann, um aus Sicherheitsgründen die korrekte Funktionsfähigkeit der Flüssigkristallzelle der jeweiligen Brille zu prüfen.

80. System nach einer der Klauseln 73 bis 79 in Kombination mit dem System nach einer der Klauseln 1 bis 21.

81. System nach einer der Klauseln 73 bis 79 in Kombination mit dem System nach einer der Klauseln 22 bis 39.

82. System nach einer der Klauseln 73 bis 79 in Kombination mit dem System nach einer der Klauseln 40 bis 46.

83. System nach einer der Klauseln 73 bis 79 in Kombination mit dem System nach einer der Klauseln 47 bis 67.

84. Verfahren zum farblichen Kennzeichnen von Objekten im Sichtfeld von einer Mehrzahl von Brillenträgern mit folgenden Schritten:

84.1 jeder Brillenträger trägt eine Brille, mit
84.1.1 jeweils mindestens einem Brillenglas;
84.1.2 wobei das jeweilige mindestens eine Brillenglas eine Flüssigkristallzelle (LC) aufweist, deren Transmission durch eine geeignete Ansteuerung veränderbar ist;
84.1.2.1 wobei die Flüssigkristallzellen (LC) derart ausgebildet sind, dass die Transmission (TR) der Flüssigkristallzellen (LC) zwischen Zuständen hoher und niedriger Transmission umgeschaltet werden kann; und mit
84.1.3 wobei die Zeiten der Zustände hoher Transmission ($T_{on}$) der jeweiligen Flüssigkristallzellen (LC) zu unterschiedlichen Zeiten auf Zustände hoher Transmission ($T_{on}$) eingestellt werden;

42

84.2 für jeden Brillenträger wird eine RGB-Lichtquelle (S1, S2, S3) zur Verfügung gestellt;

84.2.1 die Leuchtzeiten, die Farbe und die Intensität der RGB-Lichtquellen (S1, S2, S3) werden derart gesteuert, dass

84.2.1.1 die RGB-Lichtquelle (S1) für einen ersten Brillenträger zu einer Zeit des Zustands hoher Transmission ($T_{on}$) der Flüssigkristallzelle (LC) seiner Brille mit einer ersten Farbe leuchtet; und dass

84.2.2.2 die RGB-Lichtquelle (S2) für einen zweiten Brillenträger zu einer Zeit des Zustands hoher Transmission ($T_{on}$) der Flüssigkristallzelle (LC) der Brille des zweiten Brillenträgers mit einer zweiten, von der ersten verschiedenen Farbe leuchtet.

85. System zum Verstärken des räumlichen Eindrucks eines Objekts mit:

85.1 einer Brille für einen Träger mit mindestens zwei Augen, einem rechten (E(R)) und einem linken (E(L)) Auge, mit

85.1.1 je einem Brillenglas vor jedem der beiden Augen;

85.1.2 wobei jedes Brillenglas eine Flüssigkristallzelle (LC(L), LC(R)) aufweist, deren Transmission durch eine geeignete Ansteuerung veränderbar ist;

85.1.2.1 wobei die Flüssigkristallzellen (LC(L), LC(R)) derart ausgebildet sind, dass die Transmission (TR) der Flüssigkristallzellen jeweils zwischen Zuständen hoher und niedriger Transmission umgeschaltet werden können; und mit

85.1.2.1.1 Mitteln zum Regeln oder Steuern der Zeiten der Zustände hoher Transmission ($T_{on}$) der Flüssigkristallzellen (LC(L), LC(R)); und mit

85.2 zwei Lichtquellen (S1(L), S1(R)), die jeweils einem Auge zugeordnet sind;

85.2.1 wobei die beiden Lichtquellen unterschiedliche Farben ausstrahlen; und

85.2.2 wobei die stereoskopische Basis (DS1) der Lichtquellen größer als der Augenabstand (PD) ist; und mit

85.2.3 Mitteln zum Steuern oder Regeln der Leuchtzeiten der Lichtquellen (S1(L), S1(R)) derart,

85.2.3.1 dass die dem rechten Auge (E(R)) zugeordnete Lichtquelle (S1(R)) während einer Zeit des Zustands hoher Transmission ($T_{on}$) der Flüssigkristallzelle (LC(R)) des rechten Auges leuchtet,

85.2.3.1.1 während die dem linken Auge (E(L)) zugeordnete Lichtquelle (S1(L)) nicht leuchtet und

85.2.3.1.2 die Flüssigkristallzelle (LC(L)) des linken Auges auf niedrige Transmission eingestellt ist;

85.2.3.2 und umgekehrt.

86. System nach der vorhergehenden Klausel,
**dadurch gekennzeichnet,**
dass die von der jeweiligen Lichtquellen zu den Zeiten der Zustände hoher Transmission ($T_{on}$) ausgestrahlte Farbe während der zugehörigen Zeiten der Zustände niedriger Transmission ($T_{off}$) zu einem weißen Farbeindruck ergänzt wird.

87. System nach einer der beiden vorhergehenden Klauseln,
**dadurch gekennzeichnet,**
dass die beiden Lichtquellen mit einer vorgegebenen, vom menschlichen Auge wahrnehmbaren Frequenz amplitudenmoduliert werden.

88. System nach einer der Klauseln 85 bis 87 in Kombination mit dem System nach einer der Klauseln 1 bis 6.

89. System nach einer der Klauseln 85 bis 87,
**dadurch gekennzeichnet,**
dass die Brille

89.1 mindestens einen Sensor (IL, IR) zum Messen der Helligkeit des auf ihn treffenden sichtbaren Lichts aufweist;

89.1.1 wobei der mindestens eine Sensor (IL, IR) augenseitig des des mindestens einen Brillenglases angeordnet ist;

89.1.2 wobei der mindestens eine Sensor (IL, IR) die Helligkeit durch das mindestens eine Brillenglas misst; und

89.2 mit mindestens einem geschlossenen Regelkreis (MC) für die Regelung der Transmission der jeweiligen Flüssigkristallzelle (LC);

89.2.1 wobei mindestens ein Sollwert für die Helligkeit am Auge des Brillenträgers vorgegebenen ist;

89.2.2 wobei der Regelkreis (MC) als Istwert die von dem mindestens einen Sensor gemessene Helligkeit heranzieht.

90. System nach einer der Klauseln 85 bis 89 in Kombination mit dem System nach einer der Klauseln 73 bis 84.

91. System nach einer der Klauseln 85 bis 89 in Kombination mit dem System nach einer der Klauseln 22 bis 39.

92. System nach einer der Klauseln 85 bis 89 in Kombination mit dem System nach einer der Klauseln 1 bis 21.

93. System nach einer der Klauseln 85 bis 89 in Kombination mit dem System nach einer der Klauseln 95 bis 100.

94. Verfahren zum Verstärken des räumlichen Eindrucks eines Objekts mit folgenden Schritten:

94.1 für einen Träger mit mindestens zwei Augen, einem rechten (E(R)) und einem linken (E(L)) Auge, wird eine Brille zur Verfügung gestellt, wobei die Brille
94.1.1 je ein Brillenglas vor jedem der beiden Augen aufweist;
94.1.2 wobei jedes Brillenglas eine Flüssigkristallzelle (LC(L), LC(R)) aufweist, deren Transmission durch eine geeignete Ansteuerung veränderbar ist;
94.1.2.1 wobei die Flüssigkristallzellen (LC(L), LC(R)) derart ausgebildet sind, dass die Transmission (TR) der Flüssigkristallzellen jeweils zwischen Zuständen hoher und niedriger Transmission umgeschaltet werden können;
94.1.2.1.1 die Zeiten der Zustände hoher Transmission ($T_{on}$) der Flüssigkristallzellen (LC(L), LC(R)) werden gesteuert;
94.2 ferner werden zwei Lichtquellen (S1(L), S1(R)) zur Verfügung gestellt, die jeweils einem Auge zugeordnet sind;
94.2.1 wobei die beiden Lichtquellen unterschiedliche Farben ausstrahlen; und
94.2.2 wobei die stereoskopische Basis (DS1) der Lichtquellen größer als der Augenabstand (PD) ist;
94.2.3 die Leuchtzeiten der Lichtquellen (S1(L), S1(R)) werden derart gesteuert,
94.2.3.1 dass die dem rechten Auge (E(R)) zugeordnete Lichtquelle (S1(R)) während einer Zeit des Zustands hoher Transmission ($T_{on}$) der Flüssigkristallzelle (LC(R)) des rechten Auges leuchtet,
94.2.3.1.1 während die dem linken Auge (E(L)) zugeordnete Lichtquelle (S1(L)) nicht leuchtet und
94.2.3.1.2 die Flüssigkristallzelle (LC(L)) des linken Auges auf niedrige Transmission eingestellt ist;
94.2.3.2 und umgekehrt.

95. System zum Verbessern der Sicht auf einen räumlich zu beobachtenden Bereich durch Blendunterdrückung mit:

95.1 einer Brille, mit
95.1.1 mindestens einem Brillenglas;
95.1.2 wobei das mindestens eine Brillenglas eine Flüssigkristallzelle (LC) aufweist, deren Transmission (TR) durch eine geeignete Ansteuerung veränderbar ist;
95.1.2.1 wobei die Flüssigkristallzelle (LC) derart ausgebildet ist, dass die Transmission (TR) der Flüssigkristallzelle (LC) zwischen Zuständen hoher und niedriger Transmission umgeschaltet werden kann; und mit
95.1.2.1.1 Mitteln zum Regeln oder Steuern der Zeiten des Zustands hoher Transmission ($T_{on}$) der Flüssigkristallzelle (LC);
95.2 einer gepulsten Lichtquelle (S), die Lichtpulse aussendet;
95.2.1 wobei die Lichtquelle (S) derart ausgebildet ist, dass sie Lichtpulse erzeugen kann, deren zeitliche Dauer kürzer ist als das Licht der Lichtquelle braucht, um den räumlich zu beobachtenden Bereich in Blickrichtung des Trägers zu durchqueren; und mit
95.3 Mitteln zum Steuern oder Regeln der Zeiten des Zustands hoher Transmission ($T_{on}$) der Flüssigkristallzelle (LC), die in der Lage sind, die Zeiten des Zustands hoher Transmission ($T_{on}$) der Flüssigkristallzelle zeitlich so zu legen, dass nur das Rückstreusignal des Lichtpulses aus dem räumlich zu beobachtenden Bereich von der Flüssigkristallzelle (LC) transmittiert wird.

96. System nach Klausel 95 in Kombination mit dem System nach einer der Klauseln 40 bis 46.

97. System nach Klausel 95 in Kombination mit dem System nach einer der Klauseln 22 bis 39.

98. System nach Klausel 95 in Kombination mit dem System nach einer der Klauseln 85 bis 94.

99. System nach Klausel 95,
**dadurch gekennzeichnet,**

dass die Brille

99.1 mindestens einen Sensor (IL, IR) zum Messen der Helligkeit des auf ihn treffenden sichtbaren Lichts aufweist;

99.1.1 wobei der mindestens eine Sensor (IL, IR) augenseitig des jeweiligen Brillenglases angeordnet ist;
99.1.2 wobei der mindestens einer Sensor (IL, IR) die Helligkeit durch das mindestens eine Brillenglas misst; und
99.2 mit mindestens einem geschlossenen Regelkreis (MC) für die Regelung der Transmission der jeweiligen Flüssigkristallzelle (LC);
99.2.1 wobei ein Sollwert für die Helligkeit am Auge des Brillenträgers vorgegebenen ist;
99.2.2 wobei der Regelkreis (MC) als Istwert die von dem mindestens einen Sensor (IL, IR) gemessene Helligkeit heranzieht.

100. Verfahren zum Verbessern der Sicht auf einen räumlich zu beobachtenden Bereich durch Blendunterdrückung mit folgenden Schritten:

100.1 eine Brille wird zur Verfügung gestellt, wobei die Brille
100.1.1 mindestens ein Brillenglas aufweist;
100.1.2 wobei das mindestens eine Brillenglas eine Flüssigkristallzelle (LC) aufweist, deren Transmission (TR) durch eine geeignete Ansteuerung veränderbar ist;
100.1.2.1 wobei die Flüssigkristallzelle (LC) derart ausgebildet wird, dass die Transmission (TR) der Flüssigkristallzelle (LC) zwischen Zuständen hoher und niedriger Transmission umgeschaltet werden kann;
100.1.2.1.1 die Zeiten des Zustands hoher Transmission ($T_{on}$) der Flüssigkristallzelle (LC) werden gesteuert;
100.2 eine gepulste Lichtquelle (S), die Lichtpulse aussendet, wird zur Verfügung gestellt;
100.2.1 wobei die Lichtquelle (S) derart ausgebildet wird, dass sie Lichtpulse erzeugen kann, deren zeitliche Dauer kürzer ist als das Licht der Lichtquelle braucht, um den räumlich zu beobachtenden Bereich in Blickrichtung des Trägers zu durchqueren;
100.3 ferner werden die Zeiten des Zustands hoher Transmission ($T_{on}$) der Flüssigkristallzelle zeitlich so gelegt, dass nur das Rückstreusignal des Lichtpulses aus dem räumlich zu beobachtenden Bereich von der Flüssigkristallzelle (LC) transmittiert wird.

zitierte Literatur

zitierte Patentliteratur

DE 10 2012 217 326 A1
DE 101 34 770 A1
DE 2 001 086 A,
EP 0 813 079 A2
US 2,066,680 A
US 5,172,256 A
WO 2013/143 998 A2

zitierte Nicht-Patentliteratur

Adrian, W. and Bhanji, A.: "Fundamentals of disability glare. A formula to describe stray light in the eye as a function of the glare angle and age." Proceedings of the First International Symposium on Glare, 1991, Orlando, Florida, pp. 185-194.

Douglas Mace, Philip Garvey, Richard J. Porter, Richard Schwab, Werner Adrian: "Countermeasures for Reducing the Effects of Headlight Glare"; Prepared for: The AAA Foundation for Traffic Safety, Washington, D.C., December 2001

Prof. Dr.-Ing. Gert Hauske: "Systemtheorie der visuellen Wahrnehmung", Teubner Verlag, Stuttgart, 1994

**Patentansprüche**

1. System zum Sichtverbessern durch Blendunterdrückung mit:

    1.1 einer Brille für einen Träger mit mindestens einem Auge, mit
    1.1.1 mindestens einem Brillenglas;
    1.1.1.1 wobei das mindestens eine Brillenglas eine Flüssigkristallzelle (LC) aufweist, deren Transmission durch eine geeignete Ansteuerung veränderbar ist;
    1.1.1.1.1 wobei die Flüssigkristallzelle (LC) derart ausgebildet ist, dass die Transmission (TR) der Flüssigkristallzelle (LC) zwischen Zuständen hoher und niedriger Transmission umgeschaltet werden kann; und mit
    1.1.2 Mitteln zum Regeln oder Steuern der Zeiten des Zustands hoher Transmission ($T_{on}$) der Flüssigkristallzelle (LC);
    1.1.3 mindestens einem Sensor (IL, IR) zum Messen der Helligkeit des auf ihn treffenden sichtbaren Lichts;
    1.1.3.1 wobei der mindestens eine Sensor (IL, IR) augenseitig des Brillenglases angeordnet ist;
    1.1.3.2 wobei der mindestens eine Sensor (IL, IR) die Helligkeit durch das mindestens eine Brillenglas misst; und mit
    1.1.4 einem geschlossenen Regelkreis (MC) für die Regelung der Transmission der Flüssigkristallzelle (LC);
    1.1.4.1 wobei die Regelung derart ausgebildet ist, dass die Zeiten des Zustands hoher Transmission ($T_{on}$) mit zunehmender Blendung kürzer werden;
    1.1.4.22 wobei ein Sollwert für die Helligkeit am Auge des Brillenträgers vorgegeben ist;
    1.1.4.3 wobei der Regelkreis als Istwert die vom Sensor gemessene Helligkeit heranzieht; und mit
    1.2 einer Anzeige mit
    1.2.1 Mitteln zum Steuern oder Regeln der Leuchtzeiten und der Leuchtintensität der Anzeige derart, dass diese während der Zeiten des Zustands hoher Transmission ($T_{on}$) der Flüssigkristallzelle (LC) leuchtet;
    1.2.2 wobei das zeitliche Integral des Produkts aus
    1.2.2.1 der Leuchtintensität der Anzeige und
    1.2.2.2 der Transmission (TR) der Flüssigkristallzelle (LC)
    bei einer Veränderung der Zeiten des Zustands hoher Transmission ($T_{on}$) innerhalb einer vorgegebenen Toleranz konstant bleibt.

2. System nach dem vorhergehenden Anspruch,
   **dadurch gekennzeichnet,**
   **dass** die Anzeige

    - eine Anzeige an der vom Auge abgewandten Seite des Brillenglases und/oder
    - eine Anzeige augenseitig des Brillenglases und/oder
    - ein Head-Up-Display (HUD)

   umfasst.

3. System nach einem der Ansprüche 1 bis 2,
   **dadurch gekennzeichnet,**
   **dass** die Flüssigkristallzelle (LC) derart ausgebildet ist, dass sie ihre Transmission von 90% auf 10% und von 10% auf 90% in maximal 10 ms ändern kann.

4. System nach einem der Ansprüche 1 bis 3,
   **dadurch gekennzeichnet,**
   **dass** der Sollwert des Regelkreises eine Helligkeit am Auge von 20 bis 400 Ix vorgibt.

5. System nach einem der Ansprüche 1 bis 4,
   **dadurch gekennzeichnet,**
   **dass** die Helligkeit des Umgebungslichts aus dem Sollwert und einem Stellsignal des Regelkreises erschlossen wird.

6. System nach einem der Ansprüche 1 bis 5,
   **gekennzeichnet durch**
   mindestens einen weiteren Helligkeits-Sensor (OL, OR), der auf der vom Auge abgewandten Seite der Brille angeordnet ist (Außensensor) und die Helligkeit des Umgebungslichts bestimmt.

**7.** System nach einem der Ansprüche 1 bis 6,
**dadurch gekennzeichnet,**

7.1 dass der Sollwert des Regelkreises abhängig von der Helligkeit des Umgebungslichts verändert werden kann; und
7.2 dass die Veränderung des Sollwerts um mindestens einen Faktor 10 langsamer ist als die Regelung der Transmission der Flüssigkristallzelle.

**8.** System nach dem vorhergehenden Anspruch,
**dadurch gekennzeichnet,**

8.1 dass die Veränderung des Sollwerts in vorgegebenen Stufen erfolgt;
8.2 wobei die stufenweise Veränderung des Sollwerts um mindestens einen Faktor 100 langsamer ist als die Regelung der Transmission der Flüssigkristallzelle.

**9.** System nach einem der Ansprüche 1 bis 8,
**dadurch gekennzeichnet,**
**dass** die Regelung derart ausgebildet ist, dass sie auf extreme Helligkeitswerte innerhalb von 10 µs bis einer Sekunde derart reagiert, dass die Flüssigkristallzelle (LC) auf den Zustand niedriger Transmission eingestellt wird.

**10.** System nach einem der Ansprüche 1 bis 9,
**dadurch gekennzeichnet,**

**dass** die Brille
10.1 zwei Brillengläser für zwei Augen eines Brillenträgers;
10.2 je einen augenseitigen Sensor für jedes Brillenglas zum Messen der Helligkeit des auf das jeweilige Auge treffenden sichtbaren Lichts; und durch
10.3 je einen Regelkreis für jedes Brillenglas
aufweist.

**11.** System nach dem vorhergehenden Anspruch,
**dadurch gekennzeichnet,**
**dass** die Sollwerte für die beiden Augen um 1% bis 60% voneinander abweichen.

**12.** System nach Anspruch 9,
**dadurch gekennzeichnet,**
**dass** bei der Regelung der Helligkeit des auf ein Auge treffenden sichtbaren Lichts die Regelung der Helligkeit für das andere Auge berücksichtigt wird.

**13.** System nach einem der Ansprüche 1 bis 12,
**dadurch gekennzeichnet,**

13.1 dass ein Eye-Tracker (ET) vorhanden ist, der die Blickrichtung des Auges bestimmen kann;
13.2 wobei der mindestens eine Sensor (IL, IR)
13.2.1 ein abbildendes System mit einer Kamera oder
13.2.2 mindestens drei Sensoren, die ein Koordinatensystem aufspannen oder
13.2.3 ein Facettenauge
aufweist;
13.3 wobei der mindestens eine Sensor (IL, IR) die Helligkeit des aus der mit dem Eye-Tracker (ET) bestimmten Blickrichtung des Auges auf ihn treffenden sichtbaren Lichts bestimmen kann; und
13.4 wobei der Regelkreis (MC) als Istwert die vom Sensor gemessene Helligkeit in Blickrichtung des Auges heranzieht.

**14.** System nach einem der Ansprüche 1 bis 13,
**dadurch gekennzeichnet,**
**dass** der Regelkreis derart ausgebildet ist, dass er bei der Bestimmung der Helligkeit aus der Blickrichtung des Auges eine nutzerspezifische Augen/Netzhaut-Empfindlichkeitskurve zur Gewichtung der Helligkeit berücksichtigen kann.

**15.** Verfahren zum Sichtverbessern durch Blendunterdrückung mit folgenden Schritten:

15.1 eine Brille für einen Träger mit mindestens einem Auge wird zur Verfügung gestellt, wobei die Brille derart ausgebildet wird, dass sie folgendes aufweist:

15.1.1 mindestens ein Brillenglas;

15.1.1.1 wobei das mindestens eine Brillenglas eine Flüssigkristallzelle (LC) aufweist, deren Transmission durch eine geeignete Ansteuerung veränderbar ist;

15.1.1.1.1 wobei die Flüssigkristallzelle (LC) derart ausgebildet ist, dass die Transmission (TR) der Flüssigkristallzelle (LC) zwischen Zuständen hoher und niedriger Transmission umgeschaltet werden kann;

15.1.2 Mittel zum Regeln oder Steuern der Zeiten des Zustands hoher Transmission ($T_{on}$) der Flüssigkristallzelle (LC);

15.1.3 mindestens einen Sensor (IL, IR) zum Messen der Helligkeit des auf ihn treffenden sichtbaren Lichts;

15.1.3.1 wobei der mindestens eine Sensor (IL, IR) augenseitig des Brillenglases angeordnet ist;

15.1.3.2 wobei der mindestens eine Sensor (IL, IR) die Helligkeit durch das mindestens eine Brillenglas misst;

15.1.4 einen geschlossenen Regelkreis (MC) für die Regelung der Transmission der Flüssigkristallzelle (LC);

15.1.4.1 wobei die Regelung derart ausgebildet wird, dass die Zeiten des Zustands hoher Transmission ($T_{on}$) mit zunehmender Blendung kürzer werden;

15.1.4.2 wobei ein Sollwert für die Helligkeit am Auge des Brillenträgers vorgegeben wird;

15.1.4.3 wobei der Regelkreis als Istwert die vom Sensor gemessene Helligkeit heranzieht;

15.2 eine Anzeige wird zur Verfügung gestellt, wobei die Anzeige derart ausgebildet wird, dass sie folgendes aufweist:

15.2.1 Mittel zum Steuern oder Regeln der Leuchtzeiten und der Leuchtintensität der Anzeige derart, dass diese während der Zeiten des Zustands hoher Transmission ($T_{on}$) der Flüssigkristallzelle (LC) leuchtet;

15.2.2 wobei das zeitliche Integral des Produkts aus

15.2.2.1 der Leuchtintensität der Anzeige und

15.2.2.2 der Transmission (TR) der Flüssigkristallzelle (LC)

bei einer Veränderung der Zeiten des Zustands hoher Transmission ($T_{on}$) innerhalb einer vorgegebenen Toleranz konstant bleibt.

Fig. 1

Fig. 2

50

Fig. 3

Fig. 4

Fig. 5

HUD

F

OS

LC

IS

LED

IS2

LS

E(L)

Fig. 5B

Fig. 6

Fig. 7

EP 4 238 621 A2

Fig. 8

Fig. 9

EP 4 238 621 A2

Fig. 10

Fig. 11

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5172256 A
- DE 102012217326 A1
- US 2066680 A
- DE 10134770 A1
- DE 2001086 A
- WO 2013143998 A2
- EP 0813079 A2
- DE 102014107587

**In der Beschreibung aufgeführte Nicht-Patentliteratur**

- **DOUGLAS MACE ; PHILIP GARVEY ; RICHARD J. PORTER ; RICHARD SCHWAB ; WERNER ADRIAN.** Counter-measures for Reducing the Effects of Headlight Glare. *Prepared for: The AAA Foundation for Traffic Safety, Washington, D.C.,* Dezember 2001
- **ADRIAN, W. ; BHANJI, A.** Fundamentals of disability glare. A formula to describe stray light in the eye as a function of the glare angle and age. *Proceedings of the First International Symposium on Glare, Orlando, Florida,* 1991, 185-194
- **ADRIAN, W. ; BHANJI, A.** Fundamentals of disability glare. A formula to describe stray light in the eye as a function of the glare angle and age. *Proceedings of the First International Symposium on Glare,* 1991, 185-194
- **DOUGLAS MACE ; PHILIP GARVEY ; RICHARD J. PORTER ; RICHARD SCHWAB ; WERNER ADRIAN.** Countermeasures for Reducing the Effects of Headlight Glare. *Prepared for: The AAA Foundation for Traffic Safety, Washington, D.C.,* Dezember 2001
- **GERT HAUSKE.** Systemtheorie der visuellen Wahrnehmung. Teubner Verlag, Stuttgart, 1994